(19)

(11) **EP 2 714 006 B1**

(12) # EUROPEAN PATENT SPECIFICATION

(45) Date of publication and mention of the grant of the patent: **09.12.2020 Bulletin 2020/50**

(21) Application number: **12748245.3**

(22) Date of filing: **15.05.2012**

(51) Int Cl.:
***A61K 9/00*** *(2006.01)* ***A61K 9/06*** *(2006.01)*
***A61K 31/568*** *(2006.01)* ***A61K 47/34*** *(2017.01)*
***A61K 47/44*** *(2017.01)* ***A61P 5/26*** *(2006.01)*

(86) International application number: **PCT/IB2012/001112**

(87) International publication number: **WO 2012/156820 (22.11.2012 Gazette 2012/47)**

(54) **INTRANASAL TESTOSTERONE BIO-ADHESIVE GEL FORMULATIONS AND USE THEREOF FOR TREATING MALE HYPOGONADISM**

INTRANASALE BIOADHÄSIVE TESTOSTERONGELFORMULIERUNGEN UND IHRE VERWENDUNG ZUR BEHANDLUNG VON HYPOGONADISMUS BEI MÄNNERN

FORMULATIONS INTRANASALES BIO-ADHÉSIVES DE GEL DE TESTOSTÉRONE ET LEUR UTILISATION POUR TRAITER L'HYPOGONADISME CHEZ LES HOMMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2011 US 201161486324 P**
**16.05.2011 US 201161486634 P**

(43) Date of publication of application: **09.04.2014 Bulletin 2014/15**

(73) Proprietor: **Acerus Biopharma Inc.**
**Mississauga, ON L5L 1J9 (CA)**

(72) Inventors:
- **KREPPNER, Wayne**
  **Georgetown, ON L7G 5H1 (CA)**
- **FOGARTY, Siobhan**
  **Dublin (IE)**
- **OBEREGGER, Werner**
  **Waterdown, ON L0R 2H4 (CA)**
- **MAES, Paul, José, Pierre, Marie**
  **4600 Visé (BE)**

(74) Representative: **Wakerley, Helen Rachael**
**Reddie & Grose LLP**
**The White Chapel Building**
**10 Whitechapel High Street**
**London E1 8QS (GB)**

(56) References cited:
**EP-A1- 2 191 833** **WO-A1-03/088974**
**US-A1- 2010 311 707**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 714 006 B1

## Description

### Field of the Invention

The present invention relates to 4.5% intranasal testosterone bio-adhesive gels for providing sustained intranasal delivery of testosterone to a male and intranasal treatment methods for safely providing sustained release testosterone to treat males with hypogonadism. In particular, the present invention relates to improved testosterone replacement therapy (TRT) and sustained intranasal testosterone gel formulations for treating male hypogonadism. The present invention also relates to a system for dispensing intranasally a precise dosage amount of such gels in smaller volumes at an optimal anatomical location within each nostril of the male, so that an effective amount of testosterone is deposited within each nostril at the optimal anatomical location for TRT, including to effectively treat testosterone deficiency in male subjects, such as hypogonadism.

### Background

Androgens are a group of C19 steroids that cause masculinization of the genital tract and the development and maintenance of male secondary sex characteristics. They also contribute to muscle bulk, bone mass, libido, and sexual performance in men. Testosterone is the main androgen secreted by the Leydig cells of the testes, and its production increases during puberty. See. e.g., Tietz: Textbook of Clinical Chemistry and Molecular Diagnostics, 4th edition, Editors: Burtis CA, Ashwood ER, and Bruns DE (2006.). Androgen deficiency is now recognized to be a relatively common condition in the aging male. See, e.g., 2. Wang C, Swerdloff R.S.: Androgen replacement therapy. Ann Med, 29: 365-370 (1997); Matsumoto A.M.: Andropause: clinical implications of the decline in serum Testosterone levels with aging in men. J Gerontol A Med Sci, 57: M76-M99 (2002); and Haren Mtet al.: Andropause: a quality-of-life issue in older males. Med Clin North Am, 90: 1005-1023 (2006). Testosterone hormone therapy is indicated for replacement therapy and males having conditions associated with a deficiency or absence of endogenous testosterone, such as to treat male hypogonadism. This may cause sexual dysfunction, muscle loss, increase in fat, infertility, decreased beard and body hair and other conditions. An example of a composition for such treatments can be found in US2010/311707.

Hypogonadism is defined as testosterone deficiency. Male hypogonadism may be congenital or it may develop later in life due to, e.g., injury, trauma, surgery, infection, disease, drugs and/or aging. Generally, child-onset male hypogonadism has minimal consequences and generally remains undiagnosed until puberty is delayed. The symptoms or signs associated with child-onset male hypogonadism, if left untreated, include poor muscle and body hair development, including poor facial, pubic, chest and axillary hair growth, a high-pitched voice, excessive growth of arms and legs in relation to the trunk of the body, a small scrotum, abnormal phallic and testicular growth, and other growth problems, e.g., growth and maturation of the prostate and seminal vesicles. In adult-onset male hypogonadism, the symptoms may include a deficiency in spermatozoa production, osteoporosis, muscle loss or alterations in body musculature, fat distribution, fatigue and loss of energy, weakness, anemia, mood swings, e.g., depression and anger, a decline in cognitive skills, including memory loss and inability to concentrate, sleep disturbances, gynecomastia, a reduction in both beard and body hair, impotence, erectile dysfunction; a decrease in ejaculate volume, infertility, a decrease in sexual desire (loss of libido), and a regression of other secondary sexual characteristics.

Male hypogonadism is designated as either primary hypogonadism, which is due to a disorder of the testes, or central or secondary hypogonadism that results from a disorder in the hypothalamic-pituitary axis. In primary hypogonadism, there is a lack of testosterone production in the testes because the testes do not respond to FSH and LH. As a result, elevations in both hormones, FSH and LH, are observed in primary male hypogonadism. The most common cause of primary male hypogonadism is Klinefelter's syndrome. Other congenital causes of primary gonadism may include, e.g., Bilateral Congenital Anorchia, Leydig Cell Hypoplasia (Leydig Cell Aplasia), undescended testicles (Cryptorchidism), Noonan syndrome, Myotonic Dystrophy (MD) and defects in testosterone enzymatic synthesis. Causes of adult-onset primary hypogonadism may include aging, autoimmune disorders, surgery, chemotherapy, radiation, infection, disease, surgery, alcoholism, drug therapy and recreational drug use.

In secondary or central hypogonadism, insufficient amounts of FSH and LH are produced in the hypothalamus. Genital causes of secondary or central hypogonadism include, e.g., Kallmann syndrome, Prader-Willi syndrome (PWS), Dandy-Walker malformation, Isolated luteinizing hormone (LH) deficiency and Idiopathic hypogonadotropic hypogonadism (IHH). Causes of adult-onset secondary or central hypogonadism may include aging, disease, infections, tumors, bleeding, nutritional deficiencies, alcoholism, cirrhosis of the liver, obesity, weight loss, Cushing's syndrome, hypopituitarism, hyperprolactinemia, hemochromatosis, surgery, trauma, drug therapy, and recreational drug use.

In primary male hypogonadism, the levels observed for testosterone are below normal but are generally above normal for FSH and LH. In secondary or central male hypogonadism, the levels observed for testosterone, FSH and LH are below normal. Thus, diagnosis of primary or secondary male hypogonadism is typically confirmed by hormone levels and, on testing, blood levels of testosterone in both primary and secondary hypogonadism are characterized as low and

should be replaced. Treatment generally varies with etiology, but typically includes testosterone replacement therapy. In the United States, testosterone may be administered as an intramuscular injection, a transdermal patch or a transdermal gel. In other countries, oral preparations of testosterone may be available.

In view of the fact that millions of men in the United States, as well as through out the world, suffer from hypogonadism, there is a real and immediate need for an effective and convenient medical therapy that can treat this disorder, so that the quality of life of these individuals can be improved. One therapeutic goal of one such therapy to solve this immediate need might be to restore testosterone levels in men to young adulthood levels in hopes to alleviate the symptoms generally associated with hypogonadism due possibly to testosterone deficiency.

## Summary of the Invention

The present invention overcomes the limitations and disadvantages associated with current testosterone replacement therapy (TRT) and, in particular, current testosterone therapy to treat hypogonadism in male subjects through the discovery of novel per nasal testosterone gels and methods of use fort TRT and to treat hypogonadism. Particularly, the present invention overcomes the limitations and disadvantages of currently available options for administration of testosterone through the discovery of novel and improved dosage strength testosterone gel formulations specifically designed for intranasal administration to deliver therapeutically effective amounts of testosterone to treat males who suffer from and/or have been diagnosed with testosterone deficiency, including hypogonadism.

The term "a therapeutically effective amount" means an amount of testosterone sufficient to induce a therapeutic or prophylactic effect for use in testosterone replacement or supplemental therapy to treat male testosterone deficiency, namely, hypogonadism in males.

Thus, generally speaking, the present invention provides for new and improved, substantially less-irritating, novel dosage strength testosterone gel formulations formulated with testosterone in an amount of 4.5% by weight, for nasal administration, as defined by the claims. The formulation is intended to deliver a therapeutically of an effective amount of testosterone to effectively treat males who are diagnosed with testosterone deficiency, including hypogonadism.

In acordance with the present invention, the rates of diffusion of the testosterone in the intranasal gels of the present invention through a Franz cell membrane, as contemplated by the present invention, are between about 28 and 100 slope/mgT%, and preferably about 30 and 95 slope/mgT%. For those intranasal gels formulated with between about 4.0% and 4.5% testosterone, the preferred rates of diffusion of testosterone are between about 28 and 35 slope/mgT%.

Also disclosed are methods for pernasal administration of the nasal testosterone gels. Generally speaking, the novel methods involve depositing the intranasal testosterone gels topically into the nasal cavity of each nostril to deliver a therapeutically effective amount of testosterone in smaller volumes over dose life for providing constant effective testosterone brain and/or blood levels for use TRT, especially for effectively treating males in need of testosterone to treat hypogonadism.

More specifically, the present invention is directed to bioavailable intranasal testosterone gel formulations suitable for pernasal administration to for use in TRT and to treat hypogonadal subjects. In accordance with the present invention, and by way of example. The present invention contemplates:

- Treatment with unit-dose devices pre-filled with 125 μL 4.0% testosterone gel to deliver about 5.0 mg of testosterone per nostril (intra-nasal) given, e.g., three times a day (total dose 30 mg/day);
- Treatment with unit-dose devices pre-filled with about 150 μL 4.5% gel to deliver about 6.75 mg of Testosterone per nostril (intra-nasal) given, e.g., twice daily (total dose 27.0 mg/day); and/or
- Treatment with unit-dose devices pre-filled with about 125 μL 4.5% gel to deliver about 5.625 mg of Testosterone per nostril (intra-nasal) given, e.g., three times a day (total dose 33.75 mg/day).

Generally speaking, the intranasal testosterone gel formulations of the present invention are formulated with 4.5% testosterone by weight, and the testosterone is well absorbed when such gel formulations are administered pernasally to hypogonadal subjects. More specifically, testosterone is rapidly absorbed following pernasal administration with a peak concentration reached within 36 minutes to 1 hour 6 minutes (mean Tmax) following intra-nasal administration and maximal serum concentration is reached after about 1-2 hours post nasal administration. The maximum Testosterone concentration over a 24-hour interval is observed during the first administration (0-10 hours) in approximately 57% to 71% of the hypogonadal men while approximately 29% to 43% of the subjects had their maximum 24-h Testosterone concentration during subsequent administrations.

The formulations containing 4.5% testosterone by weight provide surprising properties. Importantly, the solubility of testosterone in castor oil pure is 3.6% maximum, falling to 3.36% about with 4% Labrafil. Addition of fumed silica (Aerosil, CabOsil) can increase the solubility of testosterone in castor oil up to 4.5% even with 4.0% Labrafil. This is counter intuitive for a person skilled in the art. However, without wishing to be bound by any particular theory, it is believed

that this increase in solubility in the presence of silica is due, at least in part, to the fact that $SiO_2$ adsorbs about 10% of the testosterone.

In accordance with the disclosed methods the intranasal testosterone gels are topically deposited on the outer external walls (opposite the nasal septum) inside the naval cavity of each nostril, preferably at about the middle to about the upper section of the outer external wall (opposite the nasal septum) just under the cartilage section of the outer external wall inside the naval cavity of each nostril. Once gel deposition is complete within each nostril of the nose, the outer nose is then gently and carefully squeezed and/or rubbed by the subject, so that the deposited gel remains in contact with the mucosal membranes within the nasal cavity for sustained release of the testosterone over dose life. Typical testosterone gel dosage amounts deposited pernasal application is between about 50 to about 150 microliters per nostril, and preferably about 125 to about 150 microliters per nostril.

In carrying out the disclosed methods, approximately between about 50 microliters and about 150 microliters of an intranasal testosterone gel of the present invention is applied to each nostril of a subject once or twice daily or three times a day, e.g., for one, two, three, four or more consecutive weeks, or for two, three, four, five or six consecutive days or more, or intermittently such as every other day or once, twice or three times weekly, or on demand once or twice during the same day, as TRT or to treat male testosterone deficiency, including male hypogonadism.

In addition, the present invention contemplates testosterone gel formulations for nasal administration that are pharmaceutically equivalent, therapeutically equivalent, bioequivalent and/or interchangeable, regardless of the method selected to demonstrate equivalents or bioequivalence, such as pharmacokinetic methodologies, microdialysis, in vitro and in vivo methods and/or clinical endpoints described herein. Thus, the present invention contemplates testosterone gel formulations for nasal administration that are bioequivalent, pharmaceutically equivalent and/or therapeutically equivalent, especially testosterone gel formulations for nasal administration that are 0.15% testosterone by weight of the gel formulation, 0.45% testosterone by weight of the gel formulation and 0.6% testosterone by weight of the gel formulation, when used in accordance with the therapy of the present invention to treat anorgasmia and/or HSDD by intranasal administration. Thus, the present invention contemplates: (a) pharmaceutically equivalent testosterone gel formulations for nasal administration which contain the same amount of testosterone in the same dosage form; (b) bioequivalent testosterone gel formulations for nasal administration which are chemically equivalent and which, when administered to the same individuals in the same dosage regimens, result in comparable bioavailabilities; (c) therapeutic equivalent testosterone gel formulations for nasal administration which, when administered to the same individuals in the same dosage regimens, provide essentially the same efficacy and/or toxicity; and (d) interchangeable testosterone gel formulations for nasal administration of the present invention which are pharmaceutically equivalent, bioequivalent and therapeutically equivalent.

While the intranasal testosterone gels of the present invention are preferred pharmaceutical preparations when practicing the novel methods of the present invention, it should be understood that the novel topical intranasal gel formulations and methods of the present invention also contemplate the pernasal administration of any suitable active ingredient, either alone or in combination with testosterone or other active ingredients, such as neurosteroids or sexual hormones (e.g., androgens and progestins, like testosterone, estradiol, estrogen, oestrone, progesterone, etc.), neurotransmitters, (e.g., acetylcholine, epinephrine, norepinephrine, dopamine, serotonin, melatonin, histamine, glutamate, gamma aminobutyric acid, aspartate, glycine, adenosine, ATP, GTP, oxytocin, vasopressin, endorphin, nitric oxide, pregnenolone, etc.), prostaglandin, benzodiazepines like diazepam, midazolam, lorazepam, etc., and PDEF inhibitors like sildenafil, tadalafil, vardenafil, etc., in any suitable pharmaceutical preparation, such as a liquid, cream, ointment, salve or gel. Examples of additional topical formulations for practice in accordance with the novel methods of the present invention include the topical pernasal formulations disclosed in, for example, U.S. Patent Nos. 5,578,588, 5,756,071 and 5,756,071 and U.S. Patent Publication Nos. 2005/0100564, 2007/0149454 and 2009/0227550.

The present invention is also directed to packaged pharmaceuticals comprising the novel and improved testosterone gel formulations for nasal administration of the invention. For example, the present invention contemplates prefilled, single or multi-dose applicator systems for pernasal administration to strategically and uniquely deposit the nasal testosterone gels at the preferred locations within the nasal cavity for practicing the novel methods and teachings of the present invention. Generally, speaking the applicator systems of the present invention are, e.g., airless fluid, dip-tube fluid dispensing systems, pumps, pre-filled, unit-dose syringes or any other system suitable for practicing the methods of the present invention. The applicator systems or pumps include, for example, a chamber, pre-filled with a single dose or multiple doses of an intranasal testosterone gel of the present invention, that is closed by an actuator nozzle or cap. The actuator nozzle may comprise an outlet channel and tip, wherein the actuator nozzle is shaped to conform to the interior surface of a user's nostril for (a) consistent delivery of uniform dose amounts of an intranasal testosterone gel of the present invention during pernasal application within the nasal cavity, and (b) deposition at the instructed location within each nostril of a patient as contemplated by the novel methods and teachings of the present invention. Examples of pre-filled, multi-dose applicator systems include, e.g., (a) the COMOD system available from Ursatec, Verpackung-GmbH, Schillerstr. 4, 66606 St. Wendel, Germany, (b) the Albion or Digital airless applicator systems available from Airlessystems, RD 149 27380 Charleval, France or 250 North Route 303 Congers, NY 10950, (c) the nasal applicators

from Neopac, The Tube, Hoffmann Neopac AG, Burgdorfstrasse 22, Postfach, 3672 Oberdiessbach, Switzerland, or (d) the syringes described in the Examples herein below.

A nasal multi-dose dispenser device according to embodiments of the present invention, such as the Albion or Digital airless applicator systems available from Airlessystems, is comprised of a fluid container and a distributor pump for delivery of multiple doses of a gel or other topical formulation. In one embodiment of the present invention, the nasal multi-dose dispenser device is adapted for an airless fluid dispensing system. In another embodiment of the present invention, the nasal multi-dose dispenser device is adapted for a dip tube fluid dispensing system.

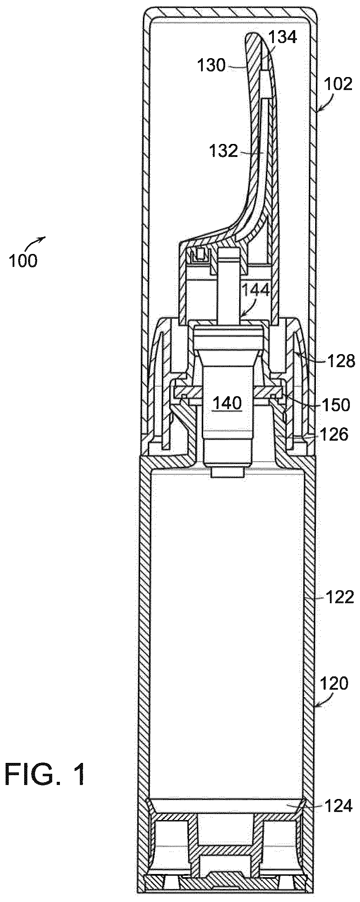

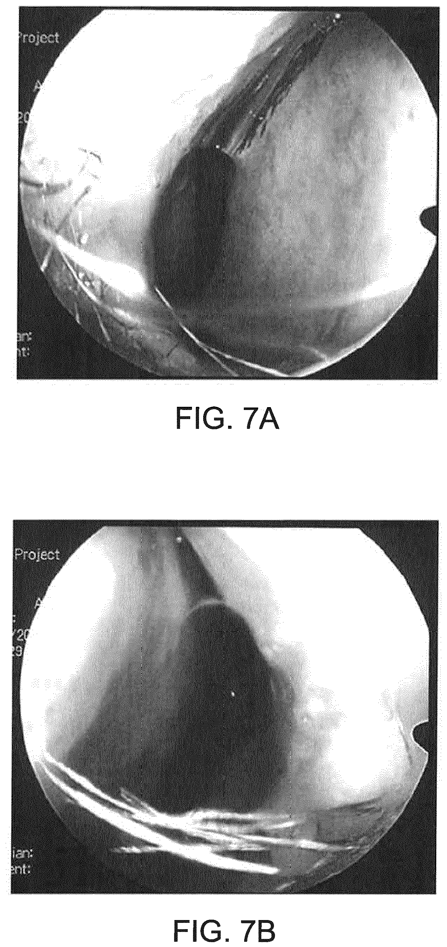

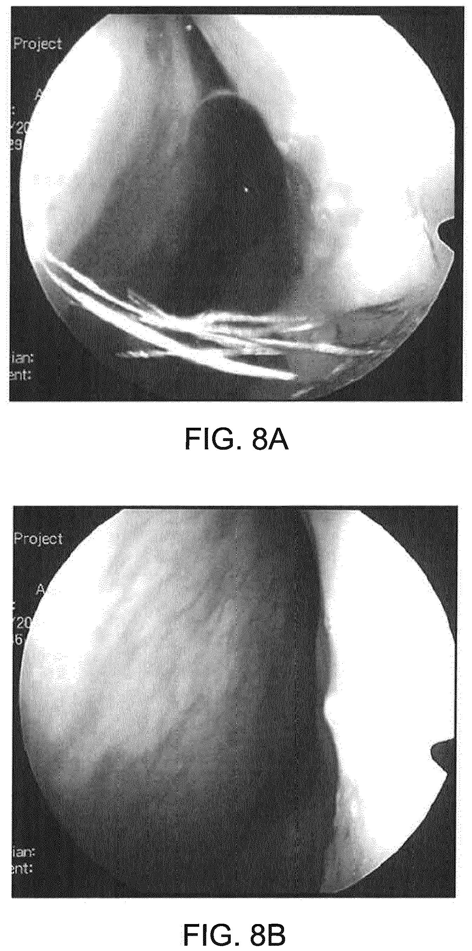

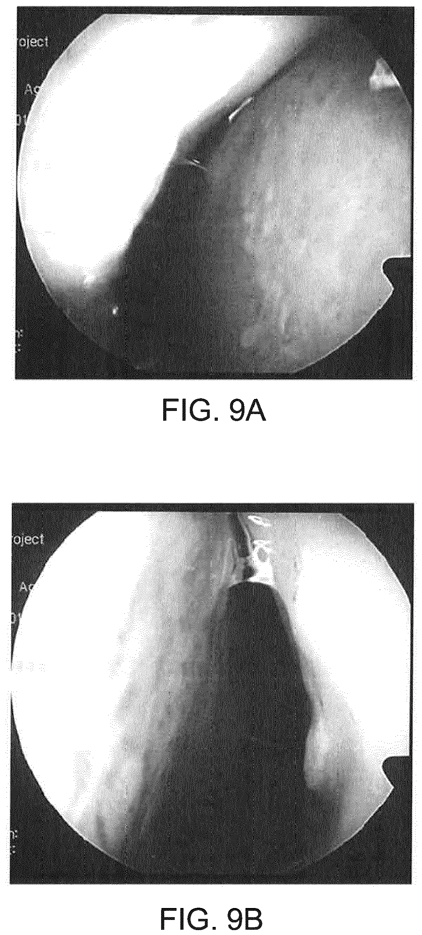

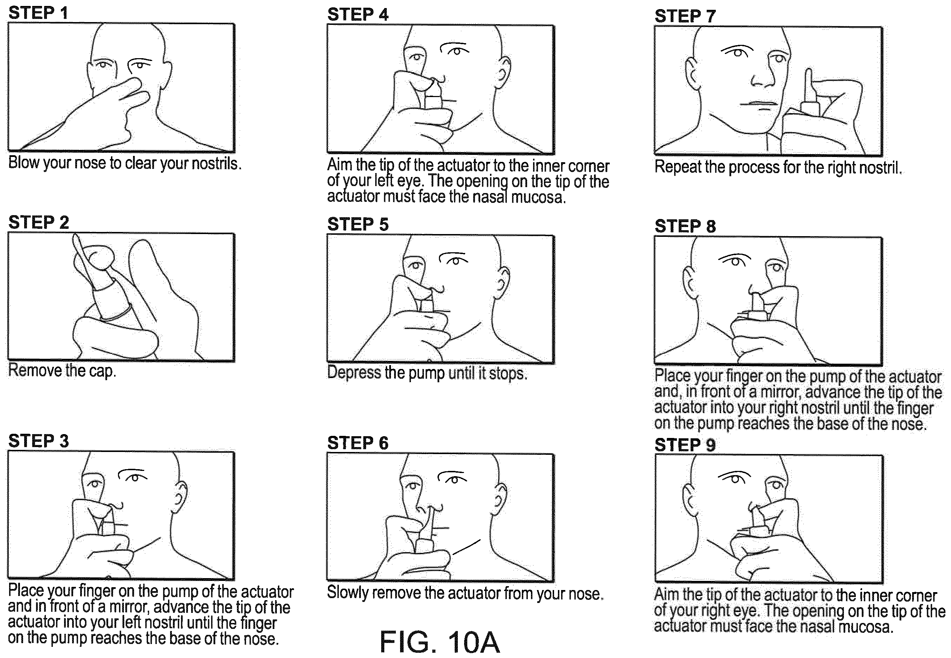

An example of an airless system that is contemplated by the present invention is one that will deliver a liquid, including gel, without the need for a pressured gas or air pump to be in contact with the liquid (or gel). In general, an airless system of the present invention comprises a flexible pouch containing the liquid, a solid cylindrical container a moving piston, an aspirating pump, a dosing valve and a delivery nozzle, as depicted, for example, in Figs. 1-4. See also Figs. 7A, 7B, 8A, 8B, 9A, 9B, 10A, 10B and 11.

In accordance with the present invention, the multi-dose dispenser 100 of Fig. 1 is provided with a fluid container 120, a distributor pump 140 and a cap 102.

The fluid container 120 comprises a container body 122, a base 124 and a neck 126. The distributor pump 140 is fastened to the neck by a sleeve 128. The top end of the container body 122 is closed by the distributor pump 140. The sleeve 128 tightly pinches a neck gasket 150 against the top end of the container body 122. The container body 122 forms a vacuum and houses the fluid to be dispensed.

The distributor pump 140 is closed by its actuator nozzle 130, which retains the stem 144 at the stem head. The actuator nozzle 130 comprises an outlet channel 132 and tip 134.

The actuator nozzle 130 is shaped to conform with the interior surface of a user's nostril. The actuator nozzle 130 is moveable between a downward open position and upward closed position. The user removes the cap 102 and inserts the actuator nozzle 130 in the user's nostril. When the user pushes the actuator nozzle 130 downwards to the open position, fluid in the dosing chamber 180 is withdrawn by the distributor pump 140 and exits at the tip 134 via the outlet channel 132 of the actuator nozzle 130.

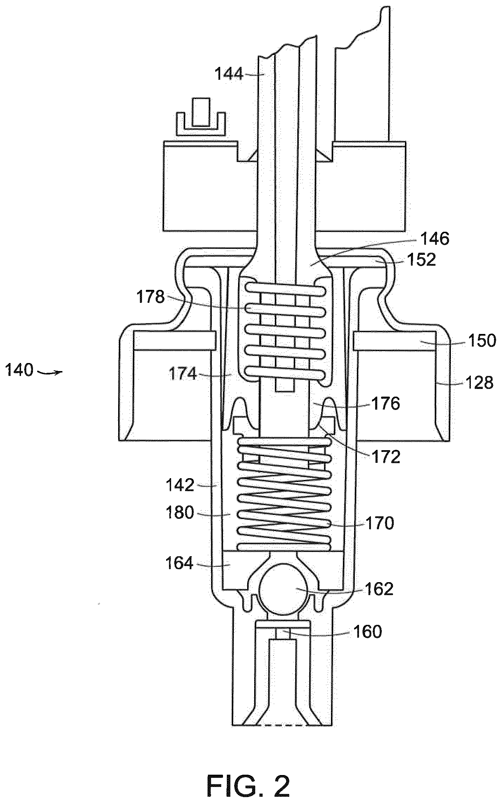

Fig. 2 shows a cross-sectional view of the distributor pump 140.

The distributor pump has a body 142 provided with a bottom intake having an inlet valve 160 with a ball 162 as its valve member. The ball 162 is held in place by a cage 164 and by a return spring 170.

At its bottom end, the stem 144 carries a spring cap 172. A piston 174 is located above the spring cap 172. The stem 144 passes through an axial orifice of the piston base 176.

The side walls of the piston 174 seals against the distributor pump body 142 via lips. The sleeve 128 tightly pinches a stem gasket 152 against the stem collar 146, distributor pump body 142 and top of the piston 174.

A precompression spring 178 placed between the piston base 176 and the stem collar 146. The precompression spring 178 biases the actuator nozzle 130 via the stem 144 to the closed position.

The return spring 170, which returns the piston 174 back upwards, is compressed between two opposed seats on the cage 164 and the spring cap 172.

The distributor pump 140 has a dosing chamber 180 formed between the cage 164 and piston 174. When the user pushes the actuator nozzle downwards to the open position, fluid in the dosing chamber is withdrawn by the distributor pump 140 and dispensed from the tip of the actuator nozzle 130.

When the user releases the actuator nozzle 130 upwards to the closed position, a fluid in the container body 122 is withdrawn into the dosing chamber 180 by the distributor pump 140. Thus, a dose of fluid is ready for the next actuation of the actuator nozzle by the user.

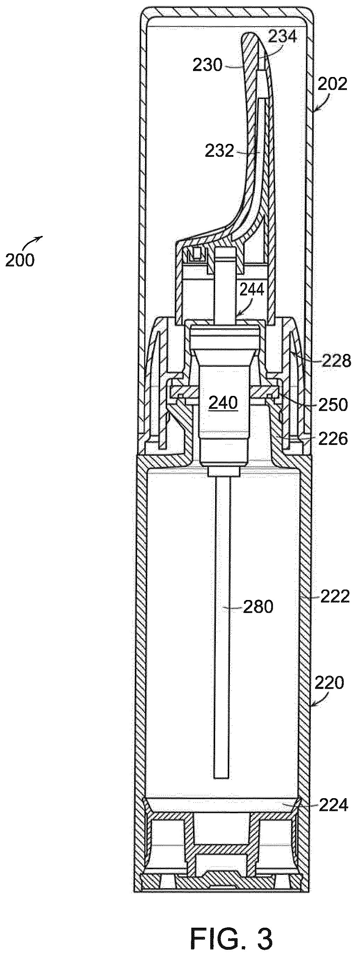

In another embodiment of the present invention, the dispenser 200 of Fig. 3 is provided with a fluid container 220, a distributor pump 240 and a cap 202.

The fluid container 220 comprises a container body 222, a base 224 and a neck 226. The distributor pump 240 is fastened to the neck by a sleeve 228. The top end of the container body 222 is closed by the distributor pump 240. The sleeve 228 tightly pinches a neck gasket 250 against the top end of the container body 222. The container body 222 houses the fluid to be dispensed.

The distributor pump 240 is closed by its actuator nozzle 230, which retains the stem 244 at the stem head. The actuator nozzle 230 comprises an outlet channel 232 and tip 234. The actuator nozzle 230 is shaped to conform with the interior surface of a user's nostril. The actuator nozzle 230 is moveable between a downward open position and upward closed position. The user removes the cap 202 and inserts the actuator nozzle 230 in the user's nostril. When the user pushes the actuator nozzle 230 downwards to the open position, fluid in the dosing chamber 280 is withdrawn by the distributor pump 240 and exits at the tip 234 via the outlet channel 232 of the actuator nozzle 230.

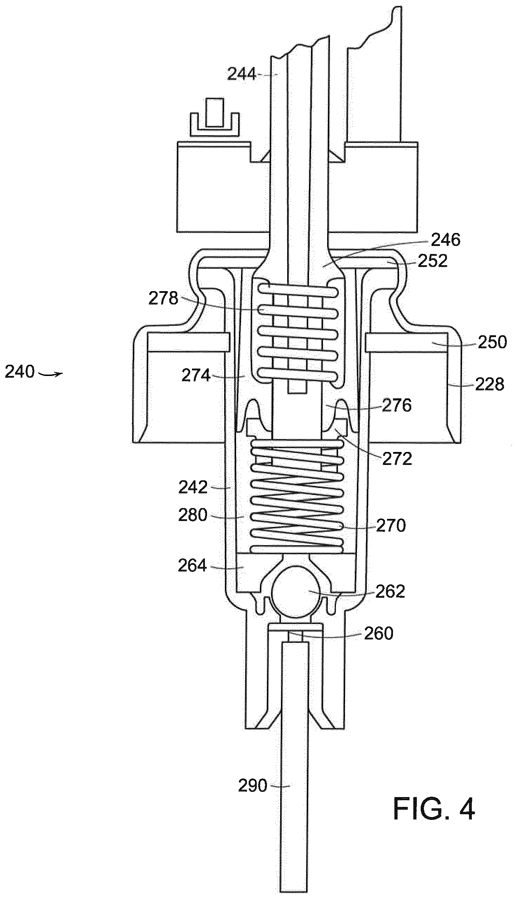

Fig. 4 shows a cross-sectional view of the distributor pump 240.

The distributor pump has a body 242 provided with a bottom intake having an inlet valve 260 with a ball 262 as its valve member. The ball 262 is held in place by a cage 264 and by a return spring 270. Optionally, a dip tube 290 can extend downward from the inlet valve 260 and is immersed in the liquid contained in the container body.

At its bottom end, the stem 244 carries a spring cap 272. A piston 274 is located above the spring cap 272. The stem 244 passes through an axial orifice of the piston base 276.

The side walls of the piston 274 seals against the distributor pump body 242 via lips. The sleeve 228 tightly pinches a stem gasket 252 against the stem collar 246, distributor pump body 242 and top of the piston 274.

A precompression spring 278 placed between the piston base 276 and the stem collar 246. The precompression spring 278 biases the actuator nozzle 230 via the stem 244 to the closed position.

The return spring 270, which returns the piston 274 back upwards, is compressed between two opposed seats on the cage 264 and the spring cap 272. The distributor pump 240 has a dosing chamber 280 formed between the cage 264 and piston 274. When the user pushes the actuator nozzle downwards to the open position, air enters the dosing chamber 280, which forces the fluid in the dosing chamber to be withdrawn by the distributor pump 240 and dispensed from the tip of the actuator nozzle 230.

When the user releases the actuator nozzle 230 upwards to the closed position, the air contained in the dosing chamber 280 forces the fluid in the container body 222 to be withdrawn into the dosing chamber 280. Thus, a dose of fluid is ready for the next actuation of the actuator nozzle by the user.

The amount of fluid withdrawn by the distributor pump into the dosing chamber may be a fixed volume. The distributor pumps may be of a variety of sizes to accommodate a range of delivery volumes. For example, a distributor pump may have a delivery volume of 140 $\mu$l.

The dispensers of the present invention may dispense topical intranasal gel or other topical intranasal formulations, preferably pernasally, which contain alternative or additional active ingredients, such as neurosteroids or sexual hormones (e.g., androgens and progestins, like testosterone, estradiol, estrogen, oestrone, progesterone, etc.), neurotransmitters, (e.g., acetylcholine, epinephrine, norepinephrine, dopamine, serotonin, melatonin, histamine, glutamate, gamma aminobutyric acid, aspartate, glycine, adenosine, ATP, GTP, oxytocin, vasopressin, endorphin, nitric oxide, pregnenolone, etc.), prostaglandin, benzodiazepines like diazepam, midazolam, lorazepam, etc., and PDEF inhibitors like sildenafil, tadalafil, vardenafil, etc., in the form of a liquid, cream, ointment, salve or gel. The dispensers may be suitable for cosmetic, dermatological or pharmaceutical applications. Examples of topical intranasal formulations for topical pernasal application, which can be dispensed in accordance with the present invention include the pernasal testosterone gels of the present invention or other intranasal topical gels wherein the testosterone is replaced or combined with a another active ingredient in effective amounts, such as those active ingredients discussed herein above. In addition, other testosterone formulations suitable and contemplated for dispensing from the dispensers and/or in accordance with the methods of the present invention include the formulations dislcosed in, for example, U.S. Patent Nos. 5,578,588, 5,756,071 and 5,756,071 and U.S. Patent Publication Nos. 2005/0100564, 2007/0149454 and 2009/0227550.

It should be understood by those versed in this art that the amount of testosterone in a lower dosage strength intranasal testosterone gel of the present invention that will be therapeutically effective in a specific situation will depend upon such things as the dosing regimen, the application site, the particular gel formulation, dose longevity and the condition being treated. As such, it is generally not practical to identify specific administration amounts herein; however, it is believed that those skilled in the art will be able to determine appropriate therapeutically effective amounts based on the guidance provided herein, information available in the art pertaining to testosterone replacement therapy, and routine testing.

It should be further understood that the above summary of the present invention is not intended to describe each disclosed embodiment or every implementation of the present invention. The description further exemplifies illustrative embodiments. In several places throughout the specification, guidance is provided through examples, which examples can be used in various combinations. In each instance, the examples serve only as representative groups and should not be interpreted as exclusive examples.

## Brief Description of the Drawings

The foregoing and other objects, advantages and features of the present invention, and the manner in which the same are accomplished, will become more readily apparent upon consideration of the following detailed description of the invention taken in conjunction with the accompanying figures and examples, which illustrate embodiments, wherein:

Fig. 1 is a side view of a first embodiment of the invention;.
Fig. 2 is a cross-sectional side view of the distributor pump of the first embodiment of the invention;
Fig. 3 is a side view of a second embodiment of the invention;
Fig. 4 is a cross-sectional side view of the distributor pump of the second embodiment of the invention;

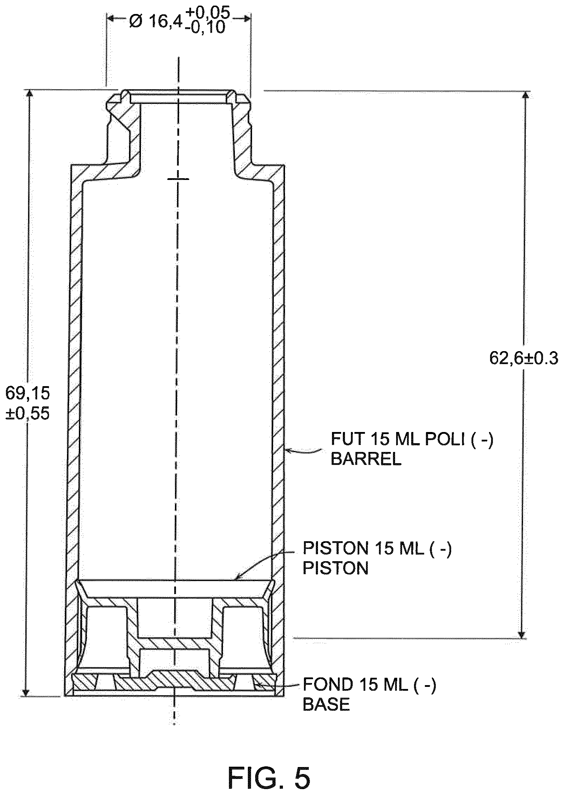

Fig. 5 is a side view of a second embodiment of the invention concerning an airless bottle assembly of the invention;

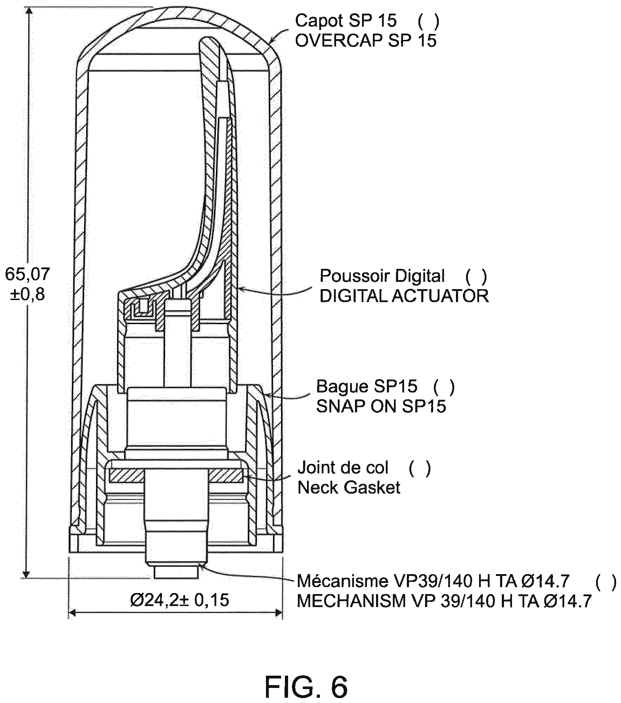

Fig. 6 is a side view of a second embodiment of the invention concerning digital actuator and rounded cap;
Fig. 7A depicts the right nostril of subject #1 after a single dose syringe administration;
Fig.7B depicts the left nostril of subject #1 after a multiple dose dispenser administration;

Fig. 8A depicts the right nostril of subject #2 after a single dose syringe administration;
Fig. 8B depicts the left nostril of subject #2 after a multiple dose dispenser administration;
Fig. 9A depicts the right nostril of subject #3 after a single dose syringe administration;
Fig. 9B depicts the left nostril of subject #3 after a multiple dose dispenser administration;
Figs. 10A and 10B illustrate use of a multiple dose dispenser in accordance with the present invention;
Fig. 11 illustrates a multiple dose dispenser in accordance with the present invention;
Fig. 12 depicts a Franz Cell apparatus position layouts for comparing testing in accordance with Example 5;

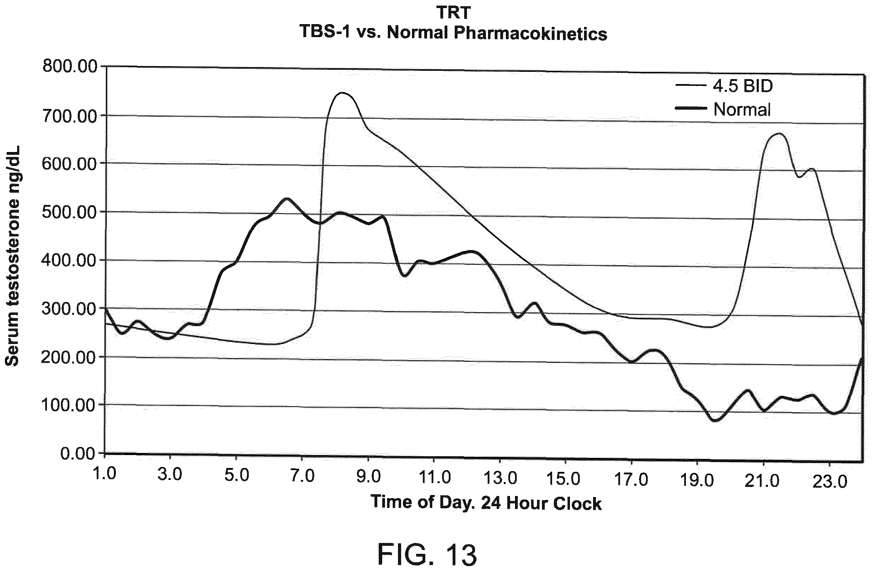

Fig. 13 is a graph showing the change in testerosterone levels in serum over time for a 4.5% testosterone bio-adhesive gel administered in each nostril of a hypogonadal male twice daily in accordance with the present invention as compared to normal testosterone pharmacokinetics in young healthy adult males, as reported in Diver MJ. et al: Diurnal rhythms of total, free and bioavailable testosterone and of SHBG in middle-aged men compared with those in young men. Clinical Endocrinology, 58: 710-717 (2003);
Fig. 14 depicts a comparison between TBS 1 A 8% (Part I);

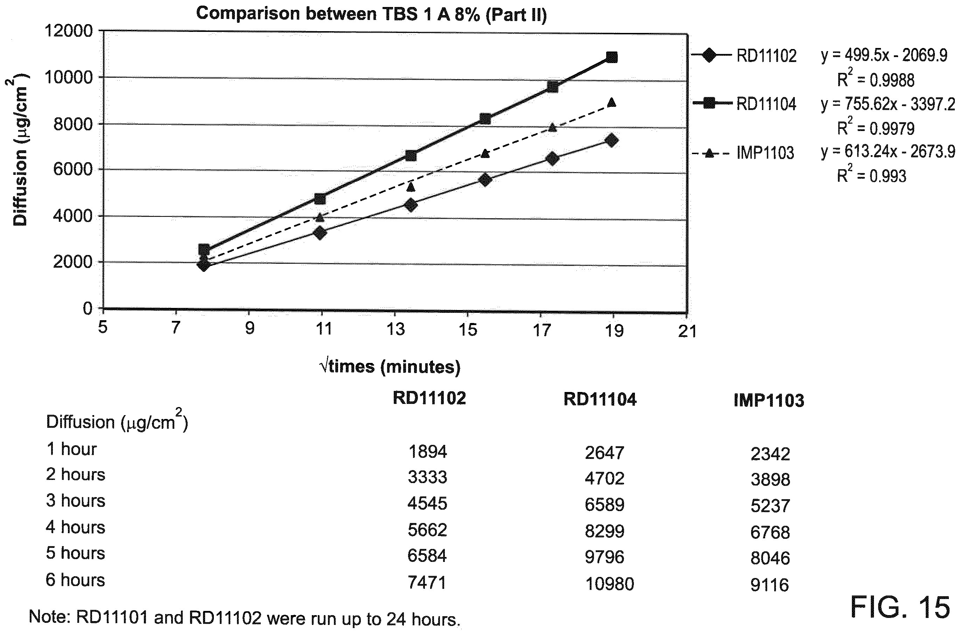

Fig. 15 depicts a comparison between TBS 1 A 8% (Part I);

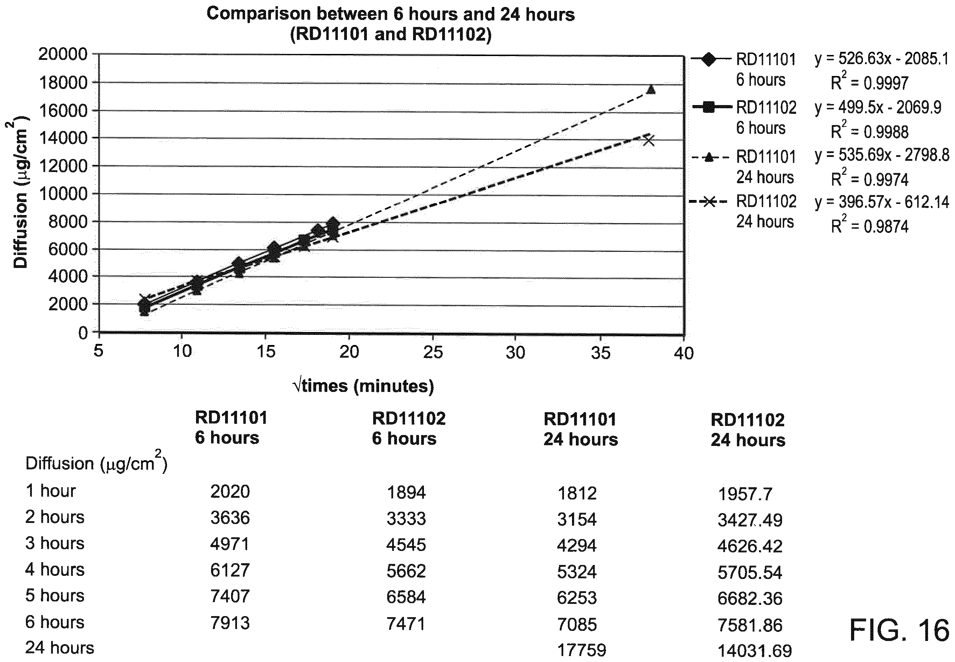

Fig. 16 depicts a comparison between 6 hours and 24 hours run (RD11101 and RD11102)

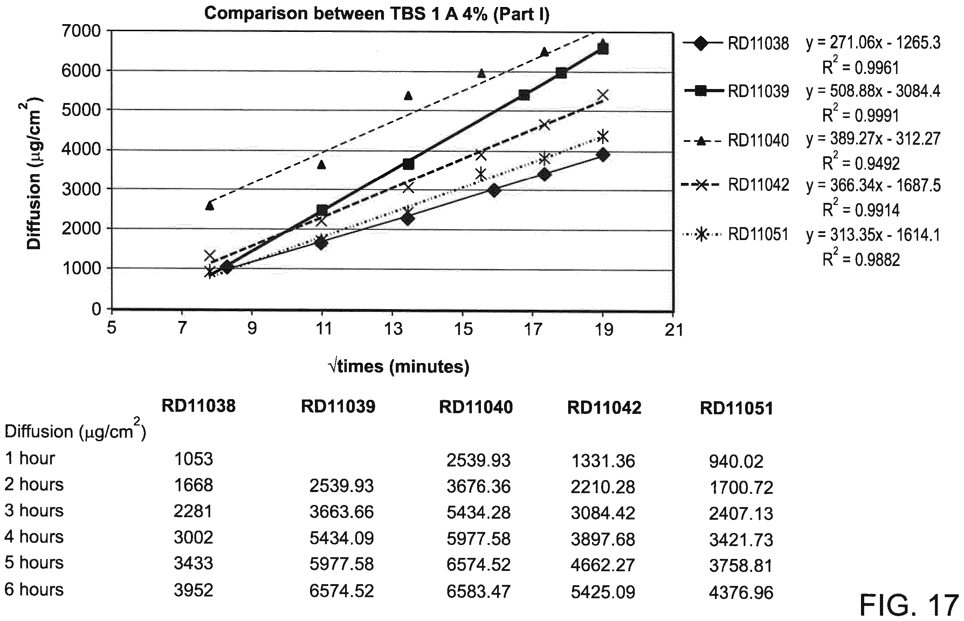

Fig. 17 depicts a comparison between TBS 1 A 4% (Part I);

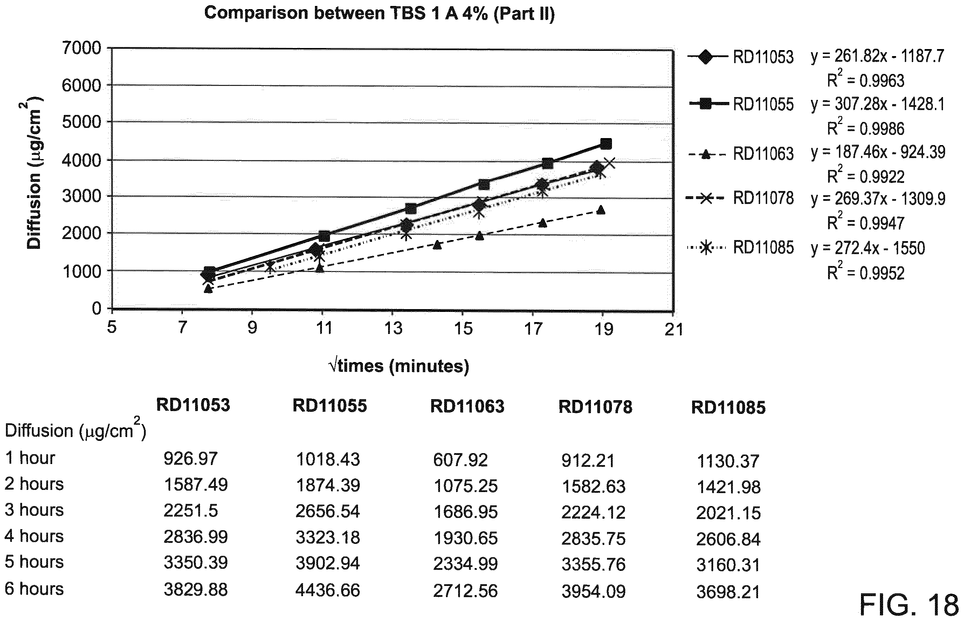

Fig. 18 depicts a comparison between TBS 1 A 4% (Part II);

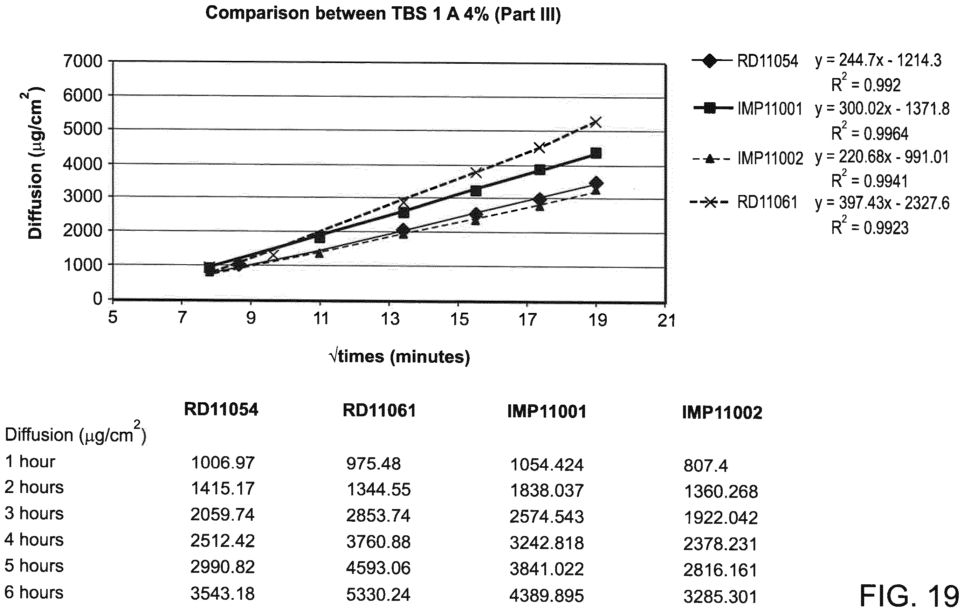

Fig. 19 depicts a comparison between TBS 1 A 4% (Part III);

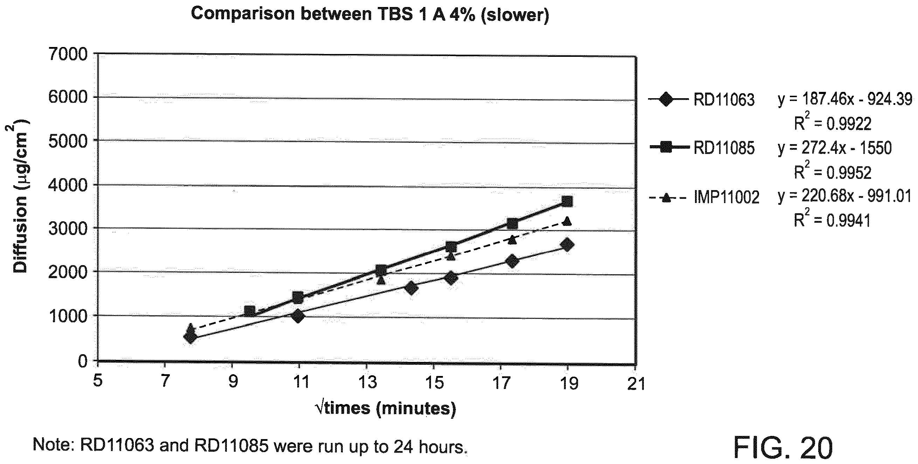

Fig. 20 depicts a comparison slower diffusion;

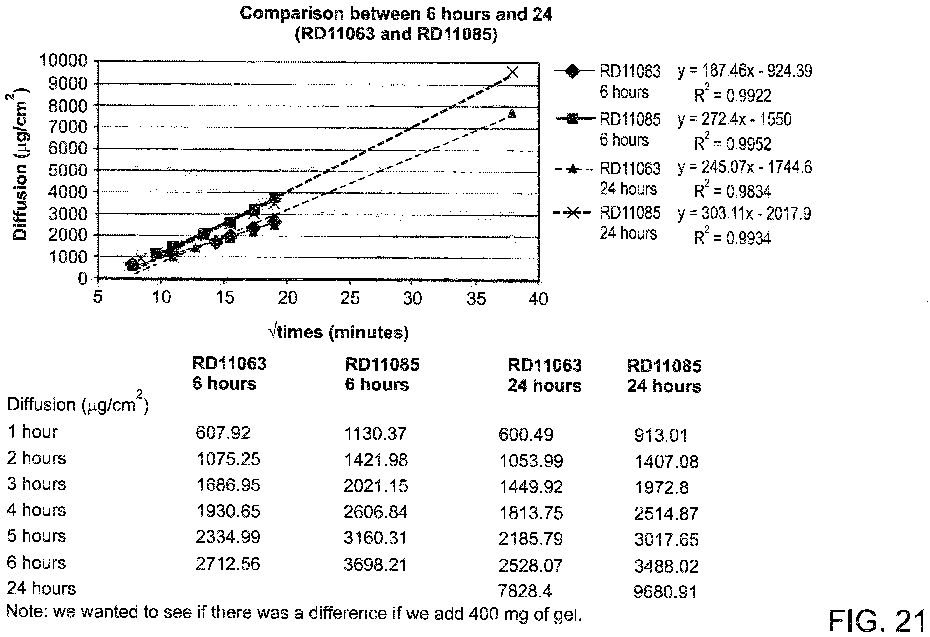

Fig. 21 depicts a comparison between 6 hours and 24 hours run (RD11063 and RD11085); and

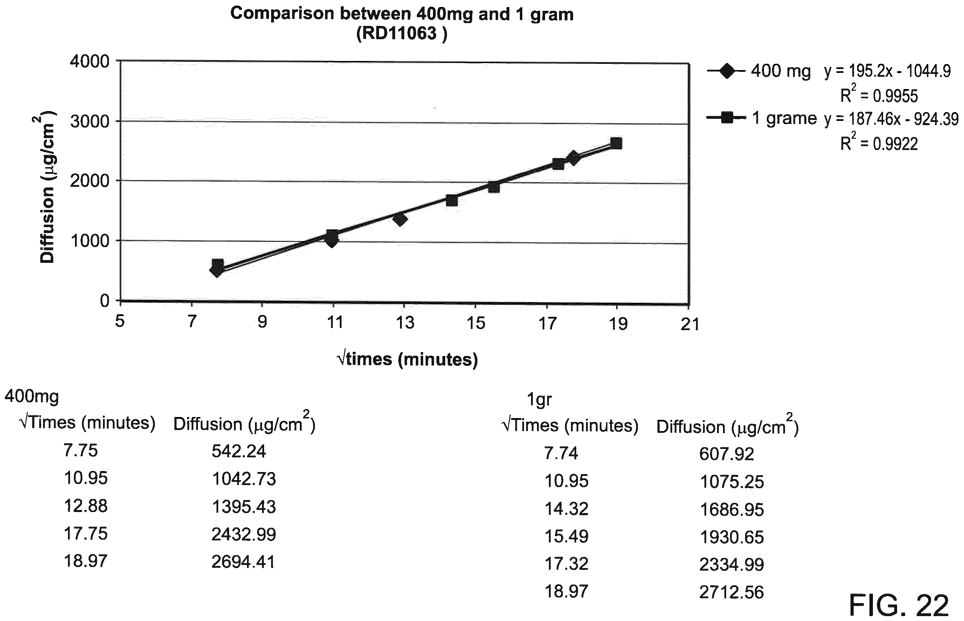

Fig. 22 depicts a comparison between 400mg and 1 gram of gel (RD11063).

## Detailed Description

By way of illustrating and providing a more complete appreciation of the present invention and many of the attendant advantages thereof, the following detailed description and examples are given concerning the novel lower dosage strength intranasal testosterone gels, application devices and methods of the present invention.

As used in the description of the invention and the appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein, "at least one" is intended to mean "one or more" of the listed elements.

Singular word forms are intended to include plural word forms and are likewise used herein interchangeably where appropriate and fall within each meaning, unless expressly stated otherwise.

Except where noted otherwise, capitalized and non-capitalized forms of all terms fall within each meaning.

Unless otherwise indicated, it is to be understood that all numbers expressing quantities, ratios, and numerical properties of ingredients, reaction conditions, and so forth used in the specification and claims are contemplated to be able to be modified in all instances by the term "about".

All parts, percentages, ratios, etc. herein are by weight unless indicated otherwise.

As used herein, "bioequivalence" or "bioequivalent", refers to nasally administered testosterone gel formulations or drug products which are pharmaceutically equivalent and their bioavailabilities (rate and extent of absorption) after administration in the same molar dosage or amount are similar to such a degree that their therapeutic effects, as to safety and efficacy, are essentially the same. In other words, bioequivalence or bioequivalent means the absence of a significant difference in the rate and extent to which testosterone becomes available from such formulations at the site of testosterone action when administered at the same molar dose under similar conditions, e.g., the rate at which testosterone can leave such a formulation and the rate at which testosterone can be absorbed and/or become available at the site of action to affect TRT, including hypogonadism. In other words, there is a high degree of similarity in the bioavailabilities of two testosterone gel formulation pharmaceutical products for nasal administration (of the same galenic form) from the same molar dose, that are unlikely to produce clinically relevant differences in therapeutic effects, or adverse reactions, or both. The terms "bioequivalence", as well as "pharmaceutical equivalence" and "therapeutic equivalence" are also used herein as defined and/or used by (a) the FDA, (b) the Code of Federal Regulations ("C.F.R."), Title 21, (c) Health Canada, (d) European Medicines Agency (EMEA), and/or (e) the Japanese Ministry of Health and Welfare. Thus, it should be understood that the present invention contemplates testosterone gel formulations for nasal administration or drug products that may be bioequivalent to other testosterone gel formulations for nasal administration or drug products of the present invention. By way of example, a first testosterone gel formulation for nasal administration or drug product is bioequivalent to a second testosterone gel formulation for nasal administration or drug product, in accordance with the present invention, when the measurement of at least one pharmacokinetic parameter(s), such as a Cmax, Tmax, AUC, etc., of the first testosterone gel formulation for nasal administration or drug product varies by no

more than about ±25%, when compared to the measurement of the same pharmacokinetic parameter for the second testosterone gel formulation for nasal administration or drug product of the present invention.

As used herein, "bioavailability" or "bioavailable", means generally the rate and extent of absorption of testosterone into the systemic circulation and, more specifically, the rate or measurements intended to reflect the rate and extent to which testosterone becomes available at the site of action or is absorbed from a drug product and becomes available at the site of action. In other words, and by way of example, the extent and rate of testosterone absorption from a lower dosage strength gel formulation for nasal administration of the present invention as reflected by a time-concentration curve of testosterone in systemic circulation.

As used herein, the terms "pharmaceutical equivalence" or "pharmaceutically equivalent", refer to testosterone gel formulations for nasal administration or drug products of the present invention that contain the same amount of testosterone, in the same dosage forms, but not necessarily containing the same inactive ingredients, for the same route of administration and meeting the same or comparable compendial or other applicable standards of identity, strength, quality, and purity, including potency and, where applicable, content uniformity and /or stability. Thus, it should be understood that the present invention contemplates testosterone gel formulations for nasal administration or drug products that may be pharmaceutically equivalent to other testosterone gel formulations for nasal administration or drug products used in accordance with the present invention.

As used herein, "therapeutic equivalence" or "therapeutically equivalent", means those testosterone gel formulations for nasal administration or drug products which (a) will produce the same clinical effect and safety profile when utilizing testosterone drug product for TRT and to treat testosterone deficiency, including hypogonadism, in male subjects in accordance with the present invention and (b) are pharmaceutical equivalents, e.g., they contain testosterone in the same dosage form, they have the same route of administration; and they have the same testosterone strength. In other words, therapeutic equivalence means that a chemical equivalent of a lower dosage strength testosterone formulation of the present invention (i.e., containing the same amount of testosterone in the same dosage form when administered to the same individuals in the same dosage regimen) will provide essentially the same efficacy and toxicity.

As used herein a "testosterone gel formulation for nasal administration" means a formulation comprising testosterone in combination with a solvent, a wetting agent, and a viscosity increasing agent.

As used herein, "plasma testosterone level" means the level of testosterone in the plasma of a subject. The plasma testosterone level is determined by methods known in the art.

"Diagnosis" or "prognosis," as used herein, refers to the use of information (e.g., biological or chemical information from biological samples, signs and symptoms, physical exam findings, psychological exam findings, etc.) to anticipate the most likely outcomes, timeframes, and/or responses to a particular treatment for a given disease, disorder, or condition, based on comparisons with a plurality of individuals sharing symptoms, signs, family histories, or other data relevant to consideration of a patient's health status, or the confirmation of a subject's affliction, e.g., testosterone deficiency, including hypogonadism.

A "subject" according to some embodiments is an individual whose signs and symptoms, physical exams findings and/or psychological exam findings are to be determined and recorded in conjunction with the individual's condition (i.e., disease or disorder status) and/or response to a candidate drug or treatment.

"Subject," as used herein, is preferably, but not necessarily limited to, a human subject. The subject may be male or female, and is preferably female, and may be of any race or ethnicity, including, but not limited to, Caucasian, African-American, African, Asian, Hispanic, Indian, etc. Subject as used herein may also include an animal, particularly a mammal such as a canine, feline, bovine, caprine, equine, ovine, porcine, rodent (e.g., a rat and mouse), a lagomorph, a primate (including non-human primate), etc., that may be treated in accordance with the methods of the present invention or screened for veterinary medicine or pharmaceutical drug development purposes. A subject according to some embodiments of the present invention include a patient, human or otherwise, in need of therapeutic treatment of testosterone deficiency, including hypogonadism.

"Treatment," as used herein, includes any drug, drug product, method, procedure, lifestyle change, or other adjustment introduced in attempt to effect a change in a particular aspect of a subject's health (i.e., directed to a particular disease, disorder, or condition).

"Drug" or "drug substance," as used herein, refers to an active ingredient, such as a chemical entity or biological entity, or combinations of chemical entities and/or biological entities, suitable to be administered to a male subject to treat testosterone deficiency, including hypogonadism. In accordance with the present invention, the drug or drug substance is testosterone or a pharmaceutically acceptable salt or ester thereof.

The term "drug product," as used herein, is synonymous with the terms "medicine," "medicament," "therapeutic intervention," or "pharmaceutical product." Most preferably, a drug product is approved by a government agency for use in accordance with the methods of the present invention. A drug product, in accordance with the present invention, is an intranasal gel formulated with a drug substance, i.e., testosterone.

"Disease," "disorder," and "condition" are commonly recognized in the art and designate the presence of signs and/or symptoms in an individual or patient that are generally recognized as abnormal and/or undesirable. Diseases or

conditions may be diagnosed and categorized based on pathological changes. The disease or condition may be selected from the types of diseases listed in standard texts, such as Harrison's Principles of Internal Medicine, 1997, or Robbins Pathologic Basis of Disease, 1998.

As used herein, "diagnosing" or "identifying a patient or subject having testosterone deficiency, such as hypogonadism, refers to a process of determining if an individual is afflicted with testosterone deficiency, such as hypogonadism.

As used herein, "control subject" means a subject that has not been diagnosed with testosterone deficiency or hypogonadism and/or does not exhibit any detectable symptoms associated with these diseases. A "control subject" also means a subject that is not at risk of developing testosterone deficiency or hypogonadism, as defined herein.

The testosterone gel formulations of the invention are viscous and thixotropic, oil-based formulations containing a solution of testosterone intended for intranasal application. The non-irritating formulation is designed to adhere to the inner nose. In addition, it acts as a controlling matrix, thus allowing sustained drug delivery through the nasal mucosa.

Other pharmacologically inactive ingredients in the testosterone intranasal gel are castor oil USP, oleoyl macrogolglycerides EP and colloidal silicon dioxide NF. None of these excipients are of human or animal origin. All excipients are well-known and listed in the "Inactive Ingredient" list for Approved Drug Products issued by the FDA.

The steroid hormone testosterone is the active ingredient in the testosterone gel formulations of the invention. The manufacture of the drug substance presents no potential risk for humans; the synthesis route is well-characterized.

Table 1: Nomenclature Testosterone

| | |
|---|---|
| INN name | Testosterone |
| Compendial name | Testosterone |
| Chemical name | 17β-Hydroxyandrost-4-en-3-one |
| Other non-proprietary names | Androst-4-en-3-one, 17-hydroxy-, (17β)-Trans-testosterone Δ4-androsten-17β-ol-3-one |
| CAS registry number | 58-22-0 |
| Proquina code | 8139 |

Structural Formula

**Molecular Formula**

$C_{19}H_{28}O_2$

**Relative Molecular Mass**

288.4

The physical chemical properties of testosterone are listed in Table 2.

**Table 2: General Properties of Testosterone**

| | |
|---|---|
| Appearance | White or slightly creamy white crystals or crystalline powder. It is odourless, and stable in air. |

(continued)

| | |
|---|---|
| Solubility | Practically insoluble in water (0.024 g/L), freely soluble in dehydrated alcohol, chloroform and in methylene chloride, soluble in dioxane and in vegetable oils; slightly soluble in ether. |
| Melting range | 153°C to 157°C |
| Specific rotation | +101° to + 105°(dioxane) |
| Loss on drying | Not more than 1.0% |
| UV max | 238 nm |
| Storage | Protected from light |

Testosterone, for testosterone gel formulations of the invention,appears as white or slightly creamy white crystals or crystalline powder. It is freely soluble in methanol and ethanol, soluble in acetone and isopropanol and insoluble in n-heptane. It can also be considered as insoluble in water ($S_{20°C}$=2.41 x $10^{-2}$ g/L $\pm$ 0.04 x $10^{-2}$ g/L); its n-Octanol/Water partition coefficient (log $P_{OW}$ determined by HPLC) is 2.84. The solubility of testosterone in oils was determined to be 0.8% in isopropylmyristate, 0.5% in peanut oil, 0.6% in soybean oil, 0.5% in corn oil, 0.7% in cottonseed oil and up to 4% in castor oil.

Because testosterone is fully dissolved within the formulations of the present invention, physical characteristics of the drug substance do not influence the performance of the drug product, testosterone gel formulations of the invention. The manufacturability of testosterone gel formulations of the invention, however is influenced by the particle size of testosterone. When using a particle size of 50% $\leq$ 25 microns, 90% $\leq$ 50 microns the solubility of the drug substance in the matrix is especially favorable.

In accordance with the present invention, the testosterone drug can be in, for instance, crystalline, amorphous, micronized, non-micronized, powder, small particle or large particle form when formulating to intranasal testosterone gels of the present invention. An Exemplary range of testosterone particle sizes include from about 0.5 microns to about 200 microns. Preferably, the testosterone particle size is in a range of from about 5 microns to about 100 microns, and the testosterone is in crystalline or amorphous and non-micronized or micronized form. Preferably, the testosterone is in crystalline or amorphous micronized form.

The molecular structure of testosterone contains no functional groups that can be protonated or deprotonated in the physiological pH-range. Therefore testosterone is to be considered as a neutral molecule with no pKa value in the range 1-14. Because it is neutral, testosterone is compatible with excipients.

The testosterone gel formulations of the invention are viscous and thixotropic, oil-based formulations containing a solution of testosterone intended for intranasal application. The non-irritating formulation is designed to adhere to the inner nose. In addition, it acts as a controlling matrix, thus allowing sustained drug delivery through the nasal mucosa.

Other pharmacologically inactive ingredients in the testosterone intranasal gel are castor oil USP, oleoyl macrogolglycerides EP and colloidal silicon dioxide NF. None of these excipients are of human or animal origin. All excipients are well-known and listed in the "Inactive Ingredient" list for Approved Drug Products issued by the FDA.

According to the "Handbook of Pharmaceutical Additives" oleoyl polyoxylglycerides are used as hydrophilic oil for topicals, injectables and nasals. In FDA-approved medicinal products it is used as co-emulsifier in topical emulsions/lotions/creams and in vaginal emulsions/creams. In France this excipient is approved for nasal preparations such as "Rhino-Sulforgan" (Laboratoire Jolly-Jatel, France; containing 10% oleoyl polyoxylglycerides) and "Huile Gomenolee 2% ("Laboratoire Goménol, France; containing 10% oleoyl polyoxylglycerides). Hence, like for castor oil it can be deduced that oleoyl polyoxylglycerides is suitable for an application route where safety and tolerability are of highest importance (e.g. injectables and nasal or vaginal preparations).

Oleoyl macrogolglycerides are also referred to as Labrafil M 1944 CS, apricot kernel oil PEG-6 esters, Peglicol-5-oleate, mixture of glycerides and polyethylene esters. The castor oil, which is used as a solvent for testosterone gel formulations of the invention, is a fixed oil. Such oils have the advantage of being non-volatile or spreading (in contrast to essential oils or liquid paraffin), but have the disadvantage of being hydrophobic. The nasal mucosa contains 95-97% water. Without the oleoyl macrogol-glycerides, the castor oil containing the active ingredient would form a non-interactive layer on the mucous membrane. In order to achieve adequate contact between the castor oil layer and the mucous membrane, the hydrophilic oleoyl macrogol-glycerides oil is added to the formulation to form an emulsion between the castor oil and the mucosa fluid.

Oleoyl macrogolglycerides are used in semi-solids at concentrations ranging from about 3 to 20%, depending on the application. The amount of oleoyl macrogol-glycerides in testosterone gel formulations of the invention is high enough to allow for a better contact of the carrier oil with the mucous membrane and low enough to have minimal impact on the amount of testosterone that can be incorporated into the carrier oil. A favourable concentration of oleoyl microgol-glycerides in testosterone gel formulations of the invention is found to be 4% of the formulation.

According to the "Handbook of Pharmaceutical Additives" colloidal silicon dioxide is used as an oil adsorbent, thermal stabiliser and gellant. In FDA-approved medicinal products it is used in dental gels, sublingual tablets, endocervical gel, suppositories, vaginal emulsions/creams/tablets/tampons and capsules for inhalation. Furthermore, it is used as an excipient in "Testoderm with adhesives" (Alza Corporation, approved in 1996) a testosterone transdermal patch. Hence, it can be deduced that colloidal silicon dioxide is suitable for an application route where safety and tolerability are of highest importance (e.g. inhalations, endocervical, vaginal or rectal preparations).

For clinical trial supplies, testosterone intranasal gel is supplied in unit-dose syringes consisting of a syringe body made from polypropylene, a plunger moulded from polyethylene and a syringe cap made from high density polyethylene. The syringes are wrapped in aluminum foil as secondary packaging. The pre-filled unit-dose syringes used in accordance with the study in the Examples are filled as follows: (a) 4% testosterone intranasal bio-adhesive gel - 148 microliters and 5.92 mgs of testosterone; (b) 4.5% testosterone intranasal bio-adhesive gel - 148 microliters and 6.66 mgs of testosterone; and (c) 4.5% testosterone intranasal bio-adhesive gel - 148 microliters and 7.785 mgs of testosterone.

The oil in testosterone gel formulations of the invention is thickened with colloidal silicon dioxide, which acts as a gel-forming agent. This compound is used commonly for stiffening oleogels.

The intended dosage form for testosterone gel formulations of the invention is a semi-solid, not a liquid. The formulation is thickened with colloidal silicon dioxide. It is believed that colloidal silicon dioxide contributes to the thixotropic properties of the gel, simplifying drug delivery to the nostril.

Colloidal silicon dioxide is generally an inert material which is well tolerated as an excipient in mucosal applications such as suppositories. Colloidal silicon dioxide is typically used in these preparations at concentrations ranging from about 0.5 to 10%. The concentration of colloidal silicon dioxide in testosterone gel formulations of the invention is high enough to achieve gel formation but at a level that has minimal impact on testosterone incorporation into the carrier oil.

Preferably, the intranasal testosterone gels of the present invention have in general, a viscosity in the range of between about 3,000 cps and about 27,000 cps. It should nevertheless be understood by those versed in this art that, while the above-mentioned viscosity range is believed to be a preferred viscosity range, any suitable viscosities or viscosity ranges that do not defeat the objectives of the present invention are contemplated.

A detailed description of batches of a testosterone gel formulation of the invention is shown in Table 3.

Table 3: Composition of a testosterone gel formulation of the invention

| Component | Amount (%w/w) 4.0% | Amount (%w/w) 0.45% |
|---|---|---|
| Testosterone | 4.0% | 4.5% |
| Castor oil | 88% | 87.5% |
| Oleoyl macrogol-glycerides | 4.0% | 4.0% |
| Colloidal silicon dioxide | 4.0% | 4.0% |

The testosterone gel formulations of the invention are stored at room temperature (20-25°C or 68 to 77°F). Temperature excursions from 15 to 30°C or 59 to 86°F are permissible for the testosterone gel formulations of the inventions. The stability data supports a 12-month shelf life. Unit dose syringes are chosen for the primary packaging of the clinical materials for the clinical trial described below to allow for ease of dosing, ability to generate multiple doses by varying the fill volume and consistency of dose delivered. The syringe consists of a syringe body, a plunger and a syringe cap. The syringes body is moulded from polypropylene, the plunger is moulded from polyethylene and the cap is HDPE. These syringes are designed and manufactured to deliver sterile and non-sterile solutions, liquids and gels at low volumes. For additional protection from the environment (i.e., exposure to dirt, light, humidity and oxygen), the syringes are packed in a foil-laminate overwrap pouch.

The syringes and caps are designed for use in a clinical setting and meet the requirements of the EU Medical Devices Directive 93/42/EEC of June 14, 1993 and as amended. As this container closure is only intended for use in this portion of the clinical program, no additional studies will be performed on the syringe and syringe components.

For a further element of protection, two syringes are contained in secondary packaging consisting of an aluminium foil pouch. Two syringes are packaged in the aluminium foil pouch and each pouch is sealed.

The pouch consists of a flexible, 3-layered-foil-laminate of a) polyester 12 micron, b) aluminum 12 micron and c) a polyethylene 75 micron. It is manufactured by Floeter Flexibles GmbH, and supplied under the name "CLIMAPAC II 12-12-75".

The invention provides for intranasal bio-adhesive gel formulations of testosterone to be administered intranasally, wherein the dosage of the formulation is from about 4.0% or 4.5% testosterone by weight of said gel.

The methods and treatments of the present invention are suitable for TRT in men and are especially suitable to treat testosterone deficient male subjects, such as those who are diagnosed with hypogonadism.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

## EXAMPLES

Having now generally described the invention, the same will be more readily understood through reference to the following Examples which are provided by way of illustration.

## EXAMPLE 1

### Description and Composition of Testosterone Gel Formulations of the Invention

The compositions of three different concentrations of the drug product to be administered in this clinical trial are provided in the tables below.

Description of Dosage Form

The testosterone gel formulations of the invention are viscous and thixotropic, oil-based formulations containing solubilized testosterone intended for intranasal application. The drug product is formulated with the compendial inactive ingredients: castor oil, oleoyl polyoxylglycerides and colloidal silicon dioxide.

Two different doses of the testosterone gel formulations of the invention are intranasally administered: 0.4% w/w and 0.45% w/w. An overage is added to each syringe to account for the gel that is retained in the syringe after dosing. This overage remains consistent at 23 μl, regardless of volume of gel in the syringe.

4.0% and 4.5% Intranasal Testosterone Compositions

Table 1: Components, Quantity, Quality Standards and Function - 0.4% testosterone gel formulation of the invention

| Component | Amount (% w/w) | Amount per Syringe (mg) | Amount Delivered per Dose (mg) | Function | Quality Standard |
|---|---|---|---|---|---|
| Testosterone | 4.0% | 5.92 | 5.0 | Active ingredient | USP |
| Castor oil | 88.0% | 130.24 | 110 | Solvent | USP |
| Oleoyl macrogol-glycerides | 4.0% | 5.92 | 5.0 | Wetting agent (hydrophilic oil) | Ph. Eur. |
| Colloidal silicon dioxide | 4.0% | 5.92 | 5.0 | Viscosity increasing agent | USP/NF |

Table 6 Components, Quantity, Quality Standards and Function - 0.6% testosterone gel formulation of the invention

| Component | Amount (% w/w) | Amount per Syringe (mg) | Amount Delivered per Dose (mg) | Function | Quality Standard |
|---|---|---|---|---|---|
| Testosterone | 0.6% | 0.74 | 0.6 | Active ingredient | USP |

(continued)

| Component | Amount (% w/w) | Amount per Syringe (mg) | Amount Delivered per Dose (mg) | Function | Quality Standard |
|---|---|---|---|---|---|
| Castor oil | 91.4% | 112.42 | 91.4 | Solvent | USP |
| Oleoyl polyoxylglycerides | 4.0% | 4.92 | 4.0 | Wetting agent (hydrophilic oil) | Ph. Eur/NF. |
| Colloidal silicon dioxide | 4.0% | 4.92 | 4.0 | Viscosity increasing agent | NF |
| Total | 100% | 123 mg | 100 mg | | |

**Table 2: Components, Quantity, Quality Standards and Function, TBS-1: 5.6mg/125μl/ syringe (4.5% gel)**

| Component | Amount (% w/w) | Amount per Syringe (mg) | Amount Delivered per Dose (mg) | Function | Quality Standard |
|---|---|---|---|---|---|
| Testosterone | 4.5% | 6.66 | 5.63 | Active ingredient | USP |
| Castor oil | 87.5% | 129.5 | 109.37 | Solvent | USP |
| Oleoyl macrogol-glycerides | 4.0% | 5.92 | 5.0 | Wetting agent (hydrophilic oil) | Ph. Eur. |
| Colloidal silicon dioxide | 4.0% | 5.92 | 5.0 | Viscosity increasing agent | USP/NF |

**Table 3: Components, Quantity, Quality Standards and Function, TBS-1: 6.75 mg/150 μl/ syringe (4.5% gel)**

| Component | Amount (% w/w) | Amount per Syringe (mg) | Amount Delivered per Dose (mg) | Function | Quality Standard |
|---|---|---|---|---|---|
| Testosterone | 4.5% | 7.79 | 6.75 | Active ingredient | USP |
| Castor oil | 87.5% | 151.37 | 131.25 | Solvent | USP |
| Oleoyl macrogol-glycerides | 4.0% | 6.92 | 6.0 | Wetting agent (hydrophilic oil) | Ph. Eur. |
| Colloidal silicon dioxide | 4.0% | 6.92 | 6.0 | Viscosity increasing agent | USP/NF |

Container

Testosterone gel formulations of the invention are supplied in unit-dose polypropylene syringes. Two syringes of each dosage are packaged in a protective aluminium foil pouch.

**EXAMPLE 2**

**Intranasal Testosterone Gel Formulations**

The testosterone gel formulations of the invention are formulations of testosterone in an intranasal gel proposed for assessing the pharmacokinetic of two different doses of testosterone gel formulations of the invention for testosterone

gel formulations of the invention in hypogonadal men.

The active ingredient, testosterone, is sourced from Bayer Schering. Challenges for nasal delivery include:

- requirements for larger particles than pulmonary administration (i.e., only particles > 10 μm are sufficiently heavy to avoid entering the respiratory tract);
- concentrations must be higher due to the smaller volumes that can be administered;
- rapid clearance of the therapeutic agent from the site of deposition results in a shorter time available for absorption;
- potential for local tissue irritation; and
- limited formulation manipulation possibilities to alter drug delivery profiles.

Testosterone is indicated for TRT in males who are testosterone deficient for any number of reasons, including hypogonadism. The currently available options for administration of testosterone are oral, buccal, injectable, implantable and transdermal (patches and gels).

An intranasal testosterone (3.2%) gel is developed for the treatment of hypogonadism in men and has been administered to hypogonadal men in several clinical trials, see e.g., Mattern, C. et al., 2008 The Aging Male 11(4):171-178 (Dec 2008, which is incorporated herein by reference in its entirety. In a phase II study NCT00975650, which was performed in the U.S. in testosterone deficient men and which was supplemental to the Romanian study reported in *Mattern et al., Supra,* the 3.2% intranasal gel as reported in Mattern et al, *Supra,* failed to reach testosterone plasma levels required by the FDA to support TRT efficacy in testosterone deficient men. The intranasal testosterone gels formulations of the present invention are developed at concentrations of about 4.0% and 4.5% testosterone.

**EXAMPLE 3**

**Overages**

**[Testosterone Gel Formulations of the Invention]**

No overage is added to the formulation. An overage is added to each syringe to account for the gel that is retained in the syringe after dosing. This overage remains consistent at 23 μl, regardless of volume of gel in the syringe. The theoretical fill and dispensed amounts for testosterone gel formulations of the invention are provided below.

| Syringe Dosage | Theoretical Fill Volume (μl) | Theoretical Dispensed Volume (μl) |
|---|---|---|
| 4.0% Testosterone Gel formulation of the Invention | 148 | 125 |
| 4.5% Testosterone Gel formulation of the Invention | 148 | 125 |
| 4.5% Testosterone Gel formulation of the Invention | 173 | 150 |

**EXAMPLE 4**

**Physicochemical and Biological Properties**

**[Testosterone Gel Formulations of the Invention]**

The testosterone bio-adhesive gel formulations of the invention has a viscosity in the range of 3,000 to 10,000 mPa x sec. The viscosity is important because it facilitates maintenance of the gel in the nasal cavity in contact with the nasal mucosa. When the viscosity is less than approximately 3,000 mPa x sec (i.e., 3,000 centipoise), the gel tends to be drawn by gravity out of the nasal cavity.

**EXAMPLE 5**

**Batch Formula**

**[testosterone gel formulations of the invention]**

Three different concentrations of testosterone gel formulations of the invention, 0.15%, 0.45% and 0.6%, are manufactured for the proposed clinical trial. The batch formulae for these batches are presented in Table 5 below.

Table 5: 200 KG Batch Formulae for 4.0% and 4.5% bio-adhesive testosterone gel formulations of the invention at the 8 kg Batch Size

| **Components** | | |
|---|---|---|
| | **4.0%** | **4.5%** |
| Testosterone, USP | 8g | 9g |
| Castor oil, USP | 176g | 175g |
| Oleoyl polyoxylglycerides, Ph. Eur./NF | 8g | 8g |
| Colloidal silicon dioxide, NF | 8g | 8g |

**EXAMPLE 6**

**Manufacturing Process and Process Controls**

**[Testosterone Gel Formulations of the Invention]**

**Material is manufactured according to the following process.**

*Flow Diagram of the Manufacturing Process*

Compound
Activity
Control
Castor Oil (portion 1 and 2)
Testosterone (micronized)
Oleoyl polyoxylglycerides
Colloidal silicon dioxide
Weighing
QC release testing
Castor Oil (portion 1)
Testosterone (micronized)
Pre-mix
Mixing of testosterone in Castor Oil (portion 1)
Mixing I
Castor Oil (portion 2)
Mixing of Pre-mix with castor oil (Portion 2)
Mixing II
Oleoyl polyoxylglycerides
Mixing of Oleoyl polyoxylglycerides with Mixture I
Screening
Check for un-dissolved aggregates
Mixing III
Colloidal Silicon Dioxide
Mixing of Colloidal Silicon Dioxide With screened Mixture II
Visual check - Appearance
Cooling
Discharge (including in line screening)
QC release testing

*Mixing of the Ingredients - Bulk Gel*

The Pre-Mix is prepared by mixing, with a propeller mixer, the full amount of Testosterone with portion 1 of the castor oil for 10 minutes.

Mixture I is prepared by adding the Pre-Mix to the remaining castor oil and mixing for 60 minutes. The product temperature is maintained below 50 °C for the entire mixing process.

The oleoyl polyxoylglycerides are pre-heated to 40 - 50 °C and mixed for 10 minutes before being added to Mixture I. This is identified as Mixture II. It is mixed for 45 minutes while maintaining product temperature below 50 °C. Mixture II is then screened through a sieve to remove any un-dissolved Testosterone aggregates.

Mixture III is prepared by adding the colloidal silicon dioxide to Mixture II and mixing for 15 minutes while maintaining product temperature below 50 °C. A visual check is conducted after this step, to ensure that the gel is clear.

At the completion of mixing the gel is stirred and cooled to a product temperature below 30 °C. The product is then discharged into stainless steel drums and the bulk gel sample is taken for analytical testing.

*Filling and Packaging - Clinical Supplies*

After release of the final gel mixture by the quality control laboratory, the filling and packaging process is carried out by filling a pre-determined volume into the syringe followed by the application of the syringe cap. Two syringes are packaged into a foil pouch.

The syringes are filled using a pipette with the gel taken from a holding tank. The tip of the pipette is discarded after the syringe is filled and the syringe cap is applied. Each syringe is individually labelled.

Following the application of the label, two syringes are packaged in a pre-formed foil pouch and the pouch is

sealed. Each pouch is labelled.

**EXAMPLE 7**

The drug product, TBS-1, is a viscous and thixotropic, oil-based formulation containing solubilized testosterone intended for intranasal application for the treatment of hypogonadism in men.

The drug product is formulated with the following compendial inactive ingredients: castor oil, oleoyl macrogolglycerides, and colloidal silicon dioxide.

To allow for different doses to be administered in the Phase II program, a syringe is used as the unit dose container for the clinical supplies.

The syringes intended for use in the clinical program are needleless and a twist off cap is applied to the end of the syringe. The syringe consists of the syringe barrel and the plunger. The syringe barrel is formed from polypropylene. The plunger is formed from polyethylene. The syringe cap is formed from High Density Polyethylene (HDPE).

New dose formulation of TBS-1 is manufactured for clinical study TBS-1-2010-01 (submitted to the Agency on 07/28/2010 Serial Number 0019). The quantity of testosterone in these formulations is 4.0% and 4.5% along with an adjustment of the amount of castor oil. The precise formulation is listed in Tables 1, 2 and 3. TBS-1 is concentrated so that the same dose is administered intranasally in a smaller volume.

Three different concentrations of TBS-1 gel will be administered in this clinical trial 5.0mg/125µl/ syringe (4.0% gel), 5.6mg/125µl/ syringe (4.5% gel) and 6.75 mg/150 µl/ syringe (4.5% gel). An overage is added to each syringe to account for the gel that is retained in the syringe after dosing. This overage remains consistent regardless of volume of gel in the syringe.

<u>Composition</u>

The compositions of the three different concentrations of the drug product to be administered in this clinical trial are provided in Tables 1, 2 and 3.

**Table 1: Components, Quantity, Quality Standards and Function, TBS-1: 5.0m/125µl/syringe (4.0% gel)**

| Component | Amount (% w/w) | Amount perSyringe (mg) | Amount Delivered per Dose (mg) | Function | Quality Standard |
|---|---|---|---|---|---|
| Testosterone | 4.0% | 5.92 | 5.0 | Active ingredient | USP |
| Castor oil | 88.0% | 130.24 | 110 | Solvent | USP |
| Oleoyl macrogolglycerides | 4.0% | 5.92 | 5.0 | Wetting agent (hydrophilic oil) | Ph. Eur. |
| Colloidal silicon dioxide | 4.0% | 5.92 | 5.0 | Viscosity increasing agent | USP/NF |

**Table 2: Components, Quantity, Quality Standards and Function, TBS-1: 5.6m/125µl/ syringe (4.5% gel)**

| Component | Amount (% w/w) | Amount perSyringe (mg) | Amount Delivered per Dose (mg) | Function | Quality Standard |
|---|---|---|---|---|---|
| Testosterone | 4.5% | 6.66 | 5.63 | Active ingredient | USP |
| Castor oil | 87.5% | 129.5 | 109.37 | Solvent | USP |
| Oleoyl macrogolglycerides | 4.0% | 5.92 | 5.0 | Wetting agent (hydrophilic oil) | Ph. Eur. |
| Colloidal silicon dioxide | 4.0% | 5.92 | 5.0 | Viscosity increasing agent | USP/NF |

**Table 3: Components, Quantity, Quality Standards and Function, TBS-1: 6.75 mg/150 μl/ syringe (4.5% gel)**

| Component | Amount (% w/w) | Amount per Syringe (mg) | Amount Delivered per Dose (mg) | Function | Quality Standard |
|---|---|---|---|---|---|
| Testosterone | 4.5% | 7.79 | 6.75 | Active ingredient | USP |
| Castor oil | 87.5% | 151.37 | 131.25 | Solvent | USP |
| Oleoyl macrogolglycerides | 4.0% | 6.92 | 6.0 | Wetting agent (hydrophilic oil) | Ph. Eur. |
| Colloidal silicon dioxide | 4.0% | 6.92 | 6.0 | Viscosity increasing agent | USP/NF |

Container

TBS-1 gel is supplied in unit-dose polypropylene syringes. Two syringes of each dosage are packaged in a protective aluminium foil pouch.

Control of Drug Products [TBS-1, Gel]

**Specification [TBS-1, Gel]**

The TBS-1 bulk gel is tested to the following specifications for batch release.

**Table 1: Specification for TBS-1 Bulk Gel**

| Test Parameter | Method/Reference | Acceptance Criteria |
|---|---|---|
| **Appearance** | Visually | Slightly yellowish gel |
| **Colour of formulation** | APHA colour reference solution | Colour ≤ 250 |
| **Viscosity** | Rotational viscosimeter USP <911> | 3,000 - 10,000 mPa x sec |
| **Density** | Relative density USP <699> | 0.97 - 1.01 g/cm$^3$ |
| **Identification** | HPLC USP <621><br>UV USP <197U> | Retention time corresponds to reference sample<br>UV spectrum corresponds to reference sample |
| **Impurities** | HPLC USP <621> | Impurity C - Epitestosterone ≤ 0.5%<br>Impurity I - Δ-6-testosterone ≤ 0.2%<br>Each individual unknown impurity ≤ 0.1%<br>Total impurities ≤ 1.0% |
| **Assay** | HPLC USP <621> | 95-105% |

Finished product TBS-1 gel packaged in unit dose syringes is tested to the following specifications for batch release.

**Table 2: Specification for TBS-1 Gel Packaged in Unit Dose Syringes**

| Test Parameter | Method/Reference | Acceptance Criteria |
|---|---|---|
| **Appearance** | Visually | Slightly yellowish gel |
| **Identification** | HPLC USP <621><br>UV USP <197U> | Retention time corresponds to reference sample<br>UV spectrum corresponds to reference sample |
| **Impurities** | HPLC USP <621> | Impurity C - Epitestosterone ≤ 0.5%<br>Impurity I - Δ-6-testosterone ≤ 0.2% |

(continued)

| Test Parameter | Method/Reference | Acceptance Criteria | |
|---|---|---|---|
| | | Each individual unknown impurity<br>Total impurities | ≤ 0.1%<br>≤ 1.0% |
| **Assay** | HPLC USP <621> | 95 - 105% | |
| **Microbial limits** | USP <61> and <62> | TAMC<br>TYMC<br>P. aeruginosa<br>S. aureus | < $10^2$ cfu/g<br>< 10 cfu/g<br>0/g<br>0/g |
| **Mass variation** | USP <905> | Complies with USP <905> | |
| TAMC - total aerobic microbial count<br>TYMC - total combined yeast/mould count | | | |

**Batch Analyses [TBS-1, Gel]**

One preliminary batch (Batch No. 100304), four pilot scale batches (Batch No.ED 187, ED 188, ED 189 and ED 014), two pilot non-GMP batches (NA 090811-1 and NA090723-1) and three commercial scale (Batch 9256, 0823 and 0743) batches of TBS-1 have been produced. Data from the new batches, 0823 and 0743 are described in Tables 4 and 5.

**Table 3: Description of TBS-1 Batches**

| **Formulation** | 4.0% | 4.5% |
|---|---|---|
| **Batch no.** | 0823 | 0743 |
| **Batch size** | 200 kg | 200 kg |
| **Date of manufacture** | June 2010 | June 2010 |
| **Manufacturing site** | Haupt Pharma | Haupt Pharma |
| **Batch no. testosterone** | 89100760 (Bayer/ Schering) | 89100760 (Bayer/ Schering) |
| **Equipment** | Commercial Process | Commercial Process |
| **Filling quantity per container** | 148 □g | 173 □g |

Batch 0743, bulk 4.5% testosterone gel, is filled into two different dosage strengths, 5.6mg (Batch 0943) and 6.75mg (Batch 0744), by varying the weight of the gel in the finish syringe. Batch 0823, bulk 4.0% testosterone gel, is filled as one dose strength, 5.0mg (Batch 0942).

**Table 4: Batch Analysis - TBS-1 Batches 0743 and 0823**

| Test Parameter | Acceptance Criteria | Batch No. 0743 | Batch No. 0823 |
|---|---|---|---|
| Appearance of formulation | Clear, slightly yellowish gel | Complies | Complies |
| Colour | ≤ APHA solution 250 | 150 | 150 |
| Viscosity | 3,000 - 10,000 mPas/30s | 5,217 | 5,086 |
| Density | 0.97 - 1.01 g/cm$^3$ | 0.99 | 0.99 |
| Identification | Retention time corresponds to reference sample<br>UV spectrum corresponds to reference sample | Complies 5.0 min<br>Complies | Complies 5.0 min<br>Complies |
| Impurities | Imputity C - Epitestosterone ≤ 0.5%<br>Impurity I Δ-6-testosterone ≤ 0.2 %<br>Single impurity ≤ 0.1 | 0.3<br>< 0.05<br>< 0.05 | 0.3<br>< 0.05<br>< 0.05 |

(continued)

| Test Parameter | Acceptance Criteria | Batch No. 0743 | Batch No. 0823 |
|---|---|---|---|
| | Total impurities ≤ 1.0 | 0.5 | 0.5 |
| Assay | 95.0 - 105.0% | 100% | 100% |
| Microbial limits | TAMC < $10^2$ cfu/g<br>TYMC < 10 cfu/g<br>*P. aeruginosa* not detected/g<br>*S. aureus* not detected/g | Complies<br>Complies<br>Complies<br>Complies | Complies<br>Complies<br>Complies<br>Complies |
| TAMC - total aerobic microbial count<br>TYMC - total combined yeast/mould count | | | |

**Table 5: Batch Analysis - TBS-1 Batches 00744, 0942 and 0943**

| **Test** | **Acceptance** | **0744** | **0942** | **0943** |
|---|---|---|---|---|
| **Parameter** | **Criteria** | | | |
| **Batch No. Bulk** | | 0743 | 0823 | 0743 |
| **Appearance** | Slightly yellowish gel | Complies | Complies | Complies |
| **Identification** | Retention time corresponds to reference sample<br>UV spectrum corresponds to reference sample | Complies 4.9 min<br>Complies | Complies 5.0 min<br>Complies | Complies 4.9 min<br>Complies |
| **Impurities** | Impurity C ≤ 0.5%<br>Impurity I ≤ 0.2%<br>Each individual unknown impurity ≤ 0.1%<br>Total impurities ≤ 1.0% | 0.3%<br><0.05%<br>0.05%<br>0.3% | 0.3%<br><0.05%<br>0.05%<br>0.3% | 0.3%<br><0.05%<br>0.05%<br>0.3% |
| **Assay** | 95 - 105% | 99% | 100% | 100% |
| **Microbial limits** | TAMC < $10^2$ cfu/g<br>TYMC < 10 cfu/g<br>P. aeruginosa 0/g<br>S. aureus 0/g | Complies<br>Complies<br>Complies<br>Complies | Complies<br>Complies<br>Complies<br>Complies | Complies<br>Complies<br>Complies<br>Complies |
| **Mass variation** | Complies with USP <905> | Complies | Complies | Complies |

**Stability [TBS-1, Gel]**

Stability Summary and Conclusions [TBS-1, Gel]

This section has been amended to include additional data on the on-going stability studies for the initial stability batches and to provide stability data on the drug product in the syringes utilized for the Phase II clinical study. Only the updated sections and new information have been included for review.

All stability studies of TBS-1 gel have been performed by ACC GmbH Analytical Clinical Concepts, Schöntalweg 9-11, 63849 Leidersbach/Aschaffenburg, Germany. Stability studies that meet ICH requirements are on-going.

**Table 1: Stability Studies Conducted in Support of TBS-1**

| Study Type | Container Closure System | Drug Product Batch No. | Storage Conditions | Stability Data available | Study End |
|---|---|---|---|---|---|
| ICH | White LDPE unit dose container; sterile air in pressure cushion; aluminum pouch secondary package (no nitrogen) | ED 187C<br>ED 188<br>ED 189 | 25°C/60% RH<br>40°C/75% RH | 12 months<br>6 months | Study completed |
| ICH | | EI 014 | 25°C/60% RH | 36 months plus a 42 month analysis | Study completed |
| ICH Photostability | | ED 187B | 9 hours ≥ 200 Wh/m$^2$ (300-400 nm) 22 hours 1.2 Mill. Lxh. (400-800 nm) | Full exposure | Study completed |
| Thermal Cycling | | ED 188 | 12 hr -20 °C cycle to 12 hr + 40°C | 4 weeks | Study completed |
| ICH | Syringe with Syringe Cap | Pilot Scale (non GMP)<br>4.0 mg<br>5.5 mg<br>7.0 mg | 25°C/60% RH<br>40°C/75% RH | 6 months | Study completed |
| ICH | Stainless Steel Drum under Nitrogen | 9256 | Ambient temperature | 6 months | On-going |
| ICH | Syringe with Syringe Cap | Bulk 9256<br>9445-4.0 mg<br>9246-5.5 mg<br>9247-7.0 mg | 25°C/60% RH<br>40°C/75% RH | 6 months | On-going |
| ICH | Stainless Steel Drum under Nitrogen | 0743<br>0823 | 25°C/60% RH 40°C/ 75% RH | Initial | Ongoing |
| ICH | Syringe with Syringe Cap | 0943 | 25°C/60% RH<br>40°C/75% RH | initial | Ongoing |

Overall, stability data provided in this section are concluded to support a 24 month "use by" period for TBS-1 stored at controlled room temperature conditions [i.e., 25ºC (77ºF); excursions 15-30ºC (59-86ºF)]. The data also show that special storage conditions for the drug product are not required. The packaging configuration is adequate to protect the drug product from light and the drug product does not degrade or change physically following exposure to temperature cycling stress.

The clinical supplies are applied a 1 year re-test period, when stored at controlled room temperature conditions [i.e., 25ºC (77ºF); excursions 15-30ºC (59-86ºF)], to reflect the duration of the trial and the data available. As additional data is available the re-test period will be extended as appropriate.

**Stability Data [TBS-1, Gel]**

In this section, the updated stability data tables for a commercial size bulk Batch 9256, 0743 and 0823 and

finish product lots 9445, 9446, 9447, 0943 are provided.

A 6 month real time stability program is ongoing on the commercial scale bulk (Batch 9256). A 36 month real time and a 6 month accelerated stability program is ongoing on three different doses of Batch 9256 packaged in 1ml syringes: Batch 9445 4.0 mg (3.2% gel), Batch 9446 5.5 mg (3.2% gel), Batch 9447 7.0 mg (3.2% gel).

A 6 month real time stability program is underway on the commercial scale bulk batch 0743 (4.5% gel) and 0823 (4.0% gel). A 36 month real time and a 6 month accelerated stability program is underway on Batch 0943 (bulk Batch 0743 filled in 1ml syringes).

**Table 2: Stability Schedule for Commerical Scale Bulk TBS-1 gel and Finished Product Filled in 1ml Syringes**

| **Storage Conditions (°C, % RH)** | **Product** | **Completed Test Intervals** (Outstanding Test Intervals) |
|---|---|---|
| Ambient temperature | 9256 | **0m, 3m, 6m** |
| 25 ± 2°C, 60 ± 5 % | 9445 | **0m, 6m** (12m, 24m, 36m) |
| 40 ± 2°C, 75 ± 5% | 9445 | **0m, 3m, 6m** |
| 25 ± 2°C, 60 ± 5 % | 9446 | **0m, 6m** (9m, 18m, 30m, 36m) |
| 40 ± 2°C, 75 ± 5 % | 9446 | **0m, 3m, 6m** |
| 25 ± 2°C, 60 ± 5 % | 9447 | **0m, 6m,** (12m, 24m, 36m) |
| 40 ± 2°C, 75 ± 5 % | 9447 | **0m, 3m, 6m** |
| 25 ± 2°C, 60 ± 5 % | 0943 | **0m,** (3m, 9m, 18m, 30m, 36m) |
| 40 ± 2°C, 75 ± 5 % | 0943 | **0m,** (3m, 6m) |
| Ambient temperature | 0743 | **0m,** (3m, 6m) |
| Ambient temperature | 0823 | **0m,** (3m, 6m) |

**Table 3: Stability Data TBS-1 Batch 9256 (3.2% Bulk Gel) Manufactured July 2009 Stored at Ambient Temperature**

| **Test Parameter** | **Acceptance Criteria** | **07/2009** | **10/2009** | **01/2010** |
|---|---|---|---|---|
| | | **Time 0** | **3 months** | **6 months** |
| Appearance | Slightly yellow gel | Complies | Complies | Complies |
| Colour of formulation | Colour ≤ 250 | 200 | 200 | 200 |
| Viscosity | 3,000 - 10,000 mPa x sec | 5504 | 5325 | 5198 |
| Density | 0.97 - 1.01 g/cm$^3$ | 0.99 | 0.99 | 0.99 |
| Iodine value | FIPO | 78.62 | 77.39 | 76.40 |
| Acid value | FIPO (mg KOH/g) | 1.98 | 2.00 | 2.16 |
| Peroxide value | FIPO (meq $O_2$/kg) | 3.56 | 3.16 | 2.63 |
| Identification | a. Retention time corresponds to RS b. UV spectrum | Complies | Complies | Complies |
| | corresponds to RS | Complies | Complies | Complies |

(continued)

| Test Parameter | Acceptance Criteria | 07/2009 | 10/2009 | 01/2010 |
|---|---|---|---|---|
| | | Time 0 | 3 months | 6 months |
| Impurities | Imp C ≤ 0.5 % | 0.166 % | 0.148 % | 0.189% |
| | Imp I ≤ 0.1 % | < 0.05 % | 0.05 % | <0.05% |
| | Each individual unknown imp. ≤ 0.1 % | 0.064 % | 0.05 % | 0.075% |
| | Total imp. ≤ 1.0 % | 0.230 % | 0.198 % | 0.264% |
| | Imp. D ≤ 0.2 % | < 0.2% | < 0.2 % | 0.2% |
| Assay | 95.0 - 105 % | 99.4 % | 98.3 % | 100.4% |
| Microbial limits | TAMC < $10^2$ cfu/g | < 10 cfu/g | < 10 cfu/g | < 10 cfu/g |
| | TYMC < 10 cfu/g | < 10 cfu/g | < 10 cfu/g | < 10 cfu/g |
| | *S.aureus* 0/g | Not detected/g | Not detected/g | Not detected/g |
| | *P. aeruginosa* 0/g | Not detected/g | Not detected/g | Not detected/g |

**Table 4: Stability Data 4.0 mg TBS-1 Batch 9445 (3.2 % gel) 1 ml Syringe (25 ± 2°C, 60 ± 5 % RH, horizontal)**

| Test Parameter | Acceptance Criteria | Time 0 | 6 months | 12 months |
|---|---|---|---|---|
| Appearance | Slightly yellow gel | Complies | | |
| Colour of formulation | Colour ≤ 250 | 200 | | |
| Dissolution | ≥ 80% within 120 min | 87.8 % within 120 minutes | | |
| Impurities | Imp C ≤ 0.5 % | 0.127 % | | |
| | Imp I ≤ 0.1 % | < 0.05 % | | |
| | Each individual unknown imp. ≤ 0.1 % | < 0.05 % | | |
| | Total imp. ≤ 1.0 % | 0.127% | | |
| | Imp. D ≤ 0.2 % | < 0.2 % | | |
| Assay | 95.0 - 105% | 99.3% | | |
| Microbial limits | TAMC < $10^2$cfu/g | < 10 cfu/g | | |
| | TYMC < 10 cfu/g | < 10 cfu/g | | |
| | *S. aureus* 0/g | Not detected/g | | |
| | *P. aeruginosa* 0/g | Not detected/g | | |

**Table 5: Stability Data 4.0 mg TBS-1 Batch 9445 (3.2 % gel) 1 ml Syringe, (40 ± 2°C, 75 ± 5 % RH, horizontal)**

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | 200 | 200 | |

(continued)

| Test Parameter | Acceptance Criteria | Time 0 | 3 months | 6 months |
|---|---|---|---|---|
| Dissolution | ≥ 80% within 120 min | 87.8 % within 120 minutes | 87.3 % within 120 minutes | |
| Impurities | Imp C ≤ 0.5 % | 0.127 % | 0.128% | |
| | Imp I ≤ 0.1 % | < 0.05 % | < 0.05 % | |
| | Each individual unknown imp. ≤ 0.1 % | < 0.05 % | Rel RT 0.38: 0.177%<br>Rel RT 2.93: 0,066% | |
| | Total imp. ≤ 1.0 % | 0.127% | 0.371% | |
| | Imp. D ≤ 0.2 % | < 0.2 % | < 0.2 % | |
| Assay | 95.0 - 105% | 99.3% | 99.3% | |
| Microbial limits | TAMC < $10^2$ cfu/g | < 10 cfu/g | < 10 cfu/g | |
| | TYMC < 10 cfu/g | < 10 cfu/g | < 10 cfu/g | |
| | *S. aureus* 0/g | Not detected/g | Not detected/g | |
| | *P. aeruginosa* 0/g | Not detected/g | Not detected/g | |

**Table 6: Stability Data 5.5 mg TBS-1 Batch 9446 (3.2 % gel) 1 ml Syringe (25 ± 2°C, 60 ± 5 % RH, horizontal)**

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | 200 | 200 | |
| Dissolution | ≥ 80% within 120 min | 86.8 % within 120 minutes | 83.6 % within 120 minutes | |
| Impurities | Imp C ≤ 0.5 % | 0.125 % | 0.126% | |
| | Imp I ≤ 0.1 % | < 0.05 % | < 0.05 % | |
| | Each individual unknown imp. ≤ 0.1 % | < 0.05 % | < 0.05 % | |
| | Total imp. ≤ 1.0 % | 0.125% | 0.126% | |
| | Imp. D ≤ 0.2 % | < 0.2 % | < 0.2 % | |
| Assay | 95.0 - 105 % | 99.1% | 99.4% | |
| Microbial limits | TAMC < $10^2$ cfu/g | < 10 cfu/g | < 10 cfu/g | |
| | TYMC < 10 cfu/g | < 10 cfu/g | < 10 cfu/g | |
| | *S. aureus* 0/g | Not detected/g | Not detected/g | |
| | *P. aeruginosa* 0/g | Not detected/g | Not detected/g | |

**Table 7: Stability Data 5.5 mg TBS-1 Batch 9446 (3.2 % gel) 1 ml Syringe (40 ± 2°C, 75 ± 5 % RH, horizontal)**

| Test Parameter | Acceptance Criteria | Time 0 | 3 months | 6 months |
|---|---|---|---|---|
| Appearance | Slightly yellow gel | Complies | Complies | |

(continued)

| Test Parameter | Acceptance Criteria | Time 0 | 3 months | 6 months |
|---|---|---|---|---|
| Colour of formulation | Colour ≤ 250 | 200 | 200 | |
| Dissolution | ≥ 80% within 120 min | 86.8 % within 120 minutes | 86.8 % within 120 minutes | |
| Impurities | Imp C ≤ 0.5% | 0.125 % | 0.127% | |
| | Imp I ≤ 0.1 % | < 0.05 % | < 0.05 % | |
| | Each individual unknown imp. ≤ 0.1 % | < 0.05 % | Rel RT 0.38: 0.102%<br>Rel RT 3.01: 0.070 | |
| | Total imp. ≤ 1.0 % | 0.125% | 0.299% | |
| | Imp. D ≤ 0.2 % | < 0.2 % | < 0.2 % | |
| Assay | 95.0 - 105% | 99.1% | 97.9% | |
| Microbial limits | TAMC < $10^2$ cfu/g | < 10 cfu/g | < 10 cfu/g | |
| | TYMC < 10 cfu/g | < 10 cfu/g | < 10 cfu/g | |
| | *S. aureus* 0/g | Not detected/g | Not detected/g | |
| | *P.aeruginosa* 0/g | Not detected/g | Not detected/g | |

**Table 8: Stability Data 7.0 mg TBS-1 Batch 9447 (3.2 % gel) 1 ml Syringe (25 ± 2°C, 60 ± 5 % RH, horizontal)**

| Test Parameter | Acceptance Criteria | Time 0 | 6 months | 12 months |
|---|---|---|---|---|
| Appearance | Slightly yellow gel | Complies | | |
| Colour of formulation | Colour ≤ 250 | 200 | | |
| Dissolution | ≥ 80% within 120 min | 83.5 % within 120 minutes | | |
| Impurities | Imp C ≤ 0.5 % | 0.132% | | |
| | Imp I ≤ 0.1 % | < 0.05 % | | |
| | Each individual unknown imp. ≤ 0.1 % | < 0.05 % | | |
| | Total imp. ≤ 1.0 % | 0.132% | | |
| | Imp. D ≤ 0.2 % | < 0.2 % | | |
| Assay | 95.0 - 105 % | 98.7% | | |
| Microbial limits | TAMC < $10^2$ cfu/g | < 10 cfu/g | | |
| | TYMC < 10 cfu/g | < 10 cfu/g | | |
| | *S. aureus* 0/g | Not detected/g | | |
| | *P. aeruginosa* 0/g | Not detected/g | | |

**Table 9: Stability Data 7.0 mg TBS-1 Batch 9447 (3.2 % gel) 1 ml Syringe (40 ± 2°C, 75 ± 5 % RH., horizontal)**

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | 200 | 200 | |
| Dissolution | ≥ 80% within 120 min | 83.5 % within 120 minutes | 85.4 % within 120 minutes | |
| Impurities | Imp C ≤ 0.5 % | 0.132 % | 0.132% | |
| | Imp I ≤ 0.1 % | < 0.05 % | < 0.05 % | |
| | Each individual unknown imp. ≤ 0.1 % | < 0.05 % | Rel RT 0.37: 0.074% Rel RT 3.13: 0.069 | |
| | Total imp. ≤ 1.0 % | 0.132% | 0.275% | |
| | Imp. D ≤ 0.2 % | < 0.2 % | < 0.2 % | |
| Assay | 95.0 - 105% | 98.7% | 99.1% | |
| Microbial limits | TAMC < $10^2$ cfu/g | < 10 cfu/g | < 10 cfu/g | |
| | TYMC < 10 cfu/g | < 10 cfu/g | < 10 cfu/g | |
| | *S. aureus* 0/g | Not detected/g | Not detected/g | |
| | *P. aeruginosa* 0/g | Not detected/g | Not detected/g | |

**Table 10: Stability Data 5.6 mg TBS-1 Batch 0943 (4.5% gel) 1 ml Syringe (25 ± 2°C, 60 ± 5 % RH, horizontal)**

| Test Parameter | Acceptance Criteria | Time 0 | 3 months | 6 months |
|---|---|---|---|---|
| Appearance | Slightly yellow gel | Complies | | |
| Colour of formulation | Colour ≤ 250 | Complies | | |
| Impurities | Imp C ≤ 0.5 % | 0.3% | | |
| | Imp I ≤ 0.1 % | < 0.05 % | | |
| | Each individual unknown imp. ≤ 0.1 % | < 0.05 % | | |
| | Total imp. ≤ 1.0 % | 0.3 | | |
| Assay | 95.0 - 105% | 100% | | |
| Microbial limits | TAMC < $10^2$ cfu/g | Complies | | |
| | TYMC < 10 cfu/g | Complies | | |
| | *S. aureus* 0/g | Complies | | |
| | *P. aeruginosa* 0/g | Complies | | |

**Table 11: Stability Data 5.6 mg TBS-1 Batch 0943 (4.5% gel) 1 ml Syringe (40 ± 2°C, 75 ± 5 % RH, horizontal)**

| Test Parameter | Acceptance Criteria | Time 0 | 3 months | 6 months |
|---|---|---|---|---|
| Appearance | Slightly yellow gel | Complies | | |
| Colour of formulation | Colour ≤ 250 | Complies | | |

(continued)

| Test Parameter | Acceptance Criteria | Time 0 | 3 months | 6 months |
|---|---|---|---|---|
| Impurities | Imp C ≤ 0.5 % | 0.3% | | |
| | Imp I ≤ 0.1 % | < 0.05 % | | |
| | Each individual unknown imp. ≤ 0.1 % | < 0.05 % | | |
| | Total imp. ≤ 1.0 % | 0.3 | | |
| Assay | 95.0 - 105% | 100% | | |
| Microbial limits | TAMC < $10^2$ cfu/g | Complies | | |
| | TYMC < 10 cfu/g | Complies | | |
| | *S. aureus* 0/g | Complies | | |
| | *P. aeruginosa* 0/g | Complies | | |

**Table 12: Stability Data TBS-1 Batch 0743 (4.5 % gel) Bulk Stored at Ambient Temperature**

| Test Parameter | Acceptance Criteria | Time 0 | 3 months | 6 months |
|---|---|---|---|---|
| Appearance | Slightly yellow gel | Complies | | |
| Colour of formulation | Colour ≤ 250 | Complies | | |
| Impurities | Imp C ≤ 0.5 % | 0.3% | | |
| | Imp I ≤ 0.1 % | < 0.05 % | | |
| | Each individual unknown imp. ≤ 0.1 % | < 0.05 % | | |
| | Total imp. ≤ 1.0 % | 0.3 | | |
| Assay | 95.0 - 105% | 100% | | |
| Microbial limits | TAMC < $10^2$ cfu/g | Complies | | |
| | TYMC < 10 cfu/g | Complies | | |
| | *S. aureus* 0/g | Complies | | |
| | *P. aeruginosa* 0/g | Complies | | |

**Table 14: Stability Data TBS-1 Batch 0823 (4.5 % gel) Bulk Stored at Ambient Temperature**

| Test Parameter | Acceptance Criteria | Time 0 | 3 months | 6 months |
|---|---|---|---|---|
| Appearance | Slightly yellow gel | Complies | | |
| Colour of formulation | Colour ≤ 250 | Complies | | |
| Impurities | Imp C ≤ 0.5 % | 0.3% | | |
| | Imp I ≤ 0.1 % | < 0.05 % | | |
| | Each individual unknown imp. ≤ 0.1 % | < 0.05 % | | |
| | Total imp. ≤ 1.0 % | 0.3 | | |
| Assay | 95.0 - 105% | 100% | | |

(continued)

| Test Parameter | Acceptance Criteria | Time 0 | 3 months | 6 months |
|---|---|---|---|---|
| Microbial limits | TAMC < $10^2$ cfu/g | Complies | | |
| | TYMC < 10 cfu/g | Complies | | |
| | *S. aureus* 0/g | Complies | | |
| | *P. aeruginosa* 0/g | Complies | | |

**EXAMPLE 8**

This is a Phase 2 study designed to investigate the intranasal absorption of 4% of the drug three times a day and 4.5% of the drug administered twice a day and three times a day, and to compare the absorption from the previous study in the same subjects that responded with a 3.2% testosterone gel. In the previous study, Nasobol-01-2009, a 3.2% Testosterone gel is used to deliver 4.0 mg, 5.5 mg and 7.0 mg of Testosterone intra-nasally using gel volumes of 125 μL, 172 μL and 219 μL, respectively. In this study, 5.0 mg, 5.65 mg and 6.75 mg of Testosterone is administered in gel volumes of 125 μL, 125 μL, and 150 μL, respectively. This study allowed investigating the delivery of similar Testosterone amounts in much smaller volumes.

In this open label study, subjects are equally randomized into three treatment arms. The treatments are administered for one week, in a parallel design. At the end of one week, the three treatments are compared by conducting a 24-hour pharmacokinetic investigation of the systemic absorption of the drug product testosterone and its two physiological metabolites dihydrotestosterone and estradiol.

**8. Study Objectives**

8.1 Primary Objective

The primary objective of this study is to determine the bioavailability through PK analysis of a 4% TBS-1 gel (applied three times a day) and 4.5% TBS-1 gel (applied twice a day and three times a day) in hypogonadal men.

8.2 Secondary Objective

The secondary objective of the study is to establish the safety profile for TBS-1.

**9. Investigational Plan**

9.1 Overall Study Design and Plan Description

This is an open label, randomized, balanced, three treatment (4.0% t.i.d. 4.5% b.i.d. and 4.5% t.i.d.), parallel design, pharmacokinetic study of TBS-1, administered intra-nasally. The serum concentrations of total Testosterone, Dihydrotestosterone and Estradiol are measured using validated LC/MS methods.

Hypogonadal subjects are required to visit the Clinic on three (3) occasions, of which one (1) visit (Visit 3) required an overnight stay for the previously described 24-hour pharmacokinetic profile.

The following pharmacokinetic parameters are determined for all subjects:

- $AUC_{0-T}$, $C_{avg}$, $C_{min}$, $C_{max}$, $t_{max}$, PTF and PTS means and standard error of the means are calculated for the 24-hour interval.

- The percentage of subjects with a $C_{avg}$ for Testosterone, Dihydrotestosterone and Estradiol, below, within and above the Reference Range for the respective analyte is calculated.

Erythrocytosis, anemia and infections are monitored by measuring complete blood counts at screening and the Close-Out visit.

It is planned to enroll approximately 30 subjects. Twenty-two (22) subjects completed the study. Study participation is 2 to 3 weeks.

## 9.2 Discussion of Study Design

Testosterone therapy for hypogonadal men should correct the clinical abnormalities of Testosterone deficiency, including disturbances of sexual function. Testosterone decreases body fat and increases lean muscle mass and bone density with minimal adverse effects.

There are several Testosterone replacement products available, which can be given intra-muscularly, orally, as a buccal tablet to the gums, or topically as a patch or gel. Current replacement therapies have certain drawbacks. Testosterone injections show wide fluctuations in serum Testosterone levels often at values above the reference range (5). Testosterone patches have a high rate of skin irritation (6,7). Testosterone gels although popular in North America are not always convenient and have a risk of skin-to-skin transfer to family members (8,9). Oral Testosterone undecanoate needs to be administered with a high fat meal and levels obtained are often low (10-12).

Intra-nasal administration of a new formulation of Testosterone (TBS-1) has been shown to be effectively absorbed and shows excellent potential as a therapeutic product in the treatment of male hypogonadism (13). The nasal mucosa offers an alternative route of administration that is not subject to the first pass effect, has high permeability and ease of administration with rapid absorption into the systemic circulation producing high plasma levels similar to those observed after intravenous administration.

The advantages of the Testosterone nasal gel, when compared to other formulations, are the following: Convenient application form permitting inconspicuous use, the much smaller amount of active ingredient needed for the subject, and knowing that this type of administration is less likely to contaminate other family members (wife and children).

Several studies have indicated the utility of testosterone administration using the nasal gel. The prior study conducted in 2009 is to demonstrate the efficacy of TBS-1 in the treatment of hypogonadal men requiring Testosterone replacement therapy. Efficacy is determined by establishing an optimal pharmacokinetic profile for serum Testosterone levels following a multiple-dose b.i.d. dosing profile for TBS-1, using three different strengths of Testosterone (8.0 mg, 11.0 mg and 14.0 mg) and comparing it to that of the active control, Androderm®. The secondary objective of this study is to establish a safety profile for TBS-1. This is to be achieved by monitoring adverse and serious adverse events during the course of the entire study, and comparing various safety parameters at follow-up to those obtained at baseline. These safety parameters consisted of vital signs, complete blood counts, a chemistry profile, an endocrine profile, and urinalysis. In addition, changes to the nasal mucosa and to the prostate at follow up are compared to baseline.

An important advantage of the power of the dose finding design of this study is that it minimizes the subject selection bias and the different host groups often observed in sequential study designs.

The three clinical sites are monitored by Schiff & Company to ensure the safety of the Subjects and performance of the clinical study according to ICH E6 and FDA guidelines.

A central laboratory is used for the analysis of hematology and biochemistry parameters in order to obtain consistent and unbiased laboratory results. A second central laboratory is used for the PK analysis.

The following are the specific activities in the study design during the subject visits:

| PROCEDURE | | | | |
|---|---|---|---|---|
| | In/Ex PERIOD | Day 1 | Day 7 | Day 8 |
| Visit Number: | 1 | 2 | 3 | |
| Informed Consent[1] | x | | | |
| Medical History | x | | | |
| Physical Exam* & Vital Signs | x | x | x | x |
| Subject Demographic Data | x | | | x |
| PROCEDURE Otorhinolaryngological Exam | x | | | x |
| Prostate Exam[2] | x | | | x |
| Chemistry Profile[3] | x | | | x |
| Hematology Profile[4] | x | | | x |
| Urinalysis[5] | x | | | x |
| Serum PSA | x | | | x |
| Hepatitis B, C, & HIV Testing | x | | | |
| Urine Drug Screen[6] | x | | | |

(continued)

| PROCEDURE | | | | |
|---|---|---|---|---|
| | In/Ex PERIOD | Day 1 | Day 7 | Day 8 |
| Visit Number: | 1 | 2 | 3 | |
| Ethanol Test[7] | x | | | |
| Hemoglobin $A_1c$ | x | | | |
| Serum Testosterone[8] | x | | | |
| Serum T, DHT & Estradiol | | x | | |
| Serum T, DHT & Estradiol PK | | | x | |
| Concomitant Medications | x | x | x | x |
| Adverse Event Recording | x | x | x | x |

* Physical Exam on Screen and Day 8 only.
[1] Informed consent will be signed prior to Screening Visit 1 In/Ex Period: Inclusion, and Exclusion Period
[2] If subject had a prior normal prostate exam in Nasobol-01-2009, it will not be required.
[3] Chemistry Profile: Na/K, Glucose, Urea, Creatinine, Total Bilirubin, Albumin, Calcium, Phosphate, Uric Acid, AST, ALT, ALP, GGT and CK.
[4] Complete Blood Count and Differential.
[5] Urine dipstick (no microscopic).
[6] Cocaine, Cannabinoids, Opiates, Benzodiazepines.
[7] Urine alcohol by dipstick.
[8] Serum Testosterone, Dihydrotestosterone & Estradiol will be measured by a reference lab using a validated LC-MS/MS method, for T and DHT and a validated LC-MS/MS or immunoassay method, for Estradiol.

Screening visit 1

- Subjects, after having voluntarily signed the Informed Consent Form, are interviewed by the Clinical Investigator or his/her designee Physician/Nurse Practitioner who took the medical and physical history, record demographic data, and performed a routine physical examination. Body weight and Height is measured and BMI calculated. Vital signs (seated 5 minutes) are measured (Blood Pressure, Heart Rate, Respiratory Rate, and Body Temperature).

- If the subject had a normal digital rectal exam of the prostate in the recent Nasobol-01-2009 trial, it is not repeated.

- The Clinical Investigator assessed the subject study eligibility based on the inclusion/exclusion criteria, and eligible subjects that are currently on Testosterone replacement therapy needed to undergo a wash-out period; four (4) weeks for depot products administered intra-muscularly (e.g., Testosterone enanthate 200 mg/mL), and two (2) weeks for products administered orally or topically (patch, gel, or buccal). At the end of the wash-out period, subjects are to return to have their serum Testosterone measured.

- Treatment naïve subjects did not require a wash-out period.

- Blood for serum Testosterone is drawn under fasting conditions, at 0900 h $\pm$ 30 minutes. The serum Testosterone level must be > 150 ng/dL, and < 300 ng/dL.

- Blood is drawn for Clinical Laboratory investigations after an overnight fast (8-10 hour fast) and included the following:

    - Complete Blood Count (Hemoglobin, Hematocrit, MCV, MCHC, RBC, WBC & Differential)

    - Clinical Chemistry profile (Na/K, Glucose, Urea, Creatinine, Total Bilirubin, Albumin, Calcium, Phosphate, Uric Acid, AST, ALT, ALP, GGT and CK)

- ◦ Serum PSA
- ◦ Testing for HBV, HCV and HIV (Hepatitis B surface antigen, Hepatitis C antibody, HIV antibodies)
- ◦ Whole blood sample for Hemoglobin A1c
- ◦ Urine for dipstick urinalysis
- ◦ Urine for Drug screen (Cocaine, Cannabis, Opiates and Benzodiazepines). Subjects with positive test are not enrolled, unless the positive test is due to interference from a drug prescribed by a Physician
- ◦ Urine for alcohol testing

- The otorhinolaryngologic nasal endoscopy examination is done by an ENT specialist.
- Subjects that met all of the inclusion and exclusion criteria are enrolled into the study and randomized into one of three treatment groups (A, B or C).

Visit 2 (day 1)

- Subjects arrived at the Clinic under fasting conditions (6 - 8 hour fast) at 2000 hours or earlier.
- Instructions are given to subjects on the proper technique for intra-nasal dosing of TBS-1 .
- Blood is drawn at 2045 hours for baseline serum Testosterone, Dihydrotestosterone, and Estradiol concentrations.
- Vital Signs (seated 5 minutes) are measured (Blood Pressure, Heart Rate, Respiratory Rate, and Body Temperature) to establish a baseline.
- Subjects are given a one week supply of pouches: 18 pouches for treatment A, 12 pouches for treatment B, and 18 pouches for treatment C. Pouches required for dosing during the pharmacokinetic profile remained with the Clinical Investigator. Each pouch contained two syringes pre-filled with TBS-1 gel for treatment A, B, or C.
- Subjects administered their first dose of TBS-1 at 2100 hours according to their treatment group.
- Vital Signs are measured at 2200 hours and subjects are sent home with their supply of pouches for their treatment group.

Telephone check (day 4)

On Day 4, all subjects are called to check compliance of study drug administration, compliance to abstention from alcohol for 48 hours, and to document any adverse events that may have occurred. Subjects are reminded to bring in all syringes for counting at Visit 3.

Visit 3 (day 7)

- Subjects arrived at the Clinic under fasting conditions (6 - 8 hour fast) at 2000 hours or earlier.
- Blood is drawn at 2045 hours for baseline serum Testosterone, Dihydrotestosterone, and Estradiol concentrations.
- Subject underwent a 24-hour pharmacokinetic profile immediately after the 2100 hour dosing. Vital signs are recorded hourly for two hours post dosing.
- Safety parameters are recorded.

- Subjects remained fasting for two hours post dose and then given supper. After supper, the subjects again fasted overnight and remained fasting until 0900 hours on Day 8. Lunch and supper on Day 8 occurred at the regular times and are not subject to fasting conditions.

Pharmacokinetic blood draws

- Administration of the drug should have occurred at ± 5 minutes from the indicated time (2100 h and 0700 h for b.i.d. dosing and 2100 h, 0700 h and 1300 h for t.i.d. dosing).

- Blood draws should have been within ± 5 minutes from the indicated times when blood draw intervals are ≤ 30 minutes and within ± 15 minutes when blood draws are > 30 minutes.

- Treatment A: Blood draws for serum Testosterone, Dihydrotestosterone, and Estradiol measurements: Blood draws for t.i.d. dosing are done at the following times after the 2100 hour drug administration; 0.33, 0.66, 1.0, 1.5, 2.0, 3.0, 6.0, 9.0, 9.75, 10.33, 10.66, 11.0, 11.5, 12.0, 13.0, 14.0, 15.75, 16.33, 16.66, 17.0, 17.5, 18.0, 20.0, 22.0 and 24.0 hours, (total blood draws; 25 + baseline).

- Treatment B: Blood draws for serum Testosterone, Dihydrotestosterone, and Estradiol measurements: Blood draws for b.i.d. dosing are done at the following times after the 2100 hour drug administration; 0.33, 0.66, 1.0, 1.5, 2.0, 3.0, 6.0, 9.0, 9.75, 10.33, 10.66, 11.0, 11.5, 12.0, 13.0, 16.0, 19.0, 22.0, and 24.0 hours, (total blood draws; 19 + baseline).

- Treatment C: Blood draws for serum Testosterone, Dihydrotestosterone, and Estradiol measurements: Blood draws for t.i.d. dosing are done at the following times after the 2100 hour drug administration; 0.33, 0.66, 1.0, 1.5, 2.0, 3.0, 6.0, 9.0, 9.75, 10.33, 10.66, 11.0, 11.5, 12.0, 13.0, 14.0, 15.75, 16.33, 16.66, 17.0, 17.5, 18.0, 20.0, 22.0 and 24.0 hours, (total blood draws; 25 + baseline).

- The last blood draw in the pharmacokinetic profile included enough blood to measure the clinical laboratory safety parameters required at Close-out.

Visit 3 (day 8), close out visit

Subjects underwent the following assessments:

- A routine physical examination including vital signs (Blood Pressure, Heart Rate, Respiratory Rate, and Body Temperature).

- Otorhinolaryngologic nasal examination.

- Blood sample is taken for a Complete Blood Count (Hemoglobin, Hematocrit, RBC, WBC and differential, MCV, MCHC).

- Blood sample for Chemistry Profile (Na/K, glucose, urea, creatinine, calcium, phosphate, uric acid, total bilirubin, albumin, AST, ALT, ALP, GGT, and CK).

- Blood sample for PSA.

- Urine sample for dipstick urinalysis.

9.3 Selection of Study Population

Subjects are included in the study according to the following inclusion/exclusion criteria:

**9.3.1 Inclusion Criteria**

1. Males who are responders to high-dose intra-nasal Testosterone in the Nasobol-01-2009 trial.
2. Written informed consent.
3. Males between 18 and 80 years of age.
4. Men with primary or secondary hypogonadism and a morning (0900 h ± 30 minutes) serum Testosterone levels >150 ng/dL and ≤ 300 ng/dL, on blood drawn under fasting conditions.
5. BMI between 18.5 - 35 kg/m$^2$.
6. All clinical laboratory assessments at the Screening Visit are from blood drawn or urine collected following an overnight fast (10 hours), and are within ±15% of the Clinical Laboratory's reference range, except for serum Testosterone.
7. Normal Otorhinolaryngological nasal endoscopy examination. See Appendix 16.1.1 for exclusion criteria pertaining to endoscopy examination.
8. Prior, normal prostate examination (no palpable prostatic mass) from the Nasobol-01-2009 trial.
9. A serum PSA ≤ 4.0 ng/mL.

**9.3.2 Exclusion Criteria**

1. Significant inter-current disease of any type, in particular liver, kidney, or heart disease, any form of diabetes mellitus or psychiatric illness.
2. Limitations in mobility, defined as having difficulty walking two blocks on a level surface or climbing 10 steps
3. Hematocrit > 54% at screening.
4. History of cancer, excluding skin cancer.
5. History of nasal surgery, specifically turbinoplasty, septoplasty, rhinoplasty, "nose job", or sinus surgery.
6. Subject with prior nasal fractures.
7. Subject with active allergies, such as rhinitis, rhinorrhea, and nasal congestion.
8. Subject with mucosal inflammatory disorders, specifically pemphigus, and Sjogren's syndrome.
9. Subject with sinus disease, specifically acute sinusitis, chronic sinusitis, or allergic fungal sinusitis.
10. History of nasal disorders (e.g., polyposis, recurrent epistaxis (> 1 nose bleed per month), abuse of nasal decongestants) or sleep apnea.
11. Subject using any form of intra-nasal medication delivery, specifically nasal corticosteroids and oxymetazoline containing nasal sprays (e.g., Dristan 12-Hour Nasal Spray).
12. History of severe adverse drug reaction or leucopenia.
13. History of abnormal bleeding tendencies or thrombophlebitis unrelated to venipuncture or intravenous cannulation.
14. Positive test for Hepatitis B, Hepatitis C, or HIV.
15. History of asthma and on-going asthma treatment.
16. History of sleeping problems.
17. Smokers (> 10 cigarettes per day).
18. Regular drinkers of more than four (4) units of alcohol daily (1 unit = 300 mL beer, 1 glass wine, 1 measure spirit) or those that may have difficulty in abstaining from alcohol during the 48 hours prior to the 24-hour blood sampling visit.
19. History of, or current evidence of, abuse of alcohol or any drug substance, licit or illicit; or positive urine drug and alcohol screen for drugs of abuse and alcohol.
20. Current treatment with androgens (e.g., Dehydroepiandrostenedione, Androstenedione) or anabolic steroids (e.g., Testosterone, Dihydrotestosterone).
21. Treatment with Estrogens, GnRH antagonists, or Growth Hormone, within previous 12 months.
22. Treatment with drugs which interfere with the metabolism of Testosterone, such as Anastrozole, Clomiphene, Dutasteride, Finasteride, Flutamide, Ketoconazole, Spironolactone and Testolactone.
23. Androgen treatment within the past four weeks (intramuscular, topical, buccal, etc.).
24. Subject with poor compliance history or unlikely to maintain attendance.
25. Participation in any other research study during the conduct of this study or 30 days prior to the initiation of this study, with the exception of Nasobol-01-2009.
26. Blood donation (usually 550 mL) at any time during this study, and within the 12 week period before the start of this study.

### 9.3.3 Removal of Subjects from Therapy or Assessment

Subjects are informed that they are free to withdraw from the study at any time without having to give reasons for their withdrawal, and without consequences for their future medical care. They are asked to inform the investigator immediately of their decision. The subject's participation in the study may have been discontinued for any of the following reasons:

- Subject's own wish.
- Significant non compliance with the study protocol and procedures.
- Inter-current illness which interferes with the progress of the study.
- Intolerable adverse event, including clinically significant abnormal laboratory findings, where, in the opinion of the Clinical Investigator, these could interfere with the subject's safety.
- Clinical Investigator's decision that the withdrawal from the study is in the best interest of the subject.

The Clinical Investigator had the right to terminate a study prematurely for safety reasons, after having informed and consulted with the Sponsor. The Sponsor had the right to terminate the study earlier if the clinical observations collected during the study suggested that it might not be justifiable to continue or for other reasons as described in the contract between Sponsor and the clinical sites (e.g., administrative, regulatory, etc.). However this is not necessary. There are no premature terminations or drops outs from the study.

## 9.4 Treatments

### 9.4.1 Treatments Administered

Subjects are centrally randomized to the following treatment groups in order to balance the numbers equally within the groups across the three centers:

- Treatment A (n=10): TBS-1 syringes pre-filled with 125 μL 4.0% gel to deliver 5.0 mg of Testosterone per nostril (intra-nasal) given t.i.d. at 2100, 0700, and 1300 hours. (total dose 30 mg/day)
- Treatment B (n=10): TBS-1 syringes pre-filled with 150 μL 4.5% gel to deliver 6.75 mg of Testosterone per nostril (intra-nasal) given b.i.d. at 2100 and 0700 hours. (total dose 27.0 mg/day)
- Treatment C (n=10): TBS-1 syringes pre-filled with 125 μL 4.5% gel to deliver 5.625 mg of Testosterone per nostril (intra-nasal) given t.i.d. at 2100, 0700, and 1300 hours. (total dose 33.75 mg/day)

### 9.4.2 Identity of Investigational Products

Name of the drug: TBS-1 (Syringes are pre-filled to contain 5.0 mg, 5.625 mg, and 6.75 mg of Testosterone/syringe).
Pharmaceutical form: Gel for nasal administration.
Content: Active ingredient: Testosterone.
Excipients: Silicon dioxide, castor oil, Labrafil®.
Mode of administration: Nasally, as a single dose to each nostril.
Manufacturer: Haupt Pharma Amareg.
Batch numbers: 0744, 0942, and 0943
Storage conditions: Between 20 - 25ºC.

**Packaging**

The TBS-1 study drug is delivered to the clinical trial site as a ready-for-use syringe in a foil pouch (two syringes per pouch). Examples of Syringe and Pouch Labels are described in Appendix 4 of the protocol.

### 9.4.3 Method of Assigning Subjects to Treatment

Subjects who met the entry criteria are assigned randomly on a 1:1:1 basis to one of the three treatment groups. At Screening, each subject is assigned a subject number by site in sequential order. Subject numbers consisted of 5 digits. The first 2 digits reflected the site number assigned to the investigator, followed by a 3-digit subject number. For example, 01-001 indicates site (01) and the first subject (001).
The subject number was used to identify the subject throughout the study and was entered on all documents. The same

subject number was not assigned to more than one subject.

### 9.4.4 Selection of Doses in the Study

In a previous study, Nasobol-01-2009, a 3.2% Testosterone gel is used to deliver 4.0 mg, 5.5 mg and 7.0 mg of Testosterone intra-nasally using gel volumes of 125 μL, 172 μL and 219 μL, respectively. In this study, 5.0 mg, 5.65 mg and 6.75 mg of Testosterone are administered in gel volumes of 125 μL, 125 μL, and 150 μL, respectively. This study permits the investigation of the delivery of similar Testosterone amounts in much smaller volumes.

### 9.4.5 Selection and Timing of Dose for Each Subject

This was based on the results of the prior study.

### 9.4.6 Blinding

There is no blinding, because this is an open label study. The rationale for not blinding is that analytical endpoints, which are quantitative rather than qualitative are measured, and are not subject to any bias being introduced by the subjects or the Investigators.

### 9.4.7 Prior and Concomitant Therapy

The following medications are prohibited during the course of the study:
Subject using any form of intra-nasal medication delivery, specifically nasal corticosteroids and oxymetazoline containing nasal sprays (e.g., Dristan 12-Hour Nasal Spray).

Current treatment with androgens (e.g., Dehydroepiandrostenedione, Androstenedione) or anabolic steroids (e.g., Testosterone, Dihydrotestosterone). Treatment with Estrogens, GnRH antagonists, or Growth Hormone, within previous 12 months.

Treatment with drugs which interfere with the metabolism of Testosterone, such as; Anastrozole, Clomiphene, Dutasteride, Finasteride, Flutamide, Ketoconazole, Spironolactone and Testolactone.

Androgen treatment within the past four weeks (intramuscular, topical, buccal, etc.).

### *9.4.8 Treatment Compliance*

All drugs are dispensed in accordance with the protocol. It is the Principal Investigator's responsibility to ensure that an accurate record of drugs issues and return is maintained. At the end of the study, the used original packages are returned to the sponsor for destruction. Drug accountability is verified by the monitors during the course of the study and prior to destruction of remaining study drugs.
During Visit 2, the subjects are given a one-week supply of pouches; 18 pouches for treatment A, 12 pouches for treatment B, and 18 pouches for treatment C. Each pouch contained two syringes prefilled with TBS-one gel for treatment A, B, or C. The subjects are instructed on how to administer the gel and are also given a diary to indicate the times of administration at their home.

### 9.5 Efficacy and Safety Variables

### 9.5.1 Efficacy and Safety Measurements Assessed

The primary efficacy parameter is the AUC is obtained in the 24 hours post administration of TBS-1. From the AUC the 24 hour $C_{avg}$ is calculated.

- Area under the concentration curve (AUC) for both b.i.d. and t.i.d. dosing is determined for the 0 to 24 hour time interval using the trapezoidal rule.
- The average concentration in the dosing interval ($C_{avg}$) is calculated from the AUC using the following formula: $C_{avg} = AUC_{0-\tau} / \tau$, with $\tau$ = dosing interval time.
- Peak Trough Fluctuation (PTF) and Peak Trough Swing (PTS) is calculated as follows:
    - 

$$PTF = (C_{max} - C_{min}) / C_{avg}$$

◦

$$PTS = (C_{max} - C_{min}) / C_{min}$$

- $C_{min}$, $C_{max}$, and $t_{max}$ is taken from the actual measured values. Values are determined relative to the Testosterone administration time in treated subjects.
- The percent of subjects with 24 hour $C_{avg}$ values for serum Testosterone, DHT and Estradiol concentration above, within, and below the respective reference range are calculated.
- Additional exploratory analyses of PK parameters may have been performed as necessary.

**Analysis of Safety Data**

Erythrocytosis, anemia, and infections are monitored by measuring complete blood counts at screening, and the Close-Out visit. An Otorhinolaryngological physician examined subjects and identifies any clinically significant changes to the nasal mucosa at follow up compared to baseline.

Clinical chemistry and urinalysis testing at Screening Visit 1 and at Close Out are assessed, hypo or hyperglycemia, renal function, liver function (hepato-cellular or obstructive liver disease), skeletal/heart muscle damage, and changes in calcium homeostasis.

Serum PSA is measured as a cautionary measure to measure possible changes to the prostate, although changes to the prostate and to serum PSA is not expected in a short treatment time frame.

Measurement of serum Testosterone, Dihydrotestosterone and Estradiol, at Screening Visit 1 and Visit 3 permitted any excursions beyond the upper limit of the reference range for the two physiological products of Testosterone; DHT, and Estradiol to be observed.

The safety analysis is performed on all subjects who received TBS-1. Occurrence of adverse events are presented by treatment group, by severity, and by relationship to the study drugs. All adverse events are described and evaluated regarding causality and severity. Adverse events are classified using MedDRA. However they are very few and all but two are not related to the drug.

Subject Safety

- Monitoring of subjects and emergency procedures: Emergency medication, equipment and Subject gurney are available at the Study Center. During the "at home" phase, the subjects have an emergency call number to be able to contact the Clinical Investigator.

- Adverse events are defined as any untoward medical occurrence in a subject or clinical trial subject having administered a medicinal product and which may or may not have a causal relationship with this treatment. An adverse event can therefore be any unfavorable and unintended sign, laboratory finding, symptom or disease temporally associated with the use of an investigational medicinal product, whether considered related to it or not. Any preexisting condition during the clinical trial which is worsened during the clinical study is to be considered an adverse event.

- An adverse reaction is defined as any untoward and unintended response to an investigational product related to any dose administered. All adverse reactions judged by either the Clinical Investigator or the Sponsor to have reasonable causal relationship to a medicinal product qualified as adverse reactions. This is meant to convey in general that there is evidence or an argument to suggest a causal relationship.

- An unexpected adverse reaction is defined as an adverse reaction, the nature, or severity of which is not consistent with the applicable product information.

- A serious adverse event or serious adverse reaction is defined as any untoward medical occurrence or effect that, at any dose, results in death, is life threatening, requires hospitalization or prolongation of existing in-Subject hospitalization, results in persistent or significant disability or incapacity, or is a congenital anomaly or birth defect.

- The observation period is extended from the time the subject began the study medication through the end of Visit 3 for hypogonadal subjects. AEs that are continuing at the end of the study period are followed until the Investigator believed that the AEs reached a stable clinical endpoint or are resolved.
- The percent of subjects with a serum DHT and Estradiol greater than the upper limit of the reference range, for the respective analytes.
- The Day 8 close-out findings are compared to the screening results, and clinically significant changes identified in the following:
    - Vital Signs and Adverse Events: Blood Pressure, Body Temperature, Respiratory Rate, Heart Rate.
    - Otorhinolaryngological examination.
    - Complete Blood Count to evaluate changes in white blood count, hemoglobin and hematocrit.
    - Clinical chemistry profile; Na/K, glucose, urea, creatinine, calcium, phosphate, uric acid, total bilirubin, albumin, AST, ALT, ALP, GGT, CK, and PSA.
- Classifications:
    - A serious adverse event (SAE) or serious adverse reaction: Defined as any untoward medical occurrence or effect that at any dose; results in death, is life-threatening, requires in-Subject hospitalization or prolongation of existing in-Subject hospitalization, results in persistent or significant disability or incapacity, is a congenital anomaly or birth defect, a medically important condition, i.e., the AE jeopardized the subject, or requires intervention to prevent one of the outcomes listed above.
    - Non-serious AE: Any AE not meeting the SAE criteria.
    - Intensity: An adverse event/reaction is classified as Mild, Moderate, or Severe.
    - Causality: The adverse event may be considered an adverse reaction to an investigational medicinal product when a "reasonable causal relationship" exists between the event and the investigational product. The following degree of causal relationship might be considered:
        - Definite: plausible temporal relationship with drug administration and withdrawal, and re-appears after drug re-start.
        - Probable: plausible temporal relationship with drug administration.
        - Possible: plausible temporal relationship with drug administration but can reasonably be associated to other factors.
        - Unlikely: does not have plausible temporal relationship with drug administration.
        - Unknown: no sufficient elements to establish a correlation with drug intake.
        - Not Related: cannot be correlated to the drug administration.
- Procedure to be followed in the case of adverse events: All adverse events detected by the Clinical Investigator are recorded in the special section of the Case Report Form. Any event that is classified as serious, regardless of causal relationship, is to have been reported to the CRO and Sponsor within 24 hours. There are no serious adverse events.

**9.5.2 Appropriateness of Measurements**

All measurements used in this study are standard indicies of efficacy, PK and safety and are generally recognised as reliable, accurate and relevant.

**9.5.3. Primary Efficacy Variable(s)**

Pharmacokinetic profiles of serum Testosterone for subjects dosed in Treatments A, B, and C that have:

1. A 24 hour $C_{avg}$ value > 300 ng/dL and < 1050 ng/dL.
2. The percent of subjects in each treatment group with a 24 hour $C_{avg}$ less than, within and above the serum Testosterone reference range of 300 ng/dL - 1050 ng/dL.

## 9.6 Data Quality Assurance

The CRF entries are verified by the monitors against source documents. All entries into the database included the CRF and Diary Card subject data, the PK results, and laboratory values. All data is 100% audited after being entered into the database for this report.

## 9.7 Statistical Methods Planned in the Protocol and Determination of Sample Size

### 9.7.1 Statistical and Analytical Plans

The PK Analysis Plan is described above. The Analysis Plan for the Vital Signs and Laboratory Results are compared baseline results with final visit results after PK analysis. Other data including demographic data is descriptive. No statistical analysis is performed because group sizes are not selected on the basis of statistical significance.

### 9.7.2 Determination of Sample Size

Based on the results are obtained from conducting several pharmacokinetic studies in groups of 10 subjects per cohort, these are sufficient for an acceptable description of the pharmacokinetic parameters in this population. As this is a relatively modest Phase II PK study with the intent of investigating two higher concentrations of TBS-1 gel, a true sample size calculation is not performed.

## 9.8 Changes in the Conduct of the Study or Planned Analysis

The protocol is amended on July 27, 2010. The change requested is in the timing of blood draws. The number of blood draws remained the same. This change is required to enable the full capture of the peak of testosterone absorption following the third TID dosing which occurred at 1300 hours on Day 8 or 1600 hours after the initial 2100 hour drug administration on the previous day (Day 7).

# 10. STUDY SUBJECTS

## 10.1 Disposition of Subjects

The study is conducted at three centers located in Miami, FL, Shreveport, LA and Tucson, AZ.

The three treatment groups are equally divided amongst the three sites. Eight Subjects received Treatment A, seven Subjects received Treatments B and C, respectively. A total of 22 subjects are in the study. In addition, five subjects who participated in the previous clinical study failed screening and are therefore not randomized to the study.

**Table 10.1. Disposition of Subjects by Site and Treatment**

| SITE ID | Treatment A: TBS-1 syringe prefilled with 125 micro-liters of drug | Treatment B: TBS-1 syringe prefilled with 150 micro-liters of drug | Treatment C: TBS-1 syringe prefilled with 125 micro-liters of drug | Total |
|---|---|---|---|---|
| 01 | 3 | 3 | 3 | 9 |
| 02 | 3 | 2 | 2 | 7 |
| 03 | 2 | 2 | 2 | 6 |
| Total | 8 | 7 | 7 | 22 |

## 10.2 Protocol Deviations

There are no meaningful pharmacokinetic deviations.

## 11. PHARMACOKINETICS AND STATISTICS

### 11.1 Datasets Analyzed

The PK population is defined as subjects who receive the Treatment A, B or C, and who complete the study without major protocol violation or for whom the PK profile can be adequately characterized. The PK population is used for the analysis of PK data.

Based on the above criteria, twenty-two (22) subjects are included in the PK population. The numbers of subjects by site and by treatment are displayed below.

**Table 11.1.1: Disposition of Subjects in the PK population:**

| Site | Number of Subjects |
|---|---|
| 1 | 9 |
| 2 | 7 |
| 3 | 6 |
| **Treatment** | **Number of Subjects** |
| A: TBS-1 125 μL of 4.0 % Gel (t.i.d.) | 8 |
| B: TBS-1 150 μl of 4.5 % Gel (b.i.d.) | 7 |
| C: TBS-1 125 μl of 4.5 % Gel (t.i.d.) | 7 |

### 11.2 Demographic and Other Baseline Characteristics

The demographic data and characteristics are presented by dose group for all the treated subjects in Table 11.2. No meaningful differences are observed amongst the three groups for any of the characteristics.

**Table 11.2: Summary of Demographic Characteristics-All Subjects**

| | Treatment A: TBS-1 syringe prefilled with 125 micro-liters of 4.0 percent gel | Treatment B: TBS-1 syringe prefilled with 150 micro-liters of 4.5 percent gel | Treatment C: TBS-1 syringe prefilled with 125 micro-liters of 4.5 percent gel | All Subjects |
|---|---|---|---|---|
| Characteristic | (N = 8) | (N = 7) | (N = 7) | (N = 22) |
| SEX | | | | |
| Male | 8 | 7 | 7 | 22 |
| RACE | | | | |
| Black or African American | | | 1 | 1 |
| White | 8 | 7 | 6 | 21 |
| ETHNIC | | | | |
| Hispanic or Latino | 4 | 3 | 3 | 10 |
| Non-Hispanic and Non-Latino | 4 | 4 | 4 | 12 |
| AGE | | | | |
| Mean | 52.38 | 53.86 | 51.57 | 52.59 |
| SD | 12.55 | 11.04 | 9.90 | 10.78 |
| Minimum | 37 | 36 | 35 | 35 |

(continued)

| | Treatment A: TBS-1 syringe prefilled with 125 micro-liters of 4.0 percent gel | Treatment B: TBS-1 syringe prefilled with 150 micro-liters of 4.5 percent gel | Treatment C: TBS-1 syringe prefilled with 125 micro-liters of 4.5 percent gel | All Subjects |
|---|---|---|---|---|
| Maximum | 73 | 63 | 67 | 73 |
| Median | 51 | 59 | 52 | 54 |

The treated populations for Group A have a mean age of 52.38, for Group B 53.86, and for Group C 51.57. The standard deviations are 12.55, 11.04, and 9.90, respectively. The ethnic and racial distribution are essentially the same in each group.

### 11.3 Measurement of Treatments Compliance

Compliance of drug utilization during the home portion of the study is determined by a review of the diaries and used returned pouches and syringes. Although the method is not absolute, it is sufficient to establish reasonable compliance. One subject could not find his diary.

### 11.4 Pharmacokinetics and Statistical Results

#### 11.4.1 Methods

The blood concentrations are received from ABL and transferred electronically from Trimel Biopharma SRL to the statistical unit of PharmaNet. Testosterone and Dihydrotestosterone serum concentrations are provided in ng/mL. However, the serum concentrations are converted to ng/dL for PK calculation to match the units of the literature's reference ranges.

During the trial, clinical site 1 performs PK sampling one day later than specified in the protocol that is it started on Day 8 rather than Day 7. This change is not planned. Consequently, the actual times are calculated relative to the 2100 drug administration on Day 8 for the subjects of clinical site 1 and the drug administration 21h00 on Day 7 for the subjects of clinical sites 2 and 3.

For subject No. 02-003, the dosing time is not recorded on Day 7. Consequently, the schedule sampling times are used instead of the actual sampling times for PK calculations. The 16.33 h and 16.67 h samples for subject 01-001 are drawn at the same time due to technical reason. The schedule sampling time is used for sample 16.33 h while the actual sampling time is used for sample 16.67 h.

Excluding the above exceptions, time deviations during sampling are treated as follows: for all sampling times, the difference between the scheduled and the actual sampling time is considered acceptable if it is less than 1 minute. When the difference exceeded this time limit, the actual sampling times (rounded off to three decimal digits) are used to calculate pharmacokinetic parameters, except for pre-dose samples, which are always reported as zero (0.000), regardless of time deviations. Scheduled sampling times are presented in concentration tables and graphs in the statistical report.

PK calculations are performed using WinNonlin™ version 5.2 (or higher), validated according to industry's expectations and regulatory requirements. Descriptive statistical calculations are also performed using Microsoft® Office Excel 2003. Microsoft® Office Excel 2003 and Microsoft® Office Word 2003 are used for report data tabulation.

Descriptive statistics (N, mean, standard deviation (SD), coefficient of variation (CV), median, minimum value (Min.), and maximum value (Min.)) of the serum concentrations versus time as well as all pharmacokinetic parameters are provided for each treatment at each dose level using the evaluable population. All figures are presented using both linear (a) and semi-log (b) scales.

For the calculation of the PK parameters from the last three drug administrations (Treatments A and C: 0 hour to 10 hours, 10 hours and 16 hours and 16 hours and 24 hours; treatment B: 0 hour to 10 hours and 10 hours and 24 hours), the serum concentration values for Testosterone, Dihydrotestosterone, and Estradiol at time points 10 hours (pre-dose for the second drug administration) and 16 hours (pre-dose for the third drug administration under Treatments A and C) are obtained by imputing the serum concentration value observed at time points 9.75 hours and 15.75 hours, respectively.

The following pharmacokinetic parameters are determined for all subjects for Testosterone, Dihydrotestosterone and Estradiol:

For Treatments A and C (t.i.d.): $AUC_{0-\tau}$, $AUC_{0-10}$, $AUC_{10-16}$, $AUC_{16-24}$, $C_{max}$, $C_{max\ 0-10}$, $C_{max\ 10-16}$, $C_{max\ 16-24}$, $C_{min}$, $C_{min\ 0-10}$, $C_{min\ 10-16}$, $C_{min\ 16-24}$, $C_{avg}$, $C_{avg\ 0-10}$, $C_{avg\ 10-16}$, $C_{avg\ 16-24}$, $t_{max}$, $t_{max\ 0-10}$, $t_{max\ 10-16}$, $t_{max\ 16-24}$, $t_{max\ 10-24}$, PTF, PTS.

For Treatment B (b.i.d.): $AUC_{0-\tau}$, $AUC_{0-10}$, $AUC_{10-24}$, $C_{max}$, $C_{max\ 0-10}$, $C_{max\ 10-24}$, $C_{min}$, $C_{min\ 0-10}$, $C_{min\ 10-24}$, $C_{avg}$, $C_{avg\ 0-10}$, $C_{avg\ 10-24}$, $t_{max}$, $t_{max\ 0-10}$, $t_{max\ 10-24}$, PTF, PTS.

Additionally, the percent of subjects with $C_{avg}$ values for serum Testosterone, Dihydrotestosterone and Estradiol above, within, and below their respective reference range is calculated for each treatment. As well, the mean percent time of serum Testosterone, Dihydrotestosterone and Estradiol values above (% TimeAbove), within (% TimeWithin), and below (% TimeBelow) the corresponding reference range are provided for each treatment. The calculation of all these pharmacokinetic parameters is explained below.

### 11.4.1.1 Maximum and Minimum Observed Concentrations and Time of Observed Peak Concentrations

$C_{max}$, the maximum is observed concentrations and $T_{max}$, the time to reach that peak concentrations, as well as $C_{min}$, the minimum observed concentrations are determined for each subject and for each treatment as follow:

| | |
|---|---|
| $C_{max}$: | Maximum observed concentration over the dosing interval. This parameter is calculated for Treatments A, B and C. |
| $C_{max\ 0-10}$: | Maximum observed concentration from time zero to 10 hours. This parameter is calculated for Treatments A, B and C. |
| $C_{max\ 10-16}$: | Maximum observed concentration from time 10 hours to 16 hours. This parameter is calculated for Treatments A and C. |
| $C_{max\ 16-24}$: | Maximum observed concentration from time 16 hours to 24 hours. This parameter is calculated for Treatments A and C. |
| $C_{max\ 10-24}$: | Maximum observed concentration from time 10 hours to 24 hours. This parameter is calculated for Treatment B only. |
| $C_{min}$: | Minimum observed concentration over the dosing interval. This parameter is calculated for Treatments A, B and C. |
| $C_{min\ 0-10}$: | Minimum observed concentration from time zero to 10 hours. This parameter is calculated for Treatments A, B and C. |
| $C_{min\ 10-16}$: | Minimum observed concentration from time 10 hours to 16 hours. This parameter is calculated for Treatments A and C. |
| $C_{min\ 16-24}$: | Minimum observed concentration from time 16 hours to 24 hours. This parameter is calculated for Treatments A and C. |
| $C_{min\ 10-24}$: | Minimum observed concentration from time 10 hours to 24 hours. This parameter is calculated for Treatment B only. |
| $t_{max}$: | Time of observed $C_{max}$ over the dosing interval. This parameter is calculated for Treatments A, B and C. |
| $t_{max\ 0-10}$: | Time of observed $C_{max}$ from time zero to 10 hours. This parameter is calculated for Treatments A, B and C. |
| $t_{max\ 10-16}$: | Time of observed $C_{max}$ from time 10 hours to 16 hours. This parameter is calculated for Treatments A and C. |
| $t_{max\ 16-24}$: | Time of observed $C_{max}$ from time 16 hours to 24 hours. This parameter is calculated for Treatments A and C. |
| $t_{max\ 10-24}$: | Time of observed $C_{max}$ from time 10 hours to 24 hours. This parameter is calculated for Treatment B only. |

### 11.4.1.2 Areas Under the Concentration-Time Curves

The calculation of AUCs is performed using the linear trapezoidal method. $AUC_{0-\tau}$ is computed from dose time (0) to dose time □ (□ = 24 h). However, in case the 24-h sample is collected with a time deviation, the $AUC_{0-\tau}$ is estimated based on the estimated concentration at 24 hours using the regression line calculated from the elimination phase, and not the concentration at the actual observation time.

In the case where the last concentration value (Y) is missing or does not correspond to a scheduled sampling time (i.e. 10 hours and 16 hours), $AUC_{X-Y}$ is extrapolated using the corresponding subject's elimination phase, if calculable.

The following AUCs are calculated:

| | |
|---|---|
| $AUC_{0-\tau}$ : | Area under the concentration-time curve for one dosing interval. This parameter is calculated for Treatments A, B and C. |
| $AUC_{0-10}$: | Area under the concentration-time curve from time zero to 10 hours. This parameter is calculated for Treatments A, B and C. |
| $AUC_{10-16}$: | Area under the concentration-time curve from time 10 hours to 16 hours. This parameter is calculated for |

Treatments A and C.

$AUC_{16-24}$: Area under the concentration-time curve from time 16 hours to 24 hours. This parameter is calculated for Treatments A and C.

$AUC_{10-24}$: Area under the concentration-time curve from time 10 hours to 24 hours. This parameter is calculated for Treatment B only.

The $C_{avg}$ are calculated as follow:

$C_{avg}$: Average concentration during the dosing interval, calculated as AUCO-τ/τ (τ=24 hours). This parameter is calculated for Treatments A, B and C.

$C_{avg\ 0-10}$: Average concentration from time zero to 10 hours, calculated as AUC0-10/10. This parameter is calculated for Treatments A, B and C.

$C_{avg\ 10-16}$: Average concentration from time 10 hours to 16 hours, calculated as AUC10-16/6. This parameter is calculated for Treatments A and C.

$C_{avg\ 16-24}$: Average concentration from time 16 hours to 24 hours, calculated as AUC16-24/8. This parameter is calculated for Treatments A and C.

$C_{avg\ 10-24}$: Average concentration from time 10 hours to 24 hours, calculated as AUC10-24/14. This parameter is calculated for Treatment B only.

### 11.4.1.3 Average Drug Concentrations

The $C_{avg}$ are calculated as follow:

$C_{avg}$: Average concentration during the dosing interval, calculated as AUCO-τ/τ (τ=24 hours). This parameter is calculated for Treatments A, B and C.

$C_{avg\ 0-10}$: Average concentration from time zero to 10 hours, calculated as AUC0-10/10. This parameter is calculated for Treatments A, B and C.

$C_{avg\ 10-16}$: Average concentration from time 10 hours to 16 hours, calculated as AUC10-16/6. This parameter is calculated for Treatments A and C.

$C_{avg\ 16-24}$: Average concentration from time 16 hours to 24 hours, calculated as AUC16-24/8. This parameter is calculated for Treatments A and C.

$C_{avg\ 10-24}$: Average concentration from time 10 hours to 24 hours, calculated as AUC10-24/14. This parameter is calculated for Treatment B only.

### 11.4.1.4 Peak Trough Fluctuation and Peak Trough Swing

The peak trough fluctuation (PTF) and the Peak trough swing are calculated as follow:

PTF: Peak trough fluctuation, calculated as $(C_{max}-C_{min})/C_{avg.}$ This parameter is calculated for Treatments A, B and C.

PTS: Peak trough swing, calculated as $(C_{max}-C_{min})/C_{min}$. This parameter is calculated for Treatments A, B and C.

11.4.1.5 Percent Time Above, Within and Below the Reference Range and Percent of aSubjects With $C_{avg}$ Above, Within and Below the Reference Range

The percent times during which observations fall above (% TimeAbove), within (% TimeWithin), and below (% TimeBelow) the reference ranges are computed for each subject and treatment for the serum Testosterone, Dihydrotestosterone and Estradiol. The percent of subjects with $C_{avg}$ values for serum Testosterone, Dihydrotestosterone and Estradiol above, within, and below their respective reference range is calculated for each treatment. The reference ranges are 300ng/dL to 1050 ng/dL for Testosterone, 25.5 ng/dL to 97.8 ng/dL for Dihydrotestosterone and 3 pg/mL to 81 pg/mL for Estradiol.

PTS: Peak trough swing, calculated as $(C_{max}-C_{min})/C_{min}$. This parameter is calculated for Treatments A, B and C.

### 11.4.1.6 Statistical Analysis

Only descriptive statistics (N, mean, SD, CV, median, Min., and Max.) are calculated on the serum concentrations and the PK parameters for each treatment. No inferential statistical analysis is performed.

## 11.4.2 Analysis of Pharmacokinetics and Statistical Issues

### 11.4.2.2 Handling of Missing Data

Samples that are not analyzed due to an insufficient volume (refer to the bioanalytical report) are recorded as INV (Insufficient volume for analysis) in the concentration tables.

These samples are set as missing for pharmacokinetic and statistical analyses. As the PK parameters could be estimated using the remaining data points, subjects with missing data are kept in the pharmacokinetic analysis.

### 11.4.2.3 Pharmacokinetic Analysis

The following pharmacokinetic parameters are determined for all subjects for Testosterone, Dihydrotestosterone and Estradiol:

For Treatments A and C (t.i.d.): $AUC_{0-\tau}$, $AUC_{0-10}$, $AUC_{10-16}$, $AUC_{16-24}$, $C_{max}$, $C_{max\ 0-10}$, $C_{max\ 10-16}$, $C_{max\ 16-24}$, $C_{min}$, $C_{min\ 0-10}$, $C_{min\ 10-16}$, $C_{min\ 16-24}$, $C_{avg}$, $C_{avg\ 0-10}$, $C_{avg\ 10-16}$, $C_{avg\ 16-24}$, $t_{max}$, $t_{max\ 0-10}$, $t_{max\ 10-16}$, $t_{max\ 16-24}$, $t_{max\ 10-24}$, PTF, PTS.

For Treatment B (b.i.d.): $AUC_{0-\tau}$, $AUC_{0-10}$, $AUC_{10-24}$, $C_{max}$, $C_{max\ 0-10}$, $C_{max\ 10-24}$, $C_{min}$, $C_{min\ 0-10}$, $C_{min\ 10-24}$, $C_{avg}$, $C_{avg\ 0-10}$, $C_{avg\ 10-24}$, $t_{max}$, $t_{max\ 0-10}$, $t_{max\ 10-24}$, PTF, PTS. Additionally, the percent of subjects with $C_{avg}$ values for serum Testosterone, Dihydrotestosterone and Estradiol above, within, and below their respective reference range is calculated for each treatment. As well, the mean percent time of serum Testosterone, Dihydrotestosterone and Estradiol values above (% TimeAbove), within (% TimeWithin), and below (% TimeBelow) the corresponding reference range are provided for each treatment. The calculation of all these pharmacokinetic parameters is explained below.

With the exception of text Tables (numbered as 11.4.2.3-1 to 11.4.2.3-3) and text Figures (numbered as 11.4.2.3-1 to 11.4.2.3-3), all tables and figures referred to in this section are displayed in sections 14.2.1 and 14.2.2, respectively. For brevity, TBS-1 treatments are identified in the text of the statistical report by their treatment code: A (125 μL of 4% gel given t.i.d. for a total dose of 30 mg/day), B (150 μL of 4.5% gel is given b.i.d. for a total dose of 27.0 mg/day) and C (125 μL of 4.5% gel given t.i.d. for a total dose of 33.75 mg/day).

Blood samples for pharmacokinetic analysis are collected prior and post the 2100 hour drug administration on Day 7 at 0.333, 0.667, 1.00, 1.50, 2.00, 3.00, 6.00, 9.00, 9.75, 10.33, 10.66, 11.0, 11.5, 12.0, 13.0, 14.0, 15.75, 16.33, 16.66, 17.0, 17.5, 18.0, 20.0, 22.0, and 24.0 hours for Treatments A and C. Blood samples for pharmacokinetic analysis are collected prior and post the 2100 hour drug administration on Day 7 at 0.333, 0.667, 1.00, 1.50, 2.00, 3.00, 6.00, 9.00, 9.75, 10.33, 10.66, 11.0, 11.5, 12.0, 13.0, 16.0, 19.0, 22.0, and 24.0 hours for Treatment B. The actual sampling times is used for PK calculation are displayed in Tables 14.2.1.22, 14.2.1.23 and 14.2.1.24 for Treatments A, B and C, respectively.

**TESTOSTERONE**

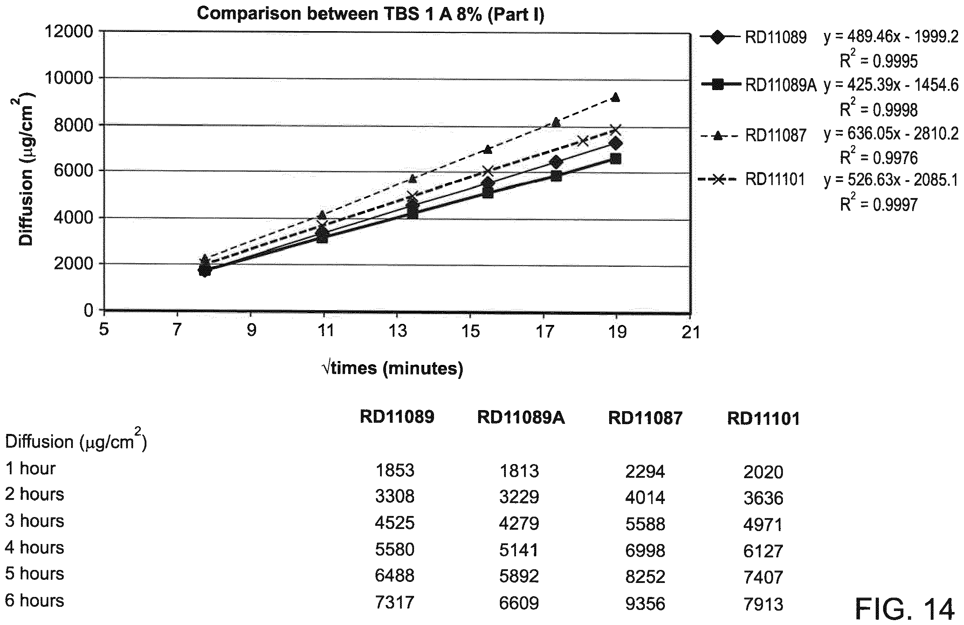

The Testosterone serum concentrations measured for each subject at each sampling time appear in Tables 14.2.1.1, 14.2.1.2 and 14.2.1.3 according to treatment. The plots of the individual serum levels over the sampling period are presented using both linear (a) and semi-log (b) scales in Figures 14.2.2.1 through 14.2.2.22. Lines for the minimum (300 ng/dL) and maximum (1050 ng/dL) bound of the reference range for the testosterone serum concentrations are also presented for information purposes. As well, a line for the average drug concentration ($C_{avg}$) during the dosing interval ($\tau$=24 hours) is also presented on the individual profiles.

The plots of the mean serum levels over the sampling period are also presented using both the linear (a) and semi-log (b) scales in Figures 14.2.2.23, 14.2.2.24 and 14.2.2.25 for Treatments A, B and C, respectively. The error bars on these mean profiles correspond to one standard deviation. The lines for the minimum and maximum bound of the reference ranges are also presented on the mean figures.

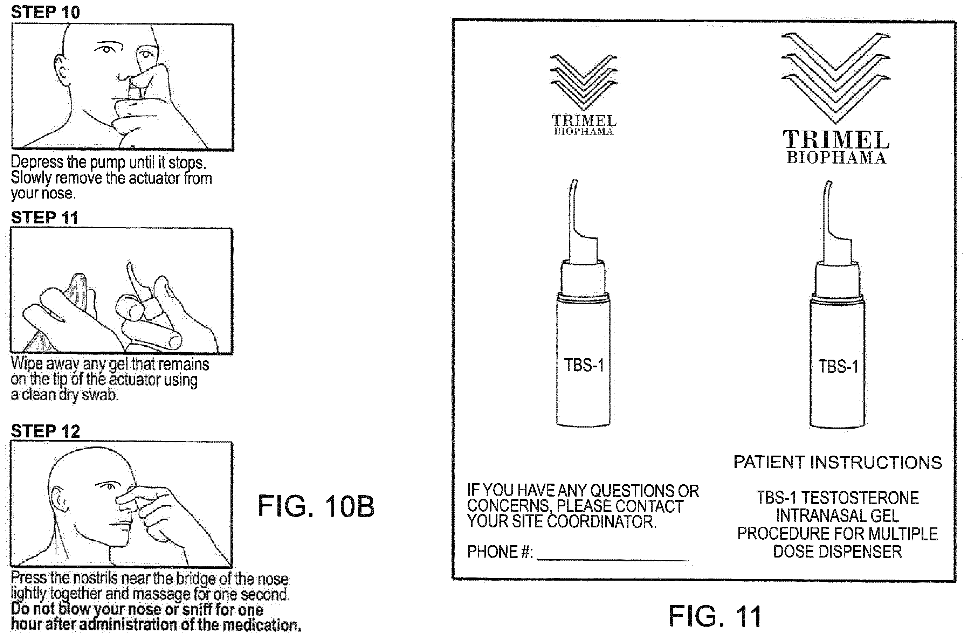

The mean plot on the linear scale for each treatment is also presented below in the text Figure 11.4.2.3-1.

## Figure 11.4.2.3-1: Mean Testosterone Serum Concentration (ng/dL) - Time Profile for Each Treatment

900
800
700
600
500
400
300
200
Serum Concentration (ng/dL)
0.0
2.0
4.0
6.0
8.0
10.0
12.0
14.0
16.0
18.0
20.0
22.0
24.0
Time (h)
TBS-1 (125 μL of 4.0 % Gel) (A)
TBS-1 (150 μL of 4.5 % Gel) (B)
TBS-1 (125 μL of 4.5 % Gel) (C)


Calculated pharmacokinetic parameters for each subject according to treatment are shown in Tables 14.2.1.4, 14.2.1.5 and 14.2.1.6 for Treatments A, B and C, respectively. They are summarized in the text Table 11.4.2.3-1.

**Table 11.4.2.3-1: Summary of Testosterone Pharmacokinetic Parameters for Each Treatment**

| Parameter | Unit | Treatment A[1] (N = 8) | | | Treatment B[2] (N = 7) | | | Treatment C[3] (N = 7) | | |
|---|---|---|---|---|---|---|---|---|---|---|
| | | Mean | SD | CV% | Mean | SD | CV% | Mean | SD | CV% |
| $AUC_{0-10}$ | h*ng/dL | 4178.68 | 1210.51 | 28.97 | 4451.64 | 1581.09 | 35.52 | 4355.19 | 1374.07 | 31.55 |
| $C_{max\ 0-10}$ | ng/dL | 786 | 209 | 26.53 | 894 | 500 | 55.90 | 857 | 323 | 37.72 |
| $C_{min\ 0-10}$ | ng/dL | 259 | 70.3 | 27.16 | 256 | 91.5 | 35.76 | 272 | 69.7 | 25.61 |
| $C_{avg\ 0-10}$ | ng/dL | 418 | 121 | 28.97 | 445 | 158 | 35.52 | 436 | 137 | 31.55 |
| $T_{max\ 0-10}$ | h | 1.01 | 0.678 | 67.21 | 0.695 | 0.279 | 40.18 | 0.905 | 0.422 | 46.62 |
| $AUC_{10-16}$ | h*ng/dL | 2635.05 | 1062.56 | 40.32 | - | - | - | 2301.51 | 658.44 | 28.61 |
| $C_{max\ 10-16}$ | ng/dL | 698 | 251 | 35.88 | - | - | - | 675 | 256 | 37.98 |
| $C_{min\ 10-16}$ | ng/dL | 270 | 90.7 | 33.63 | - | - | - | 230 | 53.9 | 23.48 |
| $C_{avg\ 10-16}$ | ng/dL | 439 | 177 | 40.32 | - | - | - | 384 | 110 | 28.61 |
| $T_{max\ 10-16}$ | h | 11.1 | 1.06 | 9.54 | - | - | - | 10.8 | 0.562 | 5.20 |
| $AUC_{10-24}$ | h*ng/dL | - | - | - | 5264.19 | 2176.63 | 41.35 | - | - | - |
| $C_{max\ 10-24}$ | ng/dL | - | - | - | 846 | 377 | 44.53 | - | - | - |
| $C_{min\ 10-24}$ | ng/dL | - | - | - | 228 | 100 | 43.88 | - | - | - |
| $C_{avg\ 10-24}$ | ng/dL | - | - | - | 376 | 155 | 41.35 | - | - | - |
| $T_{max\ 10-24}$ | h | - | - | - | 11.1 | 0.675 | 6.06 | - | - | - |
| $AUC_{16-24}$ | h*ng/dL | 3016.52 | 1083.58 | 35.92 | | | | 2766.97 | 838.13 | 30.29 |
| $C_{max\ 16-24}$ | ng/dL | 556 | 216 | 38.78 | - | - | - | 595 | 352 | 59.20 |
| $C_{min\ 16-24}$ | ng/dL | 271 | 86.9 | 32.08 | - | - | - | 225 | 59.1 | 26.26 |
| $C_{avg\ 16-24}$ | ng/dL | 377 | 135 | 35.92 | - | - | - | 346 | 105 | 30.29 |
| $T_{max\ 16-24}$ | h | 16.6 | 0.404 | 2.43 | - | - | - | 16.8 | 0.704 | 4.19 |
| $AUC_{0-□}$ | h*ng/dL | 9920.07 | 3300.65 | 33.27 | 9781.39 | 3532.43 | 36.11 | 9505.03 | 2650.59 | 27.89 |
| $C_{max}$ | ng/dL | 830 | 188 | 22.65 | 1050 | 463 | 44.19 | 883 | 346 | 39.23 |
| $C_{min}$ | ng/dL | 239 | 77.6 | 32.55 | 224 | 98.6 | 43.97 | 222 | 57.1 | 25.69 |
| $C_{avg}$ | ng/dL | 413 | 138 | 33.27 | 408 | 147 | 36.11 | 396 | 110 | 27.89 |

(continued)

| Parameter | Unit | Treatment A[1] (N = 8) | | | Treatment B[2] (N = 7) | | | Treatment C[3] (N = 7) | | |
|---|---|---|---|---|---|---|---|---|---|---|
| | | Mean | SD | CV% | Mean | SD | CV% | Mean | SD | CV% |
| $T_{max}$ | h | 4.61 | 5.27 | 114.31 | 4.99 | 5.43 | 108.81 | 4.50 | 6.44 | 143.18 |
| PTF | - | 1.51 | 0.39 | 26.03 | 2.04 | 1.07 | 52.23 | 1.61 | 0.47 | 28.92 |
| PTS | - | 2.63 | 0.73 | 27.70 | 4.49 | 3.92 | 87.27 | 3.04 | 1.65 | 54.27 |
| % TimeBelow* | % | 34.47 | 30.93 | 89.72 | 36.40 | 25.92 | 71.22 | 30.14 | 29.25 | 97.05 |
| % TimeWithin* | % | 65.16 | 30.46 | 46.75 | 59.47 | 23.10 | 38.84 | 68.21 | 28.77 | 42.17 |
| % TimeAbove* | % | 0.38 | 1.06 | 282.84 | 4.13 | 6.88 | 166.67 | 1.65 | 2.60 | 157.31 |
| $C_{avg}$ Below* [N (% of Subjects)] | % | 1 (12.50%) | - | - | 1 (14.29%) | - | - | 1 (14.29%) | - | - |
| $C_{avg}$ Within* [N (% of Subjects)] | % | 7 (87.50%) | - | - | 6 (85.71 %) | - | - | 6 (85.71%) | - | - |
| $C_{avg}$ Above* [N (% of Subjects)] | % | 0(0%) | - | - | 0(0%) | - | - | 0 (0%) | - | - |

* Reference Range = 300-1050 ng/dL.
1 = TBS-1, 125 μL 4.0% gel given t.i.d. (total dose 30 mg/day)
2 = TBS-1, 150 μL of 4.5% gel given b.i.d. (total dose 27.0 mg/day)
3 = TBS-1, 125 μL of 4.5% gel given t.i.d. (total dose 33.75 mg/day)

The percent times during which observations fall above (% TimeAbove), within (% TimeWithin), and below (% TimeBelow) the reference range are computed for each subject and are presented in Tables 14.2.1.4, 14.2.1.5 and 14.2.1.6 for Treatments A, B and C, respectively. These results are also summarized in text Table 11.4.2.3.1.

The percent of subjects with $C_{avg}$ values for serum Testosterone above, within, and below the reference range is calculated for each treatment and are presented in Table 14.2.1.7. These results are also summarized in text Table 11.4.2.3.1.

**DIHYDROTESTOSTERONE**

The Dihydrotestosterone serum concentrations are measured for each subject at each sampling time appear in Tables 14.2.1.8, 14.2.1.9 and 14.2.1.10 according to treatment. The plots of the individual serum levels over the sampling period are presented using both linear (a) and semi-log (b) scales in Figures 14.2.2.26 through 14.2.2.47. Lines for the minimum (25.5 ng/dL) and maximum (97.8 ng/dL) bound of the reference range for the Dihydrotestosterone serum concentrations are also presented for information purposes. As well, a line for the average drug concentration ($C_{avg}$) during the dosing interval (τ=24 hours) is also presented on the individual profiles.

The plots of the mean serum levels over the sampling period are also presented using both the linear (a) and semi-log (b) scales in Figures 14.2.2.48, 14.2.2.49 and 14.2.2.50 for Treatments A, B and C, respectively. The error bars on these mean profiles correspond to one standard deviation. The lines for the minimum and maximum bound of the reference ranges are also presented on the mean figures.

The mean plot on the linear scale for each treatment is also presented below in the text Figure 11.4.2.3-2.

## Figure 11.4.2.3-2: Mean Dihydrotestosterone Serum Concentration (ng/dL) - Time Profile for Each Treatment

TBS-1 (125 µL of 4.0 % Gel) (A)
TBS-1 (150 µL of 4.5 % Gel) (B)
TBS-1 (125 µL of 4.5 % Gel) (C)
Serum Concentration (ng/dL)
60
55
50
45
40
35
30
25
0.0
2.0
4.0
6.0
8.0
10.0
12.0
14.0
16.0
18.0
20.0
22.0
24.0
Time (h)


As per SAP, $AUC_{X-Y}$ is calculated based on the estimated concentration (Y) using the regression line calculated from the elimination phase data when the last concentration (Y) does not correspond to a schedule sampling time. For subject No. 01-002 and 02-007, the elimination phase is not well characterized due to fluctuation in the Dihydrotestosterone serum concentration for the 10 to 16 hours and 0 to 10 hours intervals, respectively. Therefore, $AUC_{10-16}$ and $C_{avg\ 10-16}$ (derived from $AUC_{10-16}$) could not be calculated for subject No. 01-002 for Treatment A (N = 7 for these parameters). As well, $AUC_{0-10}$ and $C_{avg\ 0-10}$ (derived from $AUC_{0-10}$) could not be calculated for subject No. 02-007 for Treatment A (N = 7 for these parameters).

Calculated pharmacokinetic parameters for each subject according to treatment are shown in Tables 14.2.1.11, 14.2.1.12 and 14.2.1.13 for Treatments A, B and C, respectively. They are summarized in the text Table 11.4.2.3-2.

**Table 11.4.2.3-2: Summary of Dihydrotestosterone Pharmacokinetic Parameters for Each Treatment**

| Parameter | Unit | Treatment A[1] (N = 8) | | | Treatment B[2] (N = 7) | | | Treatment C[3] (N = 7) | | |
|---|---|---|---|---|---|---|---|---|---|---|
| | | Mean | SD | CV % | Mean | SD | CV % | Mean | SD | CV % |
| $AUC_{0-10}$[a] | h*ng/dL | 345.77 | 133 .49 | 38.61 | 402.77 | 133 .11 | 33.05 | 411.10 | 131 .22 | 31.92 |
| $C_{max\ 0-10}$ | ng/dL | 51.4 | 18. 8 | 36.52 | 56.8 | 17. 1 | 30.08 | 59.0 | 19. 7 | 33.48 |
| $C_{min\ 0-10}$ | ng/dL | 26.6 | 10. 1 | 38.15 | 30.1 | 13. 4 | 44.57 | 31.7 | 9.3 3 | 29.41 |
| $C_{avg\ 0-10}$ [a] | ng/dL | 34.6 | 13. 3 | 38.61 | 40.3 | 13. 3 | 33.05 | 41.1 | 13. 1 | 31.92 |
| $T_{max\ 0-10}$ | h | 2.38 | 2.9 8 | 125 .22 | 1.70 | 0.501 | 29.48 | 1.32 | 0.569 | 43.20 |
| $AUC_{10-16}$[a] | h*ng/dL | 186.33 | 65.10 | 34.94 | - | - | - | 222.62 | 53.52 | 24.04 |
| $C_{\ max\ 10-16}$ | ng/dL | 44.2 | 16. 8 | 38.01 | - | - | - | 48.9 | 12. 4 | 25.37 |
| $C_{min\ 10-16}$ | ng/dL | 26.6 | 10. 4 | 38.95 | - | - | - | 30.1 | 8.4 1 | 27.94 |
| $C_{avg\ 10-16}$ [a] | ng/dL | 31.1 | 10. 8 | 34.94 | - | - | - | 37.1 | 8.9 2 | 24.04 |
| $T_{max\ 10-16}$ | h | 11.9 | 1.1 3 | 9.5 0 | - | - | - | 11.4 | 0.436 | 3.8 4 |
| $AUC_{10-24}$ | h*ng/dL | - | - | - | 543.29 | 235 .71 | 43.39 | - | - | - |
| $C_{max\ 10-24}$ | ng/dL | - | - | - | 54.6 | 21. 9 | 40.12 | - | - | - |
| $C_{min\ 10-24}$ | ng/dL | - | - | - | 28.3 | 12. 7 | 45.02 | - | - | - |
| $C_{avg\ 10-24}$ | ng/dL | - | - | - | 38.8 | 16. 8 | 43.39 | - | - | - |
| $T_{max\ 10-24}$ | h | - | - | - | 11.8 | 0.775 | 6.5 5 | - | - | - |
| $AUC_{16-24}$ | h*ng/dL | 269.16 | 114 .13 | 42.40 | - | - | - | 275.21 | 74.02 | 26.89 |
| $C_{max\ 16-24}$ | ng/dL | 41.3 | 17. 0 | 41.20 | - | - | - | 42.6 | 12. 8 | 30.15 |
| $C_{min\ 16-24}$ | ng/dL | 26.5 | 11. 3 | 42.63 | - | - | - | 26.6 | 6.4 1 | 24.11 |
| $C_{avg\ 16-24}$ | ng/dL | 33.6 | 14. 3 | 42.40 | - | - | - | 34.4 | 9.2 5 | 26.89 |
| $T_{max\ 16-24}$ | h | 17.6 | 1.3 7 | 7.7 9 | - | - | - | 17.5 | 0.433 | 2.4 8 |
| $AUC_{0-\tau}$ | h*ng/dL | 818.95 | 315 .07 | 38.47 | 946.89 | 361 .03 | 38.13 | 909.68 | 249 .37 | 27.41 |

(continued)

| Parameter | Unit | Treatment A[1] (N = 8) | | | Treatment B[2] (N = 7) | | | Treatment C[3] (N = 7) | | |
|---|---|---|---|---|---|---|---|---|---|---|
| | | Mean | SD | CV % | Mean | SD | CV % | Mean | SD | CV % |
| $C_{max}$ | ng/dL | 52.2 | 18. 1 | 34.64 | 61.0 | 22. 5 | 36.85 | 60.3 | 18. 6 | 30.84 |
| $C_{min}$ | ng/dL | 25.3 | 10. 1 | 40.14 | 27.8 | 13. 0 | 46.69 | 26.6 | 6.4 1 | 24.11 |
| $C_{avg}$ | ng/dL | 34.1 | 13. 1 | 38.47 | 39.5 | 15. 0 | 38.13 | 37.9 | 10. 4 | 27.41 |
| $T_{max}$ | h | 4.43 | 6.0 1 | 135 .63 | 4.42 | 4.8 4 | 109 .53 | 4.26 | 5.1 8 | 121 .44 |
| PTF | - | 0.82 | 0.2 8 | 34.18 | 0.89 | 0.3 3 | 36.71 | 0.88 | 0.1 7 | 19.17 |
| PTS | - | 1.14 | 0.4 4 | 39.02 | 1.36 | 0.7 0 | 51.43 | 1.24 | 0.3 0 | 23.90 |
| * Reference Range = 25.5-97.8 ng/dL.<br>1 = TBS-1, 125 µL 4.0% gel given t.i.d. (total dose 30 mg/day)<br>2 = TBS-1, 150 µL of 4.5% gel given b.i.d. (total dose 27.0 mg/day)<br>3 = TBS-1, 125 µL of 4.5% gel given t.i.d. (total dose 33.75 mg/day)<br>a = For these parameters,<br>N = 7 for Treatment A.<br>% TimeBelow* | % | 32.64 | 35.13 | 107 .62 | 26.22 | 30.06 | 114 .63 | 13.87 | 36.41 | 262 .41 |
| % TimeWithin* | % | 67.36 | 35.13 | 52.15 | 73.78 | 30.06 | 40.74 | 86.13 | 36.41 | 42.27 |
| % TimeAbove* | % | 0.00 | 0.0 0 | - | 0.00 | 0.0 0 | - | 0.00 | 0.0 0 | - |
| $C_{avg}$ Below* [N (% of Subjects)] | % | 3 (37.50 %) | - | - | 1 (14.29 %) | - | - | 1 (14.29 %) | - | - |
| $C_{avg}$ Within* [N (% of Subjects)] | % | 5 (62.50 %) | - | - | 6 (85.71 %) | - | - | 6 (85.71 %) | - | - |
| $C_{avg}$ Above* [N (% of Subjects)] | % | 0 (0%) | - | - | 0 (0%) | - | - | 0 (0%) | - | - |

The percent times during which observations fall above (% TimeAbove), within (% TimeWithin), and below (% TimeBelow) the reference range are computed for each subject and are presented in Tables 14.2.1.11, 14.2.1.12 and 14.2.1.13 for Treatments A, B and C, respectively. These results are also summarized in text Table 11.4.2.3.2. The percent of subjects with $C_{avg}$ values for serum Dihydrotestosterone above, within, and below the reference range is calculated for each treatment and are presented in Table 14.2.1.14. These results are also summarized in text Table 11.4.2.3.2.

**ESTRADIOL**

The Estradiol serum concentrations are measured for each subject at each sampling time appear in Tables 14.2.1.15, 14.2.1.16 and 14.2.1.17 according to treatment. The plots of the individual serum levels over the sampling period are presented using both linear (a) and semi-log (b) scales in Figures 14.2.2.51 through 14.2.2.72. Lines for the minimum (3 pg/mL) and maximum (81 pg/mL) bound of the reference range for the Estradiol serum concentrations are also presented for information purposes. As well, a line for the average drug concentration ($C_{avg}$) during the dosing interval ($\tau$=24 hours) is also presented on the individual profiles.

The plots of the mean serum levels over the sampling period are also presented using both the linear (a) and semi-log (b) scales in Figures 14.2.2.73, 14.2.2.74 and 14.2.2.75 for Treatments A, B and C, respectively. The error bars on these mean profiles correspond to one standard deviation. The lines for the minimum and maximum bound of the reference ranges are also presented on the mean figures.

The mean plot on the linear scale for each treatment is also presented below in the text Figure 11.4.2.3-3.

**Figure 11.4.2.3-3: Mean Estradiol Serum Concentration (pg/mL) - Time Profile for Each Treatment**

TBS-1 (125 µL of 4.0 % Gel) (A)
TBS-1 (150 µL of 4.5 % Gel) (B)
TBS-1 (125 µL of 4.5 % Gel) (C)
Serum Concentration (pg/mL)
Time (h)
34
32
30
28
26
24
22
20
18
16
0.0
2.0
4.0
6.0
8.0
10.0
12.0
14.0
16.0
18.0
20.0
22.0
24.0


As per SAP (section 8.3), $AUC_{X-Y}$ is calculated based on the estimated concentration (Y) using the regression line calculated from the elimination phase data when the last concentration (Y) does not correspond to a schedule sampling time. However, for some subjects the elimination phase is not well characterized due to fluctuation in the Estradiol serum concentration as follows:

- Subject No.: 02-007 for the 0 to 10 hours and for the 0 to 24 hours time intervals for Treatment A. The following PK parameters could not be calculated for this subject: $AUC_{0-10}$, $C_{avg\ 0-10}$, $AU_{C0-\tau}$, $C_{avg}$ and PTF for Treatment A (N = 7 for these parameters).

- Subject Nos: 01-002 and 01-007 for the 10 to 16 hours time interval for Treatment A. The $AUC_{10-16}$ and $C_{avg\ 10-16}$ could not be calculated for these subjects for Treatment A (N = 6 for these parameters).

- Subject Nos. 02-004 and 02-007 for the 16 to 24 hours time interval for Treatment A. The $AUC_{16-24}$ and $C_{avg\ 16-24}$ could not be calculated for this subject for Treatment A (N = 6 for these parameters).

- Subject Nos. 02-003 and 02-005 for the 0 to 10 hours time interval for Treatment C. The $AUC_{0-10}$ and $C_{avg\ 0-10}$ could not be calculated for these subjects for Treatment C (N = 5 for these parameters).

Calculated pharmacokinetic parameters for each subject according to treatment are shown in Tables 14.2.1.18, 14.2.1.19 and 14.2.1.20 for Treatments A, B and C, respectively. They are summarized in the text Table 11.4.2.3-3.

Table 11.4.2.3-3: Summary of Estradiol Pharmacokinetic Parameters for Each Treatment

| Parameter | Unit | Treatment A[1] (N = 8) | | | Treatment B[2] (N = 7) | | | Treatment C[3] (N = 7) | | |
|---|---|---|---|---|---|---|---|---|---|---|
| | | Mean | SD | CV% | Mean | SD | CV% | Mean | SD | CV% |
| $AUC_{0-10}^{b,c}$ | h*pg/mL | 234.96 | 95.96 | 40.84 | 242.02 | 64.26 | 26.55 | 267.78 | 75.37 | 28.15 |
| $C_{max\ 0-10}$ | pg/mL | 36.8 | 13.4 | 36.33 | 35.8 | 9.06 | 25.29 | 35.5 | 7.75 | 21.80 |
| $C_{min\ 0-10}$ | pg/mL | 17.7 | 6.43 | 36.35 | 17.4 | 5.67 | 32.63 | 22.1 | 8.07 | 36.43 |
| $C_{avg\ 0-10}^{b,c}$ | pg/mL | 23.5 | 9.60 | 40.84 | 24.2 | 6.43 | 26.55 | 26.8 | 7.54 | 28.15 |
| $T_{max\ 0-10}$ | h | 2.62 | 2.87 | 109.67 | 1.49 | 0.608 | 40.85 | 2.68 | 3.38 | 126.14 |
| $AUC_{10-16}^{d}$ | h*pg/mL | 144.76 | 51.60 | 35.65 | - | - | - | 144.30 | 53.70 | 37.21 |
| $C_{max\ 10-16}$ | pg/mL | 28.9 | 10.8 | 37.29 | - | - | - | 31.5 | 8.82 | 28.02 |
| $C_{min\ 10-16}$ | pg/mL | 16.3 | 5.42 | 33.32 | - | - | - | 19.2 | 8.62 | 45.02 |
| $C_{avg\ 10-16}^{d}$ | pg/mL | 24.1 | 8.60 | 35.65 | - | - | - | 24.0 | 8.95 | 37.21 |
| $T_{max\ 10-16}$ | h | 12.1 | 1.15 | 9.49 | - | - | - | 11.2 | 0.693 | 6.19 |
| $AUC_{10-24}$ | h*pg/mL | - | - | - | 295.12 | 81.19 | 27.51 | - | - | - |
| $C_{max\ 10-24}$ | pg/mL | - | - | - | 30.6 | 8.16 | 26.70 | - | - | - |
| $C_{min\ 10-24}$ | pg/mL | - | - | - | 15.9 | 4.46 | 27.95 | - | - | - |
| $C_{ava\ 10-24}$ | pg/mL | - | - | - | 21.1 | 5.80 | 27.51 | - | - | - |
| $T_{max\ 10-24}$ | h | - | - | - | 12.4 | 1.74 | 14.00 | - | - | - |
| $AUC_{16-24}^{d}$ | h*pg/mL | 153.02 | 42.87 | 28.02 | - | - | - | 177.97 | 48.79 | 27.41 |
| $C_{max\ 16-24}$ | pg/mL | 27.2 | 10.4 | 38.23 | - | - | - | 26.9 | 7.99 | 29.74 |
| $C_{min\ 16-24}$ | pg/mL | 17.4 | 5.75 | 33.11 | - | - | - | 17.0 | 5.65 | 33.28 |
| $C_{avg\ 16-24}$ | pg/mL | 19.1 | 5.36 | 28.02 | - | - | - | 22.2 | 6.10 | 27.41 |
| $T_{max\ 16-24}$ | h | 18.8 | 1.88 | 10.01 | - | - | - | 18.5 | 1.92 | 10.36 |
| $AUC_{0-\tau}^{b}$ | h*pg/mL | 530.27 | 196.81 | 37.12 | 537.16 | 137.99 | 25.69 | 601.91 | 188.18 | 31.26 |
| $C_{max}$ | pg/mL | 37.9 | 13.6 | 35.97 | 36.2 | 8.69 | 24.04 | 36.4 | 8.44 | 23.18 |
| $C_{min}$ | pg/mL | 16.1 | 5.36 | 33.31 | 15.7 | 4.40 | 28.03 | 17.0 | 5.65 | 33.28 |
| $C_{avg}^{b}$ | pg/mL | 22.1 | 8.20 | 37.12 | 22.4 | 5.75 | 25.69 | 25.1 | 7.84 | 31.26 |

(continued)

| Parameter | Unit | Treatment A[1] (N = 8) | | | Treatment B[2] (N = 7) | | | Treatment C[3] (N = 7) | | |
|---|---|---|---|---|---|---|---|---|---|---|
| | | Mean | SD | CV% | Mean | SD | CV% | Mean | SD | CV% |
| $T_{max}$ | h | 4.13 | 7.13 | 172.74 | 4.51 | 5.25 | 116.25 | 4.88 | 5.27 | 107.94 |
| PTF[b] | - | 0.97 | 0.35 | 36.08 | 0.93 | 0.28 | 30.25 | 0.81 | 0.21 | 25.16 |
| PTS | - | 1.36 | 0.48 | 35.44 | 1.35 | 0.49 | 35.88 | 1.21 | 0.31 | 25.44 |
| % TimeBelow* | % | 0.00 | 0.00 | - | 0.00 | 0.00 | - | 0.00 | 0.00 | - |
| % TimeWithin* | % | 100.00 | 0.00 | 0.00 | 100.00 | 0.00 | 0.00 | 100.00 | 0.00 | 0.00 |
| % Time Above* | % | 0.00 | 0.00 | - | 0.00 | 0.00 | - | 0.00 | 0.00 | - |
| $C_{avg}$ Below [b,*] [N (% of Subjects)] | % | 0 (0%) | - | - | 0 (0%) | - | - | 0 (0%) | - | - |
| $C_{avg}$ Within[b,*] [N (% of Subjects)] | % | 7 (100.00%) | - | - | 7 (100.00%) | - | - | 7 (100.00%) | - | - |
| $C_{avg}$ Above[b,*] [N (% of Subjects)] | % | 0 (0%) | - | - | 0 (0%) | - | - | 0 (0%) | - | - |

* Reference Range = 3-81 pg/mL.
1 = TBS-1, 125 μL 4.0% gel given t.i.d. (total dose 30 mg/day)
2 = TBS-1, 150 μL of 4.5% gel given b.i.d. (total dose 27.0 mg/day)
3 = TBS-1, 125 μL of 4.5% gel given t.i.d. (total dose 33.75 mg/day)
b = For these parameters, N = 7 for Treatment A.
c = For these parameters, N = 6 for Treatment A.
d = For these parameters, N = 5 for Treatment C.

The percent times during which observations fall above (% TimeAbove), within (% TimeWithin), and below (% TimeBelow) the reference range are computed for each subject and are presented in Tables 14.2.1.18, 14.2.1.19 and 14.2.1.20 for Treatments A, B and C, respectively. These results are also summarized in text Table 11.4.2.3.3.

The percent of subjects with $C_{avg}$ values for serum Estradiol above, within, and below the reference range is calculated for each treatment and are presented in Table 14.2.1.21. These results are also summarized in text Table 11.4.2.3.3.

***11.4.2.4 Pharmacodynamic Analysis***

No pharmacodynamic analysis is planned or performed during this study.

***11.4.7 Pharmacokinetic and Statistical Conclusions***

In this Phase II study, subjects are randomized into three treatment arms (4.0% TBS-1 administered t.i.d. and 4.5% TBS-1 administered bid. and t.i.d.). The treatments are administered for one week by intra-nasal route, in a parallel design. At the end of one week, the three treatments are compared by conducting a 24 hour pharmacokinetic investigation of the systemic absorption of the drug product Testosterone, and its two physiological metabolites Dihydrotestosterone and Estradiol.

**TESTOSTERONE**

The pharmacokinetic profile of TBS-1 following single and repeat dosing is examined in 2 previous studies (TST-PKP-01-MAT/04 and TST-DF-02-MAT/05). It is demonstrated in these studies that Testosterone is well absorbed following intra-nasal administration. The maximal serum concentration is reached after 1-2 hours post administration. In the current study, the Testosterone formulations (4.0% TBS-1 is administered t.i.d. and 4.5% TBS-1 is administered bid. and t.i.d.) are rapidly absorbed with a peak concentration reached within 36 minutes to 1 hour 6 minutes (mean $T_{max}$) following intra-nasal administration. The maximum Testosterone concentration over the 24-hour interval is observed during the first administration (0-10 hours) in approximately 57% to 71% of the hypogonadal men while approximately 29% to 43% of the subjects had their maximum 24-h Testosterone concentration during the subsequent administrations.

When TBS-1 administrations are compared separately for the t.i.d. treatments, although the mean AUC is similar between formulations, a greater AUC is observed following the first administration compared to the two subsequent administrations ($AUC_{0-10}$: 4178.68 and 4355.19 h*ng/dL > $AUC_{10-16}$: 2635.05 and 2301.51 h*ng/dL < $AUC_{16-24}$: 3016.52 and 2766.97 h*ng/dL for Treatments A and C, respectively). A greater AUC is observed for the second administration when compared to the first administration for Treatment B ($AUC_{0-10}$: 4451.64 h*ng/dL ~ $AUC_{10-24}$: 5264.19 h*ng/dL). The difference in AUC between administrations for both the t.i.d. and b.i.d. formulations could be due to the different time periods elapsed between each administration. The mean $AUC_{0-\tau}$ calculated over the 24-hour dosing interval, is comparable between all treatments ($AUC_{0-\tau}$: 9920.07, 9781.39 and 9505.03 h*ng/dL for Treatments A, B and C, respectively).

Although the mean $C_{max}$ is similar between Treatments A and C, a trend toward a decrease in $C_{max}$ with subsequent administrations is observed ($C_{max\ 0-10}$: 786 and 857 ng/dL > $C_{max\ 10-16}$: 698 and 675 ng/dL > $C_{max\ 16-24}$: 556 and 595 ng/dL for Treatments A and C, respectively). Comparable mean Testosterone $C_{max}$ is observed for both administrations of Treatment B ($C_{max\ 0-10}$: 894 ng/dL ~ $C_{max\ 10-24}$: 846 ng/dL). The difference in $C_{max}$ between administrations for the t.i.d. formulations could be due to the different time periods that are elapsed between each administration. The mean $C_{max}$ calculated over the 24-hour dosing interval, is slightly greater for Treatment B (150 μL of 4.5% gel (b.i.d.)) ($C_{max}$: 1050 ng/dL) comparatively to Treatments A and C ($C_{max}$: 830 and 883 ng/dL, respectively). The upper limit of the physiological reference range (1050 ng/dL) is exceeded by 1 of 8 subjects for Treatment A and 3 of 7 subjects for Treatments B and C.

A trend toward a slight decrease in $C_{avg}$ is observed when administrations are compared separately for t.i.d. and b.i.d. treatments ($C_{avg\ 0-10}$: 418 and 436 ng/dL > $C_{avg\ 10-16}$: 439 and 384 ng/dL > $C_{avg\ 16-24}$: 377 and 346 ng/dL for Treatments A and C, respectively and $C_{avg\ 0-10}$: 445 ng/dL > $C_{avg\ 10-24}$: 376 ng/dL for Treatment B). The difference in $C_{avg}$ between administrations could be due to the different time periods that are elapsed between each administration. The mean $C_{avg}$ calculated over the 24-hour dosing interval, is comparable for all treatments ($C_{avg}$: 413, 408, 396 ng/dL for Treatments A, B and C, respectively).

These results suggest a decrease in exposure (AUC, $C_{avg}$ and $C_{max}$) between each dose for the t.i.d. administrations (Treatments A and C), but not for the b.i.d. administration (Treatment B). This decrease in exposure for the t.i.d. administrations could be partly explained by the negative feedback on endogenous Testosterone production from the HPG axis. In other words, due to the smaller time intervals between each administration for the t.i.d. groups, the recovery of the HPG system from negative feedback would be less that for the b.i.d. group.

Independently of the formulation, approximately 86%-88% of the subjects had an average drug concentration ($C_{avg}$) within the physiological reference range (300 to 1050 ng/dL), 13%-14% of the subjects had a $C_{avg}$ below the reference range and no subjects had a $C_{avg}$ above the reference range.

The period of time during a day (24 hours) for which serum Testosterone concentrations are below, within and above the physiological reference range is covered respectively 30 to 35%, 59% to 68% and 0% of the 24-hour period for all formulations. That is to say that the testosterone levels are within normal range for about 14 to 16 hours a day.

**DIHYDROTESTOSTERONE**

The Dihydrotestosterone peak concentration is reached within 1 hour 24 minutes and 2 hours 23 minutes (mean $T_{max}$) following the TBS-1 administrations.
When TBS-1 administrations are compared separately for the t.i.d. treatments, although the mean AUC is similar between formulations, a trend toward a decrease in AUC with subsequent administrations is observed ($AUC_{0-10}$: 345.77 and 411.10 h*ng/dL > $AUC_{10-16}$: 186.33 and 222.62 h*ng/dL > $AUC_{16-24}$: 269.16 and 275.21 h*ng/dL for Treatments A and C, respectively). Comparable AUC is observed for both administrations of Treatment B ($AUC_{0-10}$: 402.77 h*ng/dL ~ $AUC_{10-24}$: 543.29 h*ng/dL). The difference in AUC between administrations for the t.i.d. formulations could be due to the different time periods elapsed between each administration. The mean $AUC_{0-\tau}$ calculated over the 24-hour dosing interval, is comparable between all treatments ($AUC_{0-\tau}$: 818.95, 946.89 and 909.68 h*ng/dL for Treatments A, B and C, respectively).
Although the mean $C_{max}$ is similar between the t.i.d. formulations, a trend toward a decrease in $C_{max}$ with subsequent administrations is observed ($C_{max\ 0-10}$: 51.4 and 59.0 ng/dL > $C_{max\ 10-16}$: 44.2 and 48.9 ng/dL > $C_{max\ 16-24}$: 41.3 and 42.6 ng/dL for Treatments A and C, respectively). Comparable mean Testosterone $C_{max}$ is observed for both administrations of Treatment B ($C_{max\ 0-10}$: 56.8 ng/dL ~ $C_{max\ 10-24}$: 54.6 ng/dL). The difference in $C_{max}$ between administrations for the t.i.d. formulations could be due to the different time periods elapsed between each administration. The mean $C_{max}$ is calculated over the 24-hour dosing interval, is comparable for all treatments ($C_{max}$: 52.2, 61.0 and 60.3 ng/dL for Treatments A, B and C, respectively). The upper limit of the physiological reference range (97.8 ng/dL) is not exceeded by any subjects for any treatment.

The $C_{avg}$ calculated by administration are comparable between treatments and administrations ($C_{avg\ 0-10}$: 34.6 and 41.1 ng/dL > $C_{avg\ 10-16}$: 31.1 and 37.1 ng/dL > $C_{avg\ 16-24}$: 33.6 and 34.4 ng/dL for Treatments A and C, respectively and $C_{avg\ 0-10}$: 40.3 ng/dL > $C_{avg\ 10-24}$: 38.8 ng/dL for Treatment B). The mean $C_{avg}$ calculated over the 24-hour dosing interval, is comparable for all treatments ($C_{avg}$: 34.1, 39.5, 37.9 ng/dL for Treatments A, B and C, respectively).

Approximately 63% of subjects had their $C_{avg}$ included in the physiological reference range for DHT (25.5 to 97.8 ng/dL) following administration of Treatment A, whereas this number rises to about 86% when Treatments B and C are administered. No subject had their $C_{avg}$ above the normal range while 38% and 14% of the subjects have their $C_{avg}$ below the normal range for Treatment A and both Treatments B and C, respectively.

The period of time during a day (24 hours) for which serum DHT concentrations are below, within and above the physiological reference range is covered respectively 32.64%, 67.36% and 0% for Treatment A, 26.22%, 73.78% and 0% for Treatment B and 13.87%, 86.13% and 0% for Treatment C. That is to say that the DHT levels are within normal range for about 16, 18 and 21 hours a day for Treatments A, B and C, respectively.

**ESTRADIOL**

The Estradiol peak concentration is reached within 1 hour 12 minutes and 2 hours 41 minutes (mean $T_{max}$) following the TBS-1 administrations.

When TBS-1 administrations are compared separately for the t.i.d. treatments, although the mean AUC is similar between formulations, a trend toward a decrease in AUC with subsequent administrations is observed ($AUC_{0-10}$: 234.96 and 267.78 h*pg/mL > $AUC_{10-16}$: 144.76 and 144.30 h*pg/mL < $AUC_{16-24}$: 153.02 and 177.97 h*pg/mL for Treatments A and C, respectively). Comparable AUC is observed for both administrations of Treatment B ($AUC_{0-10}$: 242.02 h*pg/mL ~ $AUC_{10-24}$: 295.12 h*pg/mL). The difference in AUC between administrations for the t.i.d. formulations could be due to the different time periods elapsed between each administration. The mean $AUC_{0-\tau}$ calculated over the 24-hour dosing interval, is comparable between all treatments ($AUC_{0-\tau}$: 530.27, 537.16 and 601.91 h*pg/mL for Treatments A, B and C, respectively).

Although the mean $C_{max}$ is similar between the t.i.d. formulations, a trend toward a decrease in $C_{max}$ with subsequent administrations is observed ($C_{max\ 0-10}$: 36.8 and 35.5 pg/mL > $C_{max\ 10-16}$: 28.9 and 31.5 pg/mL > $C_{max\ 16-24}$: 27.2 and 26.9 pg/mL for Treatments A and C, respectively). Comparable mean Testosterone $C_{max}$ is observed for both administrations of Treatment B ($C_{max\ 0-10}$: 35.8 pg/mL ~ $C_{max\ 10-24}$: 30.6 pg/mL). The difference in $C_{max}$ between administrations for the t.i.d. formulations could be due to the different time periods elapsed between each administration. The mean $C_{max}$ calculated over the 24-hour dosing interval, is comparable for all treatments ($C_{max}$: 37.9, 36.2 and 36.4

pg/mL for Treatments A, B and C, respectively). The upper limit of the physiological reference range (81 pg/mL) is not exceeded by any subjects for any treatment.

e $C_{avg}$ calculated by administration are comparable between treatments and administrations ($C_{avg\ 0-10}$: 23.5 and 26.8 pg/mL > $C_{avg\ 10-16}$: 24.1 and 24.0 pg/mL > $C_{avg\ 16-24}$: 19.1 and 22.2 pg/mL for Treatments A and C, respectively and $C_{avg\ 0-10}$: 24.2 pg/mL > $C_{avg\ 10-24}$: 21.1 pg/mL for Treatment B). The mean $C_{avg}$ is calculated over the 24-hour dosing interval, is comparable for all treatments ($C_{avg}$: 22.1, 22.4, 25.1 pg/mL for Treatments A, B and C, respectively).

All subjects have their $C_{avg}$ included in the physiological reference range for $E_2$ (3 to 81 pg/mL) following administration of all treatments. All subjects have $E_2$ concentrations within the normal range over the 24 hours period. No subjects have $E_2$ levels below or above the normal range at any time of the day.

## 12. Safety Evaluation

### 12.1 Extent of Exposure

Subjects use the drug for 7 days at two sites and 8 days in another.

### 12.2 Adverse Events

#### 12.2.1 Brief Summary of Adverse Events

There are eight adverse events that occurred in six subjects. Six of the events occur during treatment A and two occur during treatment B. Subjects 01-002 and 01 - 007 both experience dizziness and both are indicated as possibly related to the study drug. Subject 01-002 has moderate severity which resolved after 5 days. Seven of the 8 adverse events are mild. Six of the 8 events are not related to study drug.

Individual 02-004 is classified as having anemia by the investigator. The hemoglobin is at the minimal normal level and is deemed unrelated to the drug. Table 12.2.2 summarizes the events.

#### 12.2.2 Display of Adverse Events

**Table 12.2.2: Adverse Events**

| Treatment | Subject | Age | Preferred Term | Severity | Relation to Drug | Start Date | End Date | Duration (days) |
|---|---|---|---|---|---|---|---|---|
| A | 01-002 | 40 | Dizziness | MODERATE | POSSIBLY RELATED | 2010-10-25 | 2010-10-30 | 5 |
| A | 01-007 | 49 | Dizziness | MILD | POSSIBLY RELATED | 2010-10-23 | 2010-10-28 | 5 |
| A | 02-004 | 53 | Anemia | MILD | NOT RELATED | 2010-10-04 | | |
| A | 03-006 | 73 | Pain of skin | MILD | NOT RELATED | 2010-09-27 | 2010-11-04 | 37 |
| A | 03-006 | 73 | Excoriation | MILD | NOT RELATED | 2010-09-02 | 2010-11-04 | 62 |
| A | 03-006 | 73 | Excoriation | MILD | NOT RELATED | 2010-09-27 | 2010-11-04 | 37 |
| B | 03-001 | 59 | Respiratory tract congestion | MILD | NOT RELATED | 2010-09-05 | 2010-09-13 | 8 |
| B | 03-005 | 62 | Gastrooesophageal reflux disease | MILD | NOT RELATED | 2010-09-14 | 2010-09-27 | 13 |

## 12.2.4 Listing of Adverse Events by Subjects

Table 12.2.2 list of adverse events by subject.

## 12.3 Deaths, Other Serious Adverse Events, and Other Significant Adverse Events

There are no deaths, other serious adverse events or other significant adverse events during the course of this study.

## 12.4.2 Evaluation of Each Laboratory Parameter

There are no clinically significant changes in laboratory values from the beginning to the end of the study as determined by the principle investigators. All subjects did have some abnormal values at the initial visit and/or at the third visit. There are no consistent changes throughout the visits.

Subject 01-007 had a uric acid level of 539 U/L with 289 as the upper end of normal at the third visit. There are elevated glucose values in about half the subjects compared to a normal first visit value. This is spread across all three dosages and are only slightly elevated. There is no clinical significance.

## 12.5 Vital Signs, Physical Findings, and Other Observations Related to Safety

There are no meaningful or significant changes in vital signs after test drug administration.

## 12.6 Safety Conclusions

The TBS-1 gel demonstrates in this and other studies that it is safe for use. There are no serious adverse events or any events of consequence during this PK study or during the seven days of self administration. Tables 14.3.2.1 through 14.3.2.8 show all the laboratory values for visit 1 and visit 3.

## 13. Discussion and Overall Conclusions

The primary objective of this study is to determine the bioavailability of a 4.0 % TBS-1 gel (applied t.i.d.) and 4.5 % TBS-1 gel (applied b.i.d. and t.i.d.) in hypogonadal men.

In a previous study, Nasobol-01-2009, a 3.2% Testosterone gel is used to deliver 4.0 mg, 5.5 mg and 7.0 mg of Testosterone intra-nasally using gel volumes of 125 μL, 172 μL and 219 μL, respectively. In this study, 5.0 mg, 5.65 mg and 6.75 mg of Testosterone are administered in gel volumes of 125 μL, 125 μL, and 150 μL, respectively. This study allowed investigating the delivery of similar Testosterone amounts in much smaller volumes.

The secondary objective of this study is to establish a safety profile for TBS-1. In this Phase II study, subjects are randomized into three treatment arms (4.0% TBS-1 administered t.i.d. and 4.5% TBS-1 administered bid. and t.i.d.). The treatments are administered for one week by intra-nasal route, in a parallel design. At the end of one week, the three treatments are compared by conducting a 24 hour pharmacokinetic investigation of the systemic absorption of the drug product Testosterone, and its two physiological metabolites Dihydrotestosterone and Estradiol.

There are eight adverse events described by six subjects. Six of the events occurred during treatment A and two occurred during treatment B. Subjects 01-002 and 01-007 both experienced dizziness and both are indicated as possibly related to the study drug. The remainder are unrelated to study drug.

There are no vital signs or laboratory changes that are significant or meaningful. No erythrocytosis, anemia or infections are observed after measurement of complete blood counts at screening and close-out. Clinical chemistry and urinalysis showed no changes at close-out in hypo or hyperglycemia, renal function, liver function, skeletal/heart muscle damage or changes in calcium homeostasis. There are no clinically significant changes to the nasal mucosa.

The PK population is defined as subjects who received the Treatment A, B or C, and who completed the study without major protocol violation or for whom the PK profile can be adequately characterized. The PK population is used for the analysis of PK data. Based on these criteria, twenty-two (22) subjects are included in the PK population.

## TESTOSTERONE

The pharmacokinetic profile of TBS-1 following single and repeat dosing is examined in 2 previous studies (TST-PKP-01-MAT/04 and TST-DF-02-MAT/05). It is demonstrated in these studies that Testosterone is well absorbed following intra-nasal administration. The maximal serum concentration is reached after 1-2 hours post administration. In the current study, the Testosterone formulations (4.0% TBS-1 administered t.i.d. and 4.5% TBS-1 administered bid.

and t.i.d.) are rapidly absorbed with a peak concentration reached within 36 minutes to 1 hour 6 minutes (mean $T_{max}$) following intra-nasal administration. The maximum Testosterone concentration over the 24-hour interval is observed during the first administration (0-10 hours) in approximately 57% to 71% of the hypogonadal men while approximately 29% to 43% of the subjects had their maximum 24-h Testosterone concentration during the subsequent administrations.

When TBS-1 administrations are compared separately for the t.i.d. treatments, although the mean AUC is similar between formulations, a greater AUC is observed following the first administration compared to the two subsequent administrations ($AUC_{0-10}$: 4178.68 and 4355.19 h*ng/dL > $AUC_{10-16}$: 2635.05 and 2301.51 h*ng/dL < $AUC_{16-24}$: 3016.52 and 2766.97 h*ng/dL for Treatments A and C, respectively). A greater AUC is observed for the second administration when compared to the first administration for Treatment B ($AUC_{0-10}$: 4451.64 h*ng/dL ~ $AUC_{10-24}$: 5264.19 h*ng/dL). The difference in AUC between administrations for both the t.i.d. and b.i.d. formulations could be due to the different time periods elapsed between each administration. The mean $AUC_{0-t}$ calculated over the 24-hour dosing interval, is comparable between all treatments ($AUC_{0-t}$: 9920.07, 9781.39 and 9505.03 h*ng/dL for Treatments A, B and C, respectively).

When TBS-1 administrations are compared separately for the t.i.d. treatments, although the mean $C_{max}$ is similar between formulations, a trend toward a decrease in $C_{max}$ with subsequent administrations is observed ($C_{max\ 0-10}$: 786 and 857 ng/dL > $C_{max\ 10-16}$: 698 and 675 ng/dL > $C_{max\ 16-24}$: 556 and 595 ng/dL for Treatments A and C, respectively). Comparable mean Testosterone $C_{max}$ is observed for both administrations of Treatment B ($C_{max\ 0-10}$: 894 ng/dL ~ $C_{max\ 10-24}$: 846 ng/dL). The difference in $C_{max}$ between administrations for the t.i.d. formulations could be due to the different time periods elapsed between each administration. The mean $C_{max}$ calculated over the 24-hour dosing interval, is slightly greater for Treatment B (150 μL of 4.5% gel (b.i.d.)) ($C_{max}$: 1050 ng/dL) comparatively to Treatments A and C ($C_{max}$: 830 and 883 ng/dL, respectively). The upper limit of the physiological reference range (1050 ng/dL) is exceeded by 1 of 8 subjects for Treatment A and 3 of 7 subjects for Treatments B and C.

A trend toward a slight decrease in $C_{avg}$ is observed when administrations are compared separately for t.i.d. and b.i.d. treatments ($C_{avg\ 0-10}$: 418 and 436 ng/dL > $C_{avg\ 10-16}$: 439 and 384 ng/dL > $C_{avg\ 16-24}$: 377 and 346 ng/dL for Treatments A and C, respectively and $C_{avg\ 0-10}$: 445 ng/dL > $C_{avg\ 10-24}$: 376 ng/dL for Treatment B). The difference in $C_{avg}$ between administrations could be due to the different time periods elapsed between each administration. The mean $C_{avg}$ calculated over the 24-hour dosing interval, is comparable for all treatments ($C_{avg}$: 413, 408, 396 ng/dL for Treatments A, B and C, respectively).

These results suggest a decrease in exposure (AUC, $C_{avg}$ and $C_{max}$) between each dose for the t.i.d. administrations (Treatments A and C), but not for the b.i.d. administration (Treatment B). This decrease in exposure for the t.i.d. administrations could be partly explained by the negative feedback on endogenous Testosterone production from the HPG axis. In other words, due to the smaller time intervals between each administration for the t.i.d. groups, the recovery of the HPG system from negative feedback would be less that for the b.i.d. group.

Independently of the formulation, approximately 86%-88% of the subjects had an average drug concentration ($C_{avg}$) within the physiological reference range (300 to 1050 ng/dL), 13%-14% of the subjects had a $C_{avg}$ below the reference range and no subjects had a $C_{avg}$ above the reference range.

The period of time during a day (24 hours) for which serum Testosterone concentrations are below, within and above the physiological reference range covered respectively 30 to 35%, 59% to 68% and 0% of the 24-hour period for all formulations. That is to say that the Testosterone levels are within normal range for about 14 to 16 hours a day.

**DIHYDROTESTOSTERONE**

The Dihydrotestosterone peak concentration is reached within 1 hour 24 minutes and 2 hours 23 minutes (mean $T_{max}$) following the TBS-1 administrations.

When TBS-1 administrations are compared separately for the t.i.d. treatments, although the mean AUC is similar between formulations, a trend toward a decrease in AUC with subsequent administrations is observed ($AUC_{0-10}$: 345.77 and 411.10 h*ng/dL > $AUC_{10-16}$: 186.33 and 222.62 h*ng/dL > $AUC_{16-24}$: 269.16 and 275.21 h*ng/dL for Treatments A and C, respectively). Comparable AUC is observed for both administrations of Treatment B ($AUC_{0-10}$: 402.77 h*ng/dL ~ $AUC_{10-24}$: 543.29 h*ng/dL). The difference in AUC between administrations for the t.i.d. formulations could be due to the different time periods elapsed between each administration. The mean $AUC_{0-t}$ calculated over the 24-hour dosing interval, is comparable between all treatments ($AUC_{0-t}$: 818.95, 946.89 and 909.68 h*ng/dL for Treatments A, B and C, respectively).

Although the mean $C_{max}$ is similar between the t.i.d. formulations, a trend toward a decrease in $C_{max}$ with subsequent administrations is observed ($C_{max\ 0-10}$: 51.4 and 59.0 ng/dL > $C_{max\ 10-16}$: 44.2 and 48.9 ng/dL > $C_{max\ 16-24}$: 41.3 and 42.6 ng/dL for Treatments A and C, respectively). Comparable mean Testosterone $C_{max}$ is observed for both administrations of Treatment B ($C_{max\ 0-10}$: 56.8 ng/dL ~ $C_{max\ 10-24}$: 54.6 ng/dL). The difference in $C_{max}$ between administrations for the t.i.d. formulations could be due to the different time periods elapsed between each administration. The mean $C_{max}$ calculated over the 24-hour dosing interval, is comparable for all treatments ($C_{max}$: 52.2, 61.0 and 60.3 ng/dL

for Treatments A, B and C, respectively). The upper limit of the physiological reference range (97.8 ng/dL) is not exceeded by any subjects for any treatment.

The $C_{avg}$ calculated by administration are comparable between treatments and administrations ($C_{avg\ 0-10}$: 34.6 and 41.1 ng/dL > $C_{avg\ 10-16}$: 31.1 and 37.1 ng/dL > $C_{avg\ 16-24}$: 33.6 and 34.4 ng/dL for Treatments A and C, respectively and $C_{avg\ 0-10}$: 40.3 ng/dL > $C_{avg\ 10-24}$: 38.8 ng/dL for Treatment B). The mean $C_{avg}$ calculated over the 24-hour dosing interval, is comparable for all treatments ($C_{avg}$: 34.1, 39.5, 37.9 ng/dL for Treatments A, B and C, respectively).

Approximately 63% of subjects had their $C_{avg}$ included in the physiological reference range for DHT (25.5 to 97.8 ng/dL) following administration of Treatment A, whereas this number rises to about 86% when Treatments B and C are administered. No subject had their $C_{avg}$ above the normal range while 38% and 14% of the subjects had their $C_{avg}$ below the normal range for Treatment A and both Treatments B and C, respectively.

The period of time during a day (24 hours) for which serum DHT concentrations are below, within and above the physiological reference range covered respectively 32.64%, 67.36% and 0% for Treatment A, 26.22%, 73.78% and 0% for Treatment B and 13.87%, 86.13% and 0% for Treatment C. That is to say that the DHT levels are within normal range for about 16, 18 and 21 hours a day for Treatments A, B and C, respectively.

**ESTRADIOL**

The Estradiol peak concentration is reached within 1 hour 12 minutes and 2 hours 41 minutes (mean $T_{max}$) following the TBS-1 administrations.

When TBS-1 administrations are compared separately for the t.i.d. treatments, although the mean AUC is similar between formulations, a trend toward a decrease in AUC with subsequent administrations is observed ($AUC_{0-10}$: 234.96 and 267.78 h*pg/mL > $AUC_{10-16}$: 144.76 and 144.30 h*pg/mL < $AUC_{16-24}$: 153.02 and 177.97 h*pg/mL for Treatments A and C, respectively). Comparable AUC is observed for both administrations of Treatment B ($AUC_{0-10}$: 242.02 h*pg/mL ~ $AUC_{10-24}$: 295.12 h*pg/mL). The difference in AUC between administrations for the t.i.d. formulations could be due to the different time periods elapsed between each administration. The mean $AUC_{0-t}$ calculated over the 24-hour dosing interval, is comparable between all treatments ($AUC_{0-t}$: 530.27, 537.16 and 601.91 h*pg/mL for Treatments A, B and C, respectively).

Although the mean $C_{max}$ is similar between the t.i.d. formulations, a trend toward a decrease in $C_{max}$ with subsequent administrations is observed ($C_{max\ 0-10}$: 36.8 and 35.5 pg/mL > $C_{max\ 10-16}$: 28.9 and 31.5 pg/mL > $C_{max\ 16-24}$: 27.2 and 26.9 pg/mL for Treatments A and C, respectively). Comparable mean Testosterone $C_{max}$ is observed for both administrations of Treatment B ($C_{max\ 0-10}$: 35.8 pg/mL ~ $C_{max\ 10-24}$: 30.6 pg/mL). The difference in $C_{max}$ between administrations for the t.i.d. formulations could be due to the different time periods elapsed between each administration. The mean $C_{max}$ calculated over the 24-hour dosing interval, is comparable for all treatments ($C_{max}$: 37.9, 36.2 and 36.4 pg/mL for Treatments A, B and C, respectively). The upper limit of the physiological reference range (81 pg/mL) is not exceeded by any subjects for any treatment.

The $C_{avg}$ calculated by administration are comparable between treatments and administrations ($C_{avg\ 0-10}$: 23.5 and 26.8 pg/mL > $C_{avg\ 10-16}$: 24.1 and 24.0 pg/mL > $C_{avg\ 16-24}$: 19.1 and 22.2 pg/mL for Treatments A and C, respectively and $C_{avg\ 0-10}$: 24.2 pg/mL > $C_{avg\ 10-24}$: 21.1 pg/mL for Treatment B). The mean $C_{avg}$ calculated over the 24-hour dosing interval, is comparable for all treatments ($C_{avg}$: 22.1, 22.4, 25.1 pg/mL for Treatments A, B and C, respectively).
All subjects had their $C_{avg}$ included in the physiological reference range for $E_2$ (3 to 81 pg/mL) following administration of all treatments. All subjects had $E_2$ concentrations within the normal range over the 24 hours period. No subjects had $E_2$ levels below or above the normal range at any time of the day.

**CONCLUSIONS**

The TBS-1 formulations (4.0 % TBS-1 gel (applied t.i.d.) and 4.5 % TBS-1 gel (applied b.i.d. and t.i.d.)) are rapidly absorbed with mean Testosterone peak observed within 1 hour.

Overall, the Testosterone exposure ($AUC_{0-t}$ and $C_{max}$) at steady-state is comparable between all treatments. Independently of the formulation, approximately 86%-88% of the subjects had an average Testosterone drug concentration ($C_{avg}$) within the physiological reference range (300 to 1050 ng/dL).

The Testosterone levels are within normal range for about 14 to 16 hours a day.

TBS-1 is safe for intranasal administration at the dosages and frequency indicated. There are no meaningful adverse events, changes in vital signs or changes in laboratory results when compared to baseline.
Based on these results, no clear evidence is found to indicate a better performance from one of the formulations.

**EXAMPLE 9**

**TBS1A Report for 4% and 8% Bulk Gel**

***Objective:***

To follow up on IMP- Clinical batch manufacture. Main points concern process flow and bulk appearance on stability.

- Process flow improvement
- Viscosity of bulk Gel
- Stability (re-crystallization)
- Evaluation of alternate materials sources and grades
- In Vivo results, formulation changes to impact onset of release
- Testing of trials using Franz Cell, trial selection

List of Raw-materials identified for use in trials:

| Material name | Grade | Spec # | Source | Comments |
|---|---|---|---|---|
| | | | | |
| Castor Oil | (Crystal O) | RM004A | Cas-Chem | |
| Castor Oil | (Virgin) | RM004B | - | |
| Labrafil | M1944CS | RM002A | Gattfosse | |
| DMI | ----------- | RM009A | Croda | |
| Transcutol P | ----------- | RM008A | Gattfosse | |
| Plasdone | K17 | RM011A | ISP | |
| Plasdone | S630 | RM013A | ISP | |
| Plasdone | K29-32 | Sample | ISP | |
| Plasdone | K90 | Sample | ISP | |
| HPC | Klucel HF | RM014A | Hercules | |
| HPC | Nisso H | Sample | Nisso | |
| HPC | Nisso M | Sample | Nisso | |
| HPC | Nisso L | Sample | Nisso | |
| Cab-O-Sil | M-5P | RM003A | Cabot | |
| Aerosil | 200 | RM003B | Evonik | |
| Purified water | ----------- | ----------- | Trimel | |
| Testosterone | micronized | RM001A | Proquina | |
| Oleic Acid | Super-refined | sample | Croda | |
| Testosterone | Not micronized | RM | Proquina | |

***Equipment used:***

In addition to the Silverson High Shear mixer, used only during the manufacture of the TBS1A IMP Clinical

batches, included also a propeller type mixing unit for the trials on several pre-mix operations. The only application for the High shear action is for dispersion of the active in the Co-Solvents.

For more uniform mixing and control of temperature, recommend a jacketed container with wiping blades to remove material from inner bowl wall (especially critical for uniform bulk temperature during heating as well as cooling cycles.

***Background info on IMP bath manufacture***

Observation during the IMP Clinical batch manufacture included high viscosity during preparing the pre-mixture of the DMI/Transcutol co-solvent mix consisting of PVP K17/S640, Klucel HF and Testosterone micronized. Mixture resulting in a sticky mass when added to the Castor oil using the high shear mixer set up. With the same high shear mixer set up for the addition of the Cab-O-Sil (referenced in future to SiO2) could not obtain a vortex to incorporate the material and required additional manual mixing during addition stage, hence the recommendation for propeller type mixing unit). Even though the material was viscous during that addition stage, on further mixing the viscosity of the final Bulk Gel dropped to approximately 1,500 - 2,000 cps. Mixing time and speed had to be controlled not to overshoot targeted gel temperature (no cooling system).

***Outline of trials:***

The initial trials (Placebo) concentrated on changing the order of addition to identify impact on viscosity. Previous process included the addition of the SiO2 at the final stage (see comments above), changed to dispersion of the SiO2 into the Castor oil prior to addition of the alternate active mixture. The resulting viscosity of the Castor Oil/SiO2 mixture, used various percentages, increased with the addition of a small percentage of Arlasolve (DMI).

Next step was to duplicate these results using the active mixture (Co-solvents/PVP/HPC/active) and added that mixture to the premix of Castor oil and SiO2. This however resulted in a low viscosity solution, indicating an impact of the active mixture on formation of a viscous gel.

Since the co-solvent mix without additional materials resulted in an increase of viscosity, the quantities of solvent were split into 2 parts, adding part of the solvent mix only to the Oil mixture and remaining solvent mix used to disperse the PVP,HPC and active. The active mixture with the reduced co-solvent ended up more viscose, plus similar low viscosity when added to the castor Oil premix. Additional trials included the prep of active in only DMI (no PVP) and obtained good viscosity. HPC was prepared separately in the Transcutol P, creating problems of stringing when added to the mixture (similar to IMP observations). Addition of SiO2 at a level of 0.1 - 0.3% resolved the problem.

The above process to dissolve active in the Co-solvents is sufficient and doesn't require PVP to increase solubility for the 4% formulation, however not sufficient co-solvents in the formulation to achieve solubility for the 8% strength. Trials on the 8% included an alternate successful approach for preparing the active dispersion containing PVP by including SiO2 into that mixture. As demonstrated on evaluation trials evaluating impact of SiO2 added to the DMI as well as Transcutol P, resulted in good viscosity forming with DMI, however not with Transcutol. Active dispersion therefore id prepared by dissolving the PVP in DMI only, followed by addition of the active at 55 C (50-60C) and portion of available SiO2.

Please note that this process was only developed during the trial work on the 8%, hence it can be scaled down to the 4% strength if PVP indicate additional functionality (Franz Cell test).

Comments related to addition of purified water (noted in Table xxx) indicate increase in viscosity with trials containing HPC, no viscosity increase in trials using only PVP. These trials were only included for information to study water uptake and impact on viscosity after application into the nasal cavity.

Critical step during HPC set up is to provide at least 24 hours of solvating to obtain a clear solution.

As outlined in the trial objectives, formulation ratios were implemented using also alternate grades and sources of materials and are identified in the formulation table. To identify the impact of the process change (such as reaction of viscosity increase adding the co-solvents), performed trials to study impact if related to DMI or Transcutol P. Trials were initiated to disperse SiO2 (at the same ratio as used for Castor Oil mixture) in DMI only as well as in Transcutol P only. The Mixture with the DMI resulted in a viscous mixture while Transcutol P mixture was very fluid.

Similar trials were initiated to use the co-solvents individually to study solubility of the Polymers as well as active for potential reduction in Transcutol P. No noticeable difference in solubility using the mixture or individual solvents at the 4% strength. However, if PVP and HPC are prepared only in DMI, observed separation of the two materials when stored overnight (not apparent when mixed in the co-solvent mixture). To eliminate the stickiness of the dispersion when adding the active/polymer mixture, removed the HPC from the formulation and using PVP only (individual grades K17-K29/32-K90, no mixtures). This resulted in various degrees of viscosity related to the grade used.

Material also included the use of Labrafil M 1944 CS and are outlined in batch description and selected for testing in Franz Cell.

***Comments:***

The various trials are outlined below for 4 % strength as well as 8%.
Trial lots of both strength have been selected for testing on the Franz Cell. Selected lots are identified.

All trials will be monitored for physical evidence of re-crystallization and change in appearance (separation), tested for change in viscosity. Viscosity values of the trials will be documented and updated

Pending Franz Cell result evaluation, optimization of formulation and process can be implemented. This is critical to identify since the trial outline did not include impact on viscosity related to all process parameters (need to include analytical testing and stability data).

Observations during viscosity test using the Brookfield Viscometer Model DV-II+, with Spindle #6, at 50 rpm for 30 seconds, did actually show an increase in viscosity values over the test time in samples prepared with higher viscosity grade HPC. This can be attributed to the stickiness of the Gel causing agglomeration to the spindle shaft and disk creating a drag (not a true viscosity value of the results reported). The bulk Gel of several trials is not thixotropic. Also tested on some trials viscosity at 37 C.
Tested several trials using the new Haupt method with spindle 4 at 6 rpm.

The various attached tables show the trial numbers for active Gels, pre-mixes and Placebos

***Discussion and Considerations for follow up trials with both strength***

Even though ’viscosity improvement’ was not the primary target to initiate trials, it was certainly a designed effort to study the cause for low viscosity considering the high percentage of SiO2 present in the formulation. A cross check against SiO2 alternate source comparison did not indicate major differences, nor did various ratios of Co-Solvents, limited adjustment since a certain percentage required to dissolve the Testosterone. Changes in grades of PVP indicated impact on viscosity when used in the active dispersion, however not when added to the rest of the mixture. Changes in grades of HPC (used alternate source of fine material) showed impact on the final Gel, however the higher the Molecular weight of the HPC, impact of stickiness and stringing in the final Gel. Testing viscosity after several weeks did show a separation in the Gel of viscose settlement on the bottom of the container.

With indication of SiO2 retaining Testosterone, adding more to increase viscosity was not an option, aim was to reduce the % used. especially for the TBS1A 4 % strength which indicated a much higher percentage of T retained compared to the 8% TBS1A. Target was to at least obtain the same ratio of SiO2 to T of the 8% strength for the 4 % strength (hence aimed for scale down to 3 %). With the trials completed and showing impact on viscosity related to process and formulation changes, a reduction in SiO2 for the definitely possible for the 4 % strength that would also include the use of PVP in the formulation by taking advantage of the process change on the 8% strength. The above is only based on viscosity; however impact on the changes in formulation to slow down initial absorption rate in vivo can only be evaluated from the data obtained on the trials used for the analytical test using the Franz Cell. These results will be reviewed and evaluated with potential recommendations for further trials to either duplicate earlier trials or based on DOE.

The attached Tables for viscosity show the date of manufacture and latest test results (to help with trial selection on Franz Cell). In the Comment column original data will be reference or referenced in the Trial process description.

Further alternate material source evaluation is recommended once a primary formulation and process for each strength has been established for direct comparison.

**Formulation/composition of TBS1A - 4%**

**Table 1A (See the formulations in the Examples above and including Example 10)**

| Trial number | Active % | Castor oil % | Labrafil % | PVP grade % | DMI % | TranscutolP % | HPC Nisso % | SiO2 % C=Cabosil A=Aerosil200 |
|---|---|---|---|---|---|---|---|---|
| RD11037 | 4 | 52 | 000000 | K17 = 3<br>S630 = 2 | 25 | 10 | 0000000 | C=4 |
| RD11038 | 4 | 57 | 000000 | K17 = 3<br>S630 = 2 | 20 | 10 | 0000000 | C=4 |
| RD11039 | 4 | 29 | 29 | K17 = 3<br>S630 = 2 | 20 | 10 | 0000000 | C = 3 |
| RD11040 | 4 | 57 | 0000000 | 0000000 | 25 | 10 6 + 4 | 00000000 | C=4 |
| RD11041 | 4 | 53 | 0000000 | K17 = 3<br>S630 = 2 | 25 | 10 6 + 4 | 0000000 | C = 3 |
| RD11042 | 4 | 29 | 29 | 00000000 | 25 | 10 6 + 4 (split) | 000000 | C = 3 |
| RD11050 | 4 | 66.7 | 000000 | K17 = 3 | 24 20 + 4 | 0000000 | N-H = 0.3 | A = 2 |
| RD11050A | 4 | 66.7 | 000000 | K17 = 3 | 24 20 + 4 | 0000000 | N-H = 0.3 | 1 % additional to final 11050 |
| RD11051 | 4 | 66.7 | 000000 | K30 = 3 | 24 20 + 4 | 0000000 | N-M = 0.3 | A = 2 |
| RD11051A | 4 | 66.7 | 000000 | K30 = 3 | 24 20 + 4 | 0000000 | N-M = 0.3 | 1 % additional to final 11051 |
| RD11053 | 4 | 61.7 | 000000 | K17 = 3 | 22 16 +6 | 6 4+2 | N-H = 0.3 | A = 3 |
| RD11054 | 4 | 61.4 | 000000 | K30 = 3 | 23 16 +7 | 5 4+1 | N-M = 0.6 | A = 3 |
| RD11055 | 4 | 62.0 | 000000 | K90 = 3 | 23 16 +7 | 5 4 + 1 | 0000000 | C = 3 |
| RD11056 | 4 | 62.0 | 000000 | K90 = 3 | 28 20 + 8 | 00000 | 0000000 | C = 3 |
| RD11059 | 4 | 75.0 | 000000 | K30 = 2.5 | 14 10 + 4 | 2 | 0000000 | C = 2.5 |
| RD11060 | 4 | 71.5 | 000000 | K30 = 2.0 | 18 9 + 9 | 1 | 00000000 | C= 3.5 |
| RD11061 | 4 | 71.0 | 2 | K17 = 2 | 16 | 2 | 0000000 | C=3 |
| RD11062 | 4 | 62.35 | 0000000 | K17=1.5<br>K30=1.0 | 22 6+16 | 6 2+4 | N-H = 0.15 | A=3 |
| RD11063 | 4 | 70.5 | 00000oo | K17=1.5<br>K30=1.5 | 18 6 + 12 | 00000000 | N-H = 0.2 | A=4 |

(continued)

| ***Trial number*** | ***Active %*** | ***Castor oil %*** | ***Labrafil %*** | ***PVP grade %*** | ***DMI %*** | ***TranscutolP %*** | ***HPC Nisso %*** | ***SiO2 % C=Cabosil A=Aerosil200*** |
|---|---|---|---|---|---|---|---|---|
| RD11064 | Transfer from RD11062 | Add 0.3% H2O | Increase in viscosity | | | | Formula includes HPC | |
| RD11065 | Transfer from RD11063 | Add 0.3% H2O | Increase in viscosity | | | | Formula includes HPC | |
| RD11066 | Transfer from RD11041 | Add 0.3% H2O | No increase in viscosity | | | | NO HPC | |
| RD11070 | Transfer from RD11037 | Add 0.3% H2O | No increase in viscosity | | | | NO HPC | |
| RD11071 | Transfer from RD11042 | Add 0.3% H2O | No increase in viscosity | | | | NO HPC | |
| RD11072 | Transfer from RD11040 | Add 0.3% H2O | No increase in viscosity | | | | NO HPC | |
| RD11073 | 4 | 70.5 | 000000 | 0000000 | 16 10 +6 (3) | 6 (3) | N-M=0.5 (0.25) | A=3 |
| RD11074 | Transfer from RD11073 | Add 0.3% H2O | | | | | Transfer from RD11040 | Add 0.3% H2O |
| RD11075 (base) | 4 | 68.0 | 000000 | K30 =1.0 | 16 6 +10 | 0000000 | See HPC pre-mixes | A=3 |
| RD11076 | Base of RD11075 | --------- | ---------- | ----------- | ----------- | ----------- | Addition RD11067 | ----------- |
| RD11077 | Base of RD11075 | --------- | ---------- | ----------- | ----------- | ----------- | Addition RD11068 | ----------- |
| RD11078 | Base of RD11075 | --------- | ---------- | ----------- | ----------- | ----------- | Addition RD11069 | ----------- |
| RD11079 | Transfer from RD11076 | Add 0.3% H2O | ---------- | ----------- | ----------- | ----------- | Formula includes HPC | ----------- |
| RD11080 | Transfer from RD11077 | Add 0.3% H2O | ---------- | ----------- | ----------- | ----------- | Formula includes HPC | ----------- |
| RD11081 | Transfer from RD11078 | Add 0.3% H2O | ---------- | ----------- | ----------- | ----------- | Formula includes HPC | ----------- |
| RD11082 | 4 | 81.0 | 000000 | 0000000 | 10 See RD11073 (3 | See RD11073 (3 | See RD11073 (0.25) | 00000000 |

(continued)

| Trial number | Active % | Castor oil % | Labrafil % | PVP grade % | DMI % | TranscutolP % | HPC Nisso % | SiO2 % C=Cabosil A=Aerosil200 |
|---|---|---|---|---|---|---|---|---|
| RD11085 | 4 | 70.7 | 000000 | 0000000 | 16 10+6 | 6 | N-L = 0.2 N-M =0.3 | A= 2.8 |
| RD11086 | 4 Add 0.3% H2O | 70.7 | 000000 | 0000000 | 16 10+6 | 6 | N-L = 0.2 N-M =0.3 | A= 2.8 |

**Lot # RD11037**

Process duplication of IMP batch (4%) without HPC. K17 and S630 dissolved in DMI/Transcutol mixture followed by addition of the active. Clear solution. Castor oil preheated and added the above active mixture. Clear solution observed. Followed with the addition of the Cabosil with low shear. Viscosity at time of manufacture 500 cps, followed with test after 48 hours resulted in 620 cps.
Lower viscosity primarily due to missing HPC (note that IMP 4% had approx 1,500 cps)

**Lot # RD11038**

Change in order of addition using the same formulation with a reduction of DMI/Transcutol and adjusted with castor oil. Cabosil was mixed into the Castor oil obtaining a clear viscous solution. The active mixture was prepared as per RD11037. Viscosity of the Castor oil/Cabosil mixture changed to 1180 cps (expected higher viscosity based on addition of Co Solvents during the Placebo trials). Potential impact of PVP and active to solvent mixture.

**Lot # RD11039**

Duplicated performance based on Placebo mixture also containing Labrafil in castor oil plus Cabosil (for IP). Same reaction of reduced viscosity when adding the active mixture.

**Lot# RD11040**

Duplicated Placebo process adding to the Castor oil/Cabosil mixture a portion of the DMI/Transcutol P co-solvent mixture. Viscosity of the oil mixture increased. Prepared the active mixture with the remaining co-solvents without the PVP and added to the oil mixture. Final viscosity of the bulk Gel was 10,400 cps. Potential for F/C .

**Lot# RD11041**

Process was repeated as per RD11040 including the PVP K17 and S630 with the active mixture and viscosity was reduced to 500 cps (increased to 1,500 cps after 3 weeks). Clear indication of PVP impact on lowering viscosity using K17 and S630.

**Lot# RD11042**

Repeat of trial with Castor oil/ Labrafil addition as per RD11037, and reduced Cabosil, with active co solvent mixture but no PVP. Viscosity of 1,750 cps
The following trials were designed to identify impact of changing to higher PVP grades as well as alternate source of HPC (2 grades). Pre mixture were made as outlined in table 3 concentrating on mixtures without Labrafil, using Castor oil native and Aerosil 200.

**Lot # RD11050**

Dispersion (pre-mix I) of Castor Oil and Aerosil 200 was prepared and viscosity increased by adding part of the DMI (4%). The preparation of the active mixture use the pre-mix of RD11047A (PVP K17-3%) in DMI only, added 0.3% of HPC Nisso H followed by addition of active. Active mixture was added to the Pre-mix I

**Lot # RD11050A**

Same basic formulation as RD11050 with change of adding to a portion additional 1% of Aerosil 200

**Lot # RD11051**

Dispersion (pre-mix I) of Castor Oil and Aerosil 200 was prepared and viscosity increased by adding part of the DMI (4%). The preparation of the active mixture use the pre-mix of RD11047B (PVP K30-3%) in DMI only, added 0.3% of HPC Nisso M followed by addition of active. Active mixture was added to the Pre-mix I

**Lot # RD11051A**

Same basic formulation as RD11051 with change of adding to a portion additional 1% of Aerosil 200

**Lot # RD11053**

Dispersion (pre-mix I) of Castor Oil and Aerosil 200 was prepared and viscosity increased by adding part of the DMI and Transcutol P. The preparation of the active mixture use the pre-mix of RD11048A (PVP K17-3%), added 0.3% of HPC Nisso H followed by addition of active. Active mixture was added to the Pre-mix I

**Lot # RD11054**

Dispersion (pre-mix I) of Castor Oil and Aerosil 200 was prepared and viscosity increased by adding part of the DMI and Transcutol P. The preparation of the active mixture use the pre-mix of RD11048B (PVP K30-3%), added 0.3% of HPC Nisso H followed by addition of active. Active mixture was added to the Pre-mix I

**Lot # RD11055**

Dispersion (pre-mix I) of Castor Oil and Aerosil 200 was prepared and viscosity increased by adding part of the DMI and Transcutol P. The preparation of the active mixture use the pre-mix of RD11048C (PVP K90-3%). No HPC added .Active mixture was added to the Pre-mix I

**Lot # RD11056**

Dispersion (pre-mix I) of Castor Oil and Aerosil 200 was prepared and viscosity increased by adding part of the DMI. The preparation of the active mixture use the pre-mix of RD11047C (PVP K90-3%). No HPC added Active mixture was added to the Pre-mix I

**Lot # RD11059**

Prepared mixture of Castor Oil and Cabosil (2.5%). Active was dissolved in DMI and Transcutol P. Resulted in milky appearance. Adding that mix to the Castor Oil pre-mix, mixture did not clear up. Prepared the PVP (K30) solution with DMI, added to the mix, no change in appearance however reduced viscosity.
Note, no change in evaluation adding a mixture of 0.1% HPC to appearance, slight increase in viscosity. Trial not reported under trial a lot number.

**Lot # RD11060**

Prepared the Castor Oil adding 3.5% Cabosil, followed by addition of a mixture of DMI/ Transcutol P for thickening. The active dispersion was prepared in a PVP (K30) with DMI as co-solvent. (no HPC)

**Lot # RD11061**

Prepared the Castor Oil adding 3% Cabosil, followed by addition of Labrafil (2%) for thickening. The active dispersion was prepared in a DMI mixture containing PVP K17 (2%). Mix resulted in low viscosity, however could be considered for F/C test.

**Lot # RD11062**

Castor Oil native mixed with Aerosil 200 (3%) and added a mixture of DMI/Transcutol P (6+2) for thickening. A PVP mixture of K17 and K30 was dissolved in DMI/Transcutol P and followed with HPC H and solvate for 4 days. Mixture was reheated prior to addition of active. Castor Oil premix was heated prior to adding the active dispersion. Recommended for F/C

**Lot # RD11063**

Castor Oil native mixed with Aerosil 200 (4%) and added the DMI (6%) resulting in a high viscose mix. A mixture of PVP K17 and L29/32 was dissolved in DMI, plus HPC Nisso H (0.2). On overnight setup, noticed a separation, required

re-mixing. Active was added to the high viscosity Castor Oil premix. To be followed up with modification to composition Potential for F/C or to use RD11065

**Lot # RD11064**

Addition of 0.3% to portion of lot RD11062

**Lot # RD11065**

Addition of 0.3% to portion of lot RD11063

**Lot # RD11066**

Addition of 0.3% to portion of lot RD11041

**Lot # RD11070**

Addition of 0.3% to portion of lot RD11037

**Lot# RD11071**

Addition of 0.3% to portion of lot RD11042

**Lot# RD11072**

Addition of 0.3% to portion of lot RD11040

**Lot # RD11073**

Prepared Castor Oil / Aerosil 200 pre-mixture. Dissolve in DMI (6%) without PVP, the Testosterone and add to the Castor oil pre-mix. Obtained a viscosity of 6,300 cps. In a mixture of Transcutol P and DMI disperse the HPC M (only used 0.25% of prep) and add to main mix. Proposed for F/C

**Lot# RD11074**

Addition of 0.3% to portion of lot RD11072

**Lot # RD11075**

Prepared a stock mixture to complete 3x500 g trials consisting of Castor-Oil (68%) Aerosil 200 (3%) DMI (6%). To this mix was added PVP K29-32 (1%) in DMI (10) and active. Bulk split into 3 parts to be completed for 3 trials containing different mixtures and grades of HPC Nisso in Transcutol (ref lots RD11067/68/69)

**Lot # RD11076**

Used bulk from RD11075 and added HPC mix RD11067 (Transcutol P with Nisso H (0.15%)

**Lot# RD11077**

Used bulk from RD11075 and added HPC mix RD11068 (Transcutol P with Nisso H (0.2%)

**Lot # RD11078**

Used bulk from RD11075 and added HPC mix RD11069 (Transcutol P with Nisso H (0.1) and M (0.1)

**Lot # RD11079**

Addition of 0.3% to portion of lot RD11076

**Lot #RD11080**

Addition of 0.3% to portion of lot RD11077

**Lot #RD11081**

Addition of 0.3% to portion of lot RD11078

**Lot #RD11082**

Trial attempt to prepare a batch without the use of SiO2 failed

**Lot #RD11085**

Prepared Castor-Oil pre-mix adding 2.5% Aerosil 200 followed with a mix of DMI (10) and Testosterone. Obtained viscosity of 3,100 cps. Followed with the addition of HPC Nisso L (0.2%) and Nisso M (0.3%) mixed in DMI and Transcutol plus 0.3% Aerosil 200 to reduce stickiness. Material was added without any stringing to the main mixture and obtained a viscosity of 4,800 cps at day of manufacture and 4,900 cps 3 weeks later. Proposed for F/C

**Lot #RD11086**

Addition of 0.3% to portion of lot RD11085

**Table 2 TBS1A 4% strength**

| Viscosity values using spindle 6, 20 rpm, Repeat test ref to Franz Cell: F/C | | | |
|---|---|---|---|
| **Lot number** | **Trial Manuf date** | **Test date and values** | **Comments** |
| RD11037 | Jul 15/11 | Oct 04/11 940 cps | Clear solution, previous results in July 620 cps and follow up test 9/15/11 was 900 cps |
| RD11038 | Jul 15/11 | Oct 04/11 1,800 cps | Clear solution, original test 1,180 cps, follow up 09/15/11 1,660 cps |
| RD11039 | Jul 20/11 | Oct 04/11 1,380 cps | Clear solution, previous results in July 980cps and follow up test 9/15/11 was 1,300 cps |
| RD11040 | Jul 20/11 | Oct 04/11 11,040 cps | Clear Gel, previous results in July 10,400 cps and follow up test 9/15/11 was 10,140 cps |
| RD11041 | Jul 21/11 | Oct 04/11 1,420 cps | Clear solution, previous results in July 500 cps and follow up test 9/15/11 was 1,500 cps |
| RD11042 | Jul 21/11 | Oct 04/11 1,430 cps | Clear solution, test 9/15/11 was 1,720 cps |
| RD11050 | Aug.09/11 | Oct 04/11 Test not valid | Original comment sticky mixture, 09/15/11 results 2,460 Do not use trial lot for F/C Poor mixture, HPC settled to bottom as a slug |
| RD11050A | Aug.09/11 | Oct 04/11 Test not valid | Original comment sticky mixture, results 09/15/11 3,000 cps (increased during test from 2,400) Do not use trial lot for F/C Poor mixture, HPC settled to bottom as a slug |
| RD11051 | Aug.09/11 | Oct 04/11 2,100 cps▲ | Clear, results 09/15/11 1,940 cps Note: viscosity values increase during 30 sec test |
| RD11051A | Aug.09/11 | Oct 04/11 2,540 cps▲ | Clear, results 09/15/11 2,560 cps Note: viscosity values increase during 30 sec test |
| RD11053 | Aug.10/11 | Oct 04/11 4,500 cps▲ | Clear but sticky with air bubbles, results 09/15/11 4,060 cps Note: viscosity values increase during 30 sec test |

(continued)

| Lot number | Trial Manuf date | Test date and values | Comments |
|---|---|---|---|
| RD11054 | Aug.10/11 | Oct 04/11 14,000 cps▲ | 09/15/11 test HPC globules, 15,000 cps Do not use trial lot for F/C, Note: viscosity values increase during 30 sec test Build up of HPC on spindle |
| RD11055 | Aug.10/11 | Oct 04/11 EEEEEE | 09/15/11, EEEEEE Do not use trial lot for F/C Note, error message indicates above 20,000 tester limit at that setting |
| RD11056 | Aug.10/11 | Oct 04/11 EEEEEE | 09/15/11, EEEEEE Do not use trial lot for F/C<br>Note, error message indicates above 20,000 tester limit at that setting |
| RD11059 | Aug.22/11 | Oct 04/11 Test not valid | Do not use trial lot for F/C Separation of HPC (?)Build up of HPC on spindle |
| RD11060 | Aug.23/11 | Oct 05/11 3,540 cps | Uniform texture |
| RD11061 | Aug.23/11 | Oct 05/11 960 cps | Uniform texture |
| RD11062 | Aug.24/11 | Oct 05/11 3,200 cps | Original viscosity 2,400 cps |
| RD11063 | Aug.24/11 | Oct 05/11 3,460 cps | Original viscosity 1,600 cps |
| RD11064 | Aug.31/11 | Oct 05/11 6,440 cps | Original viscosity 5,800 cps Clear, thick, |
| RD11065 | Aug.31/11 | Oct 05/11 12,500 cps | Added .3% H2O to RD11063 09/31/11 resulted in 9,100 cps Air bubbles |
| RD11066 | Aug.31/11 | Oct 05/11 2,600 cps | Added .3% H2O to RD11041 09/31/11 resulted in 1,500 cps Clear, thick |
| RD11070 | Aug.31/11 | Oct 05/11 1,540 cps | Added .3% H2O to RD110370 9/31/11 resulted in 720 cps Liquid and clear |
| RD11071 | Aug.31/11 | Oct 05/11 1,820 cps | Added .3% H2O to RD11042 9/31/11 resulted in 1,760 cps Liquid and clear |
| RD11072 | Aug.31/11 | Oct 05/11 7,920 cps | Added .3% H2O to RD11040 resulted in 7,920 cps Clear and thick, no change in viscosity |
| RD11073 | Sep.07/11 | Oct 05/11 9,980 cps | Started off in Sept with viscosity of 5,500 cps |
| RD11074 | Sep.07/11 | Oct 05/11 10,100 cps | Added .3% H2O to RD11073 increases viscosity to 7,200 cps. |
| RD11076 | Sep.06/11 | Oct 05/11 1,700 cps | Clear, however noticed separation in bulk |
| RD11077 | Sep.06/11 | Oct 05/11 1,600 cps | Clear |
| RD11078 | Sep.06/11 | Oct 05/11 2,700 cps | Clear and fluid |
| RD11079 | Sep.06/11 | Oct 05/11 3,500 cps | Added 0.3% H2O to RD11076 Clear, fluid |

(continued)

| Lot number | Trial Manuf date | Test date and values | Comments |
|---|---|---|---|
| RD11080 | Sep.06/11 | Oct 05/11 3,900 cps | Added 0.3% $H_2O$ to RD11077 Clear, fluid |
| RD11081 | Sep.06/11 | Oct 05/11 2,600 cps | Added 0.3% $H_2O$ to RD11078 Clear, fluid |
| RD11085 | Sep.14/11 | Oct 05/11 4,900 cps | Original test 4,800 cps Thick and clear |
| RD11086 | Sep.20/11 | Oct 05/11 5,180 cps | Addition of 0.3% $H_2O$ to RD11085 = 5,200 cps original Thick gel and clear |

**TBS1A 8 % Formulation/composition**

**Table 3**

| ***Trial number*** | ***Active micronized %*** | ***Castor oil %*** | ***Labrafil %*** | ***PVP grade %*** | ***DMI %*** | ***TranscutolP %*** | ***HPC Nisso %*** | ***SiO2 % C=Cabosil A=Aerosil200*** |
|---|---|---|---|---|---|---|---|---|
| | | | | | | | | |
| RD11087 | 8 | 55.9 | 0000000 | 0000000 | 27 20 + 7 | 6 | N-L = 0.2<br>N-M = 0.3 | A = 2.6 |
| RD11088 | 8 | same | 0000000 | 0000000 | same | same | same | Same plus (0.3% H2O) |
| RD11089 | 8 | 46.5 | 0000000 | K17 = 3<br>S630 = 2 | 25 | 10 | N- M = 0.5 | C = 5 |
| RD11089A | 8 | same | 0000000 | same | same | same | same | Same plus (0.3% H2O) |
| RD11090 | 8 | 39.0 | 0000000 | K17 = 5.0 | 32 | 12 | N-H = 0.3<br>N-M = 0.2 | C = 3.5 |
| RD11100 | 8 | same | 0000000 | same | same | same | same | Added C = 2% for total of 5.5 |
| RD11101 | 8 | 46.1 | 0000000 | K17 = 5.0 | 25 | 10 | N-L = 0.4<br>N-M = 0.4 | C = 5.1 |
| RD11102 | 8 | 46.1 | 0000000 | K17 = 5.0 | 25 | 10 | N-L = 0.4<br>N-M = 0.4 | C = 5.1 plus Addition of 1% for total of 6.1 |
| RD11103 | 8 | 46.1 | 0000000 | K17 = 5.0 | 25 | 10 | N-L = 0.4<br>N-M = 0.4 | C = 5.1 plus addition of 0.3% water |
| RD11104 | 8 | 42.2 | 4.0 | K17 = 5.0 | 25 | 10 | N-L = 0.4<br>N-M = 0.4 | A = 5.0 |
| RD11105 | 8 | same | same | same | same | same | same | A = 5.0 addition of 0.5% total 5.5% |

**Process outline for active trials:**

**Lot # RD11087**

Trial was initiated without PVP to identify impact on T solubility. The active dispersion in % DMI used did not provide a clear solution and did not clear up when adding to the Castor Oil/SiO2 mix. Even the co-solvents present in the HPC mixture did not provide a clear bulk Gel. To the HPV mixture 0.1% SiO2 was added to reduce stringing and stickiness.

Viscosity at 4,400

This trial however will be selected for the Franz Cell test to identify diffusion rate eliminating PVP.

**Lot # RD11088**

0.3% water was added to a portion of Lot RD11087 to identify impact on viscosity. As observed on 4% trials, increase in viscosity is not evident on the bulk mixed with SiO2 in the HPC. This trial not considered for F/C.

**Lot # RD11089**

This trial used the same quantitative formulation as the IMP Clinical 8%, however using an alternate source of HPC (original HPC source Klucel HF). Also made minor process changes, dissolved PVP in DMI only and added active. HPC was prepared in Transcutol and added to main bulk separately.

Obtained a clear solution when adding the active co-solvent mixture into the Castor-oil and no significant stringing with the addition of the HPC after addition of SiO2.

Viscosity of Gel on day of manufacture was 1,800 cps, when retested after 24 hours, 3,700 and after 48 hours up to 4,300. The re-test on October 3 (see table) recorded 4,500 cps.

This trial was selected for F/C test

**Lot#RD11089A**

0.3% water was added to a portion of Lot RD11089 to identify impact on viscosity.

Viscosity change over time similar to above trial, day of manufacture 2,700 cps, when retested after 24 hours, 3,920 and after 48 hours up to 4,600. The re-test on October 3 (see table) recorded 5,040 cps.

Selected for study on impact of water

**Lot # RD11090**

Used higher percentage of DMI and Transcutol to be split for various pre-mixes, similar with SiO2 to be added HPC. Made a pre-mix of Castor oil and SiO2, however due to the lower ratio between the 2 excipients, the mixture became quite thick and further thickened up when adding part of the DMI.

Did finish off the trial, ended up at low viscosity, day of manufacture 900 cps, test Oct 03 -1,260 cps. Lower level of SiO2 was considered for study impact, however considering the processing issue (see RD11100)

not suitable for F/C test

**Lot# RD11100**

Using a portion of above trial RD11090, added an additional 2% SiO2 (for total of 5.5%) to study impact on Viscosity. Increased to 1,900 cps on day of manufacture and retest October 03 (see table) resulted in a value of 3.060

**Lot # RD11101**

To potentially reduce the impact of PVP, required to dissolve the active, during the addition to the Castor oil/SiO2 mixture, added 2% of SiO2 to the DMI-PVP-Testosterone mix, obtaining a viscous mix. After addition of that mixture to a dispersion of Castor oil containing 1% SiO2, maintained a viscous mixture at the temperature of 50% (would thicken up further on cooling). Further increase in viscosity with the addition of the HPC mix and final amount of SiO2.

Viscosity after cooling Gel to 21 C was 3,800 cps. (note that re-testing over time will be required, batch manufactured Oct 03)

This trial selected for F/C

**Lot#RD11102**

With the target for a 5,000 cps viscosity for the TBS1A project, the above RD11101 was so far the best candidate to evaluate impact of further addition of SiO2, hence to a portion of that lot additional 1% SiO2 was added. The rational for 6% was to obtain the same ratio of active to SiO2 as the targeted level of 3% SiO2 for the 4% strength.

Viscosity increase to 8,000 cps , this lot was selected for F/C study to identify impact of viscosity on rate of diffusion compared to RD11101 of same composition with exception of 1% addition in SiO2, may need to consider on assay obtained.

**Lot#RD11103**

Addition of water for impact on viscosity, not considered for follow up testing (see viscosity table for results, increase to RD11101 from 3,800 to 4,500 cps)

**Lot#RD11104**

Included this trial to evaluate addition of Labrafil. Labrafil was added to the Castor Oil mixed with SiO2 at 1%. As observed previously, addition of Labrafil to the Castor oil containing SiO2 increases viscosity. All other mixture prepared and added as per trial RD11101, with addition of 2% SiO2 to complete mixture. This mixture contains a larger percentage of air bubbles, common on formulations containing Labrafil. Viscosity obtained of 3,300 cps, will be followed up and tested at various time points.

Selected for F/C testing.

**Lot#RD11105**

Added to RD11104 an additional 0.5% SiO2 (% adjusted to avoid high increase observed on RD11102)
Increase from 3,300 to 4,100 cps
Not selected for F/C test
Note: Placebo trials are drawn up to identify impact on viscosity using the 2 different sources for Castor Oil and SiO2. These trials will also answer potential questions related to TBS1 and TBS2.

**TABLE 4 TBS1A 8 % strength**

| Viscosity values using spindle #6, 20 rpm, | | | Franz Cell = F/C |
|---|---|---|---|
| **Lot number** | **Trial Manuf date** | **Test date and values** | **Comments** |
| RD11087 | Sept 20/11 | Oct 03/11 4,400 cps | No PVP, solution not clear, 2.6% SiO2 Selected for Franz Cell |
| RD11088 | Sept 20/11 | Oct 03/11 4,040 cps | Added 0.3% H2O to RD11087 |
| RD11089 | Sept 25/11 | Oct 03/11 4,500 cps | Based on original IMP, change in HPC source and minor process step changes Selected for Franz Cell |
| RD11089A | Sept 25/11 | Oct 03/11 5,040 cps | As RD11089 plus 0.3% H2O Selected for Franz Cell |
| RD11090 | Sept 26/11 | Oct 03/11 1,260 cps | 3.5% SiO2 Potential for F/C |
| RD11091 | Sept 26/11 | Oct 03/11 | Added 0.3% H2O to RD11090 |
| RD11100 | Sept 26/11 | Oct 03/11 3,060 cps | Added to RD11090 to reach 5% SiO2 content |
| RD11101 | Oct 03/11 | Oct 04/11 3,800 cps | 5% SiO2 Selected for Franz Cell |
| RD11102 | Oct 04/11 | Oct 04/11 | 6% SiO2 |

(continued)

| Viscosity values using spindle #6, 20 rpm, | | | Franz Cell = F/C |
|---|---|---|---|
| **Lot number** | **Trial Manuf date** | **Test date and values** | **Comments** |
| | | 8,000 cps | Selected for Franz Cell |
| RD11103 | Oct 04/11 | Oct 04/11 4,500 cps | 0.3% with 5% SiO2 |
| RD11104 | Oct 04/11 | Oct 05/11 3,300 cps | Includes 4% Labrafil, same comp for polymers as RD11101 (air-bubbles) Selected or Franz Cell |
| RD11105 | Oct 05/11 | Oct 05/11 4,100 cps | Added additional 0.5% of SiO2 to RD11104 |

**Pre-mix RD Trials (used for addition in active trials)**

**Table 5**

| Trial #/ observation test | Evaluation | Composition | Results/comments | Used in RD trial # |
|---|---|---|---|---|
| EV001A (pg 41) | Dissolving HPC Nisso grade **M** | DMI - 100 g Transcutol P 50 g Nisso HPC **M** - 2.5 g | Low viscosity grade Stored for hydration 72 hrs Suitable viscosity for further additions | Not transferred for use to RD trials |
| EV001B (pg 41) | Dissolving HPC Nisso grade **H** | DMI - 100 g Transcutol P 50 g Nisso HPC **H -** 2.5 g | high viscosity grade Stored for hydration 72 hrs Viscosity too high | Not transferred for use to RD trials |
| EV002A (pg 41) | Dispersing Cabosil in DMI (purpose to study impact on viscosity in final Gel) | DMI - 125 g Cabosil 10 g Ratio related to Castor oil/ Cabosil | Obtained clear and viscous dispersion | Not transferred for use to RD trials |
| EV002B (pg 41) | Dispersing Cabosil in Transcutol P (purpose to study impact on viscosity in final Gel) | Transcutol P 250 g Cabosil 20 g Ratio related to Castor oil/ Cabosil | Obtained no increase viscosity. Solution milky in appearance | Not transferred for use to RD trials |
| RD11047 A | Addition of PVP K17 in DMI only. | DMI- 100 g PVP K17 15 g Ratio represents 3% of PVP based on final Bulk Gel formula | Suitable for additional mixing with HPC H and active. Note: used higher viscosity HPC grade with lower | Used in RD trial for addition of HPC-H and active (see RD1150 and RD1150A) |
| | | | viscosity PVP grade | |

(continued)

| Trial #/ observation test | Evaluation | Composition | Results/comments | Used in RD trial # |
|---|---|---|---|---|
| RD11047B | Addition of PVP K29/32 in DMI only. | DMI- 100 g PVP K29/32 15 g Ratio represents 3% of PVP based on final Bulk Gel formula | Suitable for additional mixing with HPC M and active. Note: used lower viscosity HPC grade with higher viscosity PVP grade | Used in RD trial for addition of HPC-M and active (see RD1151 and RD1151A) |
| RD11047C | Addition of PVP K90 in DMI only. | DMI- 100 g PVP K90 15 g Ratio represents 3% of PVP based on final Bulk Gel formula | Not suitable to add any grade HPC, however suitable to add the active portion. | Used in RD trial without HPC addition RD11056 |
| RD11048 A | Addition of PVP K17 in DMI and Transcutol P | DMI- 80 g Transcutol P 20 g PVP K17 15 g Ratio represents 3% of PVP based on final Bulk Gel formula | Suitable for additional mixing with HPC H and active. Note: used higher viscosity HPC grade with lower viscosity PVP grade | Used in RD trial for addition of HPC-H and active (see RD11053 |
| RD11048B | Addition of PVP K29/32 in DMI and Transcutol P. | DMI- 80 g Transcutol P 20 g PVP K29/32 15 g Ratio represents 3% of PVP based on final Bulk Gel formula | Suitable for additional mixing with HPC M and active. Note: used lower viscosity HPC grade with higher viscosity PVP grade | Used in RD trial for addition of HPC-M and active (see RD11054 |
| RD11048C | Addition of PVP K90 in DMI and Transcutol P | DMI- 100 g PVP K90 15 g Ratio represents 3% of PVP based on final Bulk Gel formula | Not suitable to add any grade HPC, however suitable to add the active portion. | Used in RD trial without HPC addition RD11055 |
| RD11067 | Prep of HPC in Transcutol P only | TP= 40g N-H = 0.75g | | Used in RD11076 |
| RD11068 | Prep of HPC in Transcutol P only | TP= 40g N-H = 1.0 g | | Used in RD11077 |

(continued)

| Trial #/ observation test | Evaluation | Composition | Results/comments | Used in RD trial # |
|---|---|---|---|---|
| RD11069 | Prep of HPC in Transcutol P only | TP= 40g<br>N-H = 0.5 g<br>N-M=0.5 g | | Used in RD11078 |
| RD11075 | Prep of base solution used RD11076/RD11077/ RD11078 Details in Table 2 | Castor oil / Aerosil200/ DMI/ PVP K30 Testosterone | | |

## Placebo TBS1A trials

**Table 6**

| Trial lot # | Evaluation | Composition | Results/comments |
|---|---|---|---|
| RD11032 | Evaluate change in viscosity using Labrafil versus Castor Oil Cr 0 | Labrafil M 1944 CS - 500g<br>Cab-O-Sil ---- 40 g | Viscosity 10,460 cps |
| RD11033 | Evaluate change viscosity adding Cabosil first in Castor Oil Cr 0 | Castor Oil ----- 500 g<br>Cab-O-Sil --- 40 g<br>Note: ratio used in IMP | Viscosity 14 460 cps |
| RD11034 | Impact on adding DMI and Transcutol to mixture RD11032 | RD11032-270 g<br>DMI- 125 g<br>Transcutol P 50 g | Viscosity reduced to 8,740 |
| RD11035 | Impact on adding DMI and Transcutol to mixture RD11033 | Impact on adding DMI and Transcutol to mixture RD11032 | Viscosity reduced to 3,600 |
| RD11036A | Mixture of Castor Oil and Labrafil, adding Cabosil followed by DMI/Transcutol P | Castor oil ...... 125 g<br>Labrafil ........ 125 g<br>Cabosil ....... 20 g<br>DMI ..... 125 g<br>Transcutol P 50 g | High viscosity out of range |
| RD11036B | Mixture of Castor Oil and Labrafil followed by DMI/ Transcutol P, add Cabosil last | Castor oil 0 ...... 125 g<br>Labrafil ........ 125 g<br>Cabosil ....... 20 g<br>DMI ..... 125 g<br>Transcutol P 50 g | Viscosity 7,680 cps |
| RD11043 | Castor oil and CabOsil, followed by mixture of DMI/ Transcutol P and HPC **H** | Castor oil 0 ...... 285 g<br>Cabosil ....... 20 g<br>DMI .... 100 g<br>Transcutol P 50 g<br>HPC **H** ............... 2.5 g | |

(continued)

| Trial lot # | Evaluation | Composition | Results/comments |
|---|---|---|---|
| RD11043 | Castor oil and CabOsil, followed by mixture of DMI/ Transcutol P and HPC **M and PVP K17** | Castor oil 0 ...... 285 g<br>Cabosil ....... 20 g<br>DMI .... 100 g<br>Transcutol P 50 g<br>HPC **M** .............. 2.5 g<br>PVP K15 ....... 15 g | |
| RD11057P | TBS-2 Placebo for Analytical Lab Method | | |
| RD11058P A-B-C-D-E-F | Castor oil an Cabosil Mix followed by addition of Labrafil | A to D represents % Labrafil of 2-4% with change in viscosity E impact of adding Oleic acid F impact of adding DMI to RD11058-A | RD11058P = 2740 cps<br>Part A 2% = 11,400<br>Part B 3% = 14,000<br>Part C 3.5% = 14,440<br>Part D 4% = 14,900<br>Part E with Oleic = 1,520<br>Part F - 10% DMI to part A = 13,500 cps<br>(incr. from 11,400) |
| RD11083P | Purpose of trial to decrease stringing and stickiness of HPC mixture when adding to base mix of castor oil/Aerosil and | HPC mix prep of DMI/TranscutolP solvents plus Nisso HPC L and M Solvated for 48 hours followed by addition of | Viscosity of base prior to addition of HPC mixture was 5,300 cps, after addition of HPC mixture (no stringing |
| | DMI | SiO2 | |
| RD11084P | Used part of RD1108P to add 0.3% H2O to evaluate impact on viscosity | | |

## EXAMPLE 10

### Franz Cell Studies - Testosterone Rates of Diffusion

Generally speaking, soak the membrane for 30 minutes in the diffusion solution. After put the membrane on the Franz Cell. Put the ring and the donor chamber on the membrane and clamp it. Add approx. one gram of gel (TBS 1 A 4% or 8%). Check the level of diffusion solution in Franz Cells. It's supposed to be on the mark. Put "parafilm" on the sampling port to avoid evaporation. Withdraw 0.3mL of sample at 60, 120, 180, 240, 300 and 360 minutes using syringe. Add diffusion solution to make up to the mark of Franz Cells. Each sample should be collected in insert.

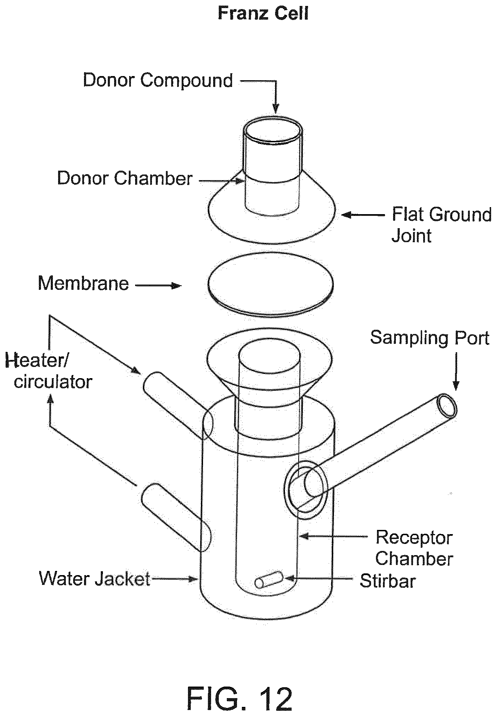

A typical Fanz cell used in accordance with this Example 9 and the invention is depicted in Fig. 12. The materials include:

Diffusion solution: Ethanol/Water 50:50
Membrane: Millipore 0.45μm.
Temperature: 37° ± 0.5 °C.
Stirring speed: 600 rpm.
Medium volume: 20mL.
Surface area: 1.7671 $cm^2$
Number of Franz Cells: 6.
Sampling time (minutes): 60, 120, 180, 240, 300 and 360.
Aliquot volume: 0.3mL.
Insert: 0.4mL.

The TBS1A formulations are as follows and as reported in the Examples above and herein. The rate of diffusion results of testosterone through the Franz cell mebrane, normalized for each gel concentrations being tested, measured as slope/mgT%, are reported below in the Franz Cell Table.

**4% TBS1A Trial formulations used in Franz Cell**

| **Trial Lot # RD11063** | | Batch size 500 g | |
|---|---|---|---|
| **Raw Materials/grade** | **%** | **Process** | **comments** |
| | | | 24 hr Franz Cell |
| Testosterone micronized | 4.0 | 12% DMI to disperse PVP and active | |
| Castor Oil **(V - O)** | 70.8 | 4% SiO2 in Castor oil plus 6% of DMI | Steps: |
| PVP **K17** | 1.5 | | A: add all SiO2 to Castor Oil |
| PVP **K30** | 1.5 | | Followed by DMI portion |
| PVP **K90** | 0.0 | | B: to the DMI add PVP, follow |
| Co PVP **S630** | 0.0 | | With HPC and hold 24 hrs |
| DMI | 18.0 | | C: add active |
| Transcutol P | 0.0 | | D: add to mix A) |
| HPC Nisso **L** | 0.0 | | |
| HPC Nisso **M** | 0.0 | | Temp range NMT 60C |
| HPC Nisso **H** | 0.2 | | Homogenize active mixture |
| SiO2 (Cabosil **-Aerosil 200)** | 4.0 | | Viscosity 3,650 cps 10/05/11) |

| **Trial Lot # RD11085** | | Batch size 500 g | |
|---|---|---|---|
| **Raw Materials/grade** | **%** | **Process** | **comments** |
| | | | 24 hrs Franz Cell |
| Testosterone micronized | 4.0 | 10% DMI used to dissolve active | |
| Castor Oil (V - **O**) | 70.7 | 2.5% of SiO2 mixed into Castor Oil | Steps: |
| PVP **K17** | 0.0 | ----------- | A: Active /DMI mixture added |
| PVP **K30** | 0.0 | ----------- | to Castor Oil/SiO2 mix |
| PVP **K90** | 0.0 | ----------- | B: add SiO2 to HPC after 24 h |
| Co PVP **S630** | 0.0 | ----------- | |
| DMI | 16.0 | 6% DMI used for HPC dispersion | C: add HPC mixture to main |
| Transcutol P | 6.0 | Used to disperse HPC and solvate for 24 hrs | bulk |
| HPC Nisso **L** | 0.2 | 0.3% of SiO2 mixed into HPC mixture | |
| HPC Nisso **M** | 0.3 | | Temp range NMT 60C |
| HPC Nisso **H** | 0.0 | | Homogenize active mixture |
| SiO2 (Cabosil **-Aerosil 200)** | 2.8 | | Viscosity 4,900 cps (10/05/11) |

| **Trial Lot # RD11038** | | Batch size 500 g | |
|---|---|---|---|
| **Raw Materials/grade** | **%** | **Process** | **comments** |
| | | | 6 hr Franz Cell |
| Testosterone micronized | 4.0 | Add to PVP mixture | |
| Castor Oil (V - **O**) | 57.0 | All Cabosil into Castor Oil | A: add to the Castor Oil /SiO2 |
| PVP **K17** | 3.0 | | Mix the PVP active mixture |
| PVP **K30** | 0.0 | | |
| PVP **K90** | 0.0 | | |
| Co PVP **S630** | 2.0 | | |
| DMI | 20.0 | All DMI and Transcutol P to disperse PVP | |
| Transcutol P | 10.0 | | |
| HPC Nisso **L** | 0.0 | | |
| HPC Nisso **M** | 0.0 | | Homogenize active mixture |
| HPC Nisso **H** | 0.0 | | |
| SiO2 **(Cabosil-Aerosil** 200) | 4.0 | | Viscosity 1,800 cps |

| ***Trial Lot # RD11039*** | | *Batch size 500 g* | |
|---|---|---|---|
| **Raw Materials/grade** | **%** | **Process** | **comments** |
| | | | 6 hr Franz Cell |
| Testosterone micronized | 4.0 | | |
| Castor Oil (V - **O**) | 29.0 | Mix Castor oil+Labrafil+Cabosil | |
| PVP **K17** | 3.0 | | |
| PVP **K30** | 0.0 | | |
| PVP **K90** | 0.0 | | |
| Co PVP **S630** | 2.0 | PVP into DMI+Tr-P followed by active | |
| DMI | 20.0 | | |
| Transcutol P | 10.0 | | |
| Labrafil | 29.0 | | |
| HPC Nisso **M** | 0.0 | | |
| HPC Nisso **H** | 0.0 | | |
| SiO2 **(Cabosil**-Aerosil 200) | 3.0 | | Viscosity 1,380 |

| **Trial Lot # RD11040** | | Batch size 500 g | |
|---|---|---|---|
| **Raw Materials/grade** | **%** | **Process** | **comments** |
| | | | 6 hr Franz Cell |
| Testosterone micronized | 4.0 | Mix in 12% DMI and 6% Tr-P | |
| Castor Oil (V - **O**) | 57.0 | Combine Castor oil + SiO2+ 13% DMI+4% TrP | |
| PVP **K17** | 0.0 | | |

(continued)

| **Trial Lot # RD11040** | | Batch size 500 g | |
|---|---|---|---|
| **Raw Materials/grade** | **%** | **Process** | **comments** |
| PVP **K30** | 0.0 | | |
| PVP **K90** | 0.0 | | |
| Co PVP **S630** | 0.0 | | |
| DMI | 25.0 | | |
| Transcutol P | 10.0 | | |
| HPC Nisso **L** | 0.0 | | |
| HPC Nisso **M** | 0.0 | | |
| HPC Nisso **H** | 0.0 | | |
| SiO2 (**Cabosil**-Aerosil 200) | 4.0 | | Viscosity 11,040 |

| ***Trial Lot # RD11042*** | | Batch size 500 g | |
|---|---|---|---|
| **Raw Materials/grade** | **%** | **Process** | **comments** |
| | | | 6 hr Franz Cell |
| Testosterone micronized | 4.0 | Active dissolve in 13% DMI+ 4% Tr-P | |
| Castor Oil (V - **O**) | 29.0 | Castor oil+Labrafil+SiO2+12 % DMI+6% Tr-P | |
| PVP **K17** | 0.0 | | |
| PVP **K30** | 0.0 | | |
| PVP **K90** | 0.0 | | |
| Co PVP **S630** | 0.0 | | |
| DMI | 25.0 | | |
| Transcutol P | 10.0 | | |
| Labrafil | 29.0 | | |
| HPC Nisso **M** | 0.0 | | |
| HPC Nisso **H** | 0.0 | | |
| SiO2 (Cabosil-Aerosil 200) | 3.0 | | Viscosity 1,430 cps |

| **Trial Lot # RD11051** | | Batch size 500 g | |
|---|---|---|---|
| **Raw Materials/grade** | **%** | **Process** | **comments** |
| | | | 6 hr Franz Cell |
| Testosterone micronized | 4.0 | 20% DMI+PVP+N-M +0.2% iO2 | |
| Castor Oil (V - O) | 66.7 | Castor Oil + SiO2 1.8 %+4% DMI | |
| PVP **K17** | 0.0 | | Easier addition of HPC adding |
| PVP **K30** | 3.0 | | Small % of SiO2 |
| PVP **K90** | 0.0 | | |
| Co PVP **S630** | 0.0 | | |
| DMI | 24.0 | | |

(continued)

| Trial Lot # RD11051 | | Batch size 500 g | |
|---|---|---|---|
| **Raw Materials/grade** | **%** | **Process** | **comments** |
| Transcutol P | 0.0 | | |
| HPC Nisso **L** | 0.0 | | |
| HPC Nisso **M** | 0.3 | | |
| HPC Nisso **H** | 0.0 | | |
| SiO2 (Cabosil-Aerosil 200) | 2.0 | | Viscosity 2,100 cps |

| Trial Lot # RD11055 | | Batch size 500 g | |
|---|---|---|---|
| | | | |
| **Raw Materials/grade** | **%** | **Process** | **comments** |
| | | | 6 hr Franz Cell |
| Testosterone micronized | 4.0 | DMI 16% +Transc 4% +pvp+active | |
| Castor Oil (V - **O**) | 62.0 | Castor Oil + SiO2 3 %+7% DMI+Trans 1% | |
| PVP **K17** | 0.0 | | |
| PVP **K30** | 0.0 | | |
| PVP **K90** | 3.0 | | |
| Co PVP **S630** | 0.0 | | |
| DMI | 23.0 | | |
| Transcutol P | 5.0 | | |
| HPC Nisso **L** | 0.0 | | |
| HPC Nisso **M** | 0.0 | | |
| HPC Nisso **H** | 0.0 | | |
| SiO2 (**Cabosil**-Aerosil 200) | 3.0 | | Exceeded test range |

| Trial Lot # RD11078 | | Batch size 500 g | |
|---|---|---|---|
| | | | |
| **Raw Materials/grade** | **%** | **Process** | **comments** |
| | | | 6 hr Franz Cell |
| Testosterone micronized | 4.0 | | |
| Castor Oil (V - **O**) | 68.0 | Castor oil +SiO2 -3% + 6% DMI | To be corrected to 67.8% |
| PVP **K17** | 0.0 | | for repeat (base) |
| PVP **K30** | 1.0 | DMI 10% + pvp + active | Base prep RD11075 |
| PVP **K90** | 0.0 | | |
| Co PVP **S630** | 0.0 | | |
| DMI | 16.0 | | |
| Transcutol P | 8.0 | Transc P + both HPC | Prep on RD11069 |
| HPC Nisso **L** | 0.0 | | |

(continued)

| Raw Materials/grade | % | Process | comments |
|---|---|---|---|
| HPC Nisso **M** | 0.1 | | Requires adjustment of |
| HPC Nisso **H** | 0.1 | | Castor oil by 0.2 % |
| SiO2 (Cabosil-**Aerosil 200)** | 3.0 | | Viscosity 2,700 cps |

| **Trial Lot # RD11054** | | Batch size 500 g | |
|---|---|---|---|
| | | | |
| **Raw Materials/grade** | **%** | **Process** | **comments** |
| | | | 6 hr Franz Cell |
| Testosterone micronized | 4.0 | | |
| Castor Oil (V - O) | 61.4 | Castor Oil +SiO2 3% + DMI 7% + Transc 1% | |
| PVP **K17** | 0.0 | | |
| PVP **K30** | 3.0 | DMI 16%+Trans 4%+pvp+HPC +active | |
| PVP **K90** | 0.0 | | |
| Co PVP **S630** | 0.0 | | |
| DMI | 23.0 | | |
| Transcutol P | 5.0 | | |
| HPC Nisso **L** | 0.0 | | |
| HPC Nisso **M** | 0.6 | | |
| HPC Nisso **H** | 0.0 | | |
| SiO2 (Cabosil-**Aerosil 200)** | 3.0 | | Viscosity 14,000 cps |

| **Trial Lot # RD11061** | | Batch size 500 g | |
|---|---|---|---|
| | | | |
| **Raw Materials/grade** | **%** | **Process** | **comments** |
| | | | 6 hr Franz Cell |
| Testosterone micronized | 4.0 | | |
| Castor Oil (V - O) | 71.0 | Castor oil + SiO2 + Labrafil | |
| PVP **K17** | 2.0 | DMI 16% + Transc 2% + PVP+ active | |
| PVP **K30** | 0.0 | | |
| PVP **K90** | 0.0 | | |
| Co PVP **S630** | 0.0 | | |
| DMI | 16.0 | | |
| Transcutol P | 2.0 | | |
| Labrafil | 2.0 | | |
| HPC Nisso **M** | 0.0 | | |
| HPC Nisso **H** | 0.0 | | |
| SiO2 (**Cabosil**-Aerosil 200) | 3.0 | | Viscosity 960 cps |

## Franz Cell Table – Slope/mgT%

| Lot nr/composition%/Franz | Testosterone | Cast or Oil | PVP K17 | PVP K30 | PVP K90 | CoPVP630 | Labrafil | DMI | Transcutol | HPC L | HPC M | HPC H | HPC XHF | SiO2 | water | Intercept | slope | Intercept/mgT% | Slope/mgT% | Povidone T | HPC Total | control |
|---|---|---|---|---|---|---|---|---|---|---|---|---|---|---|---|---|---|---|---|---|---|---|
| Reference | 4 | 88 | 0 | 0 | 0 | 0 | 4 | 0 | 0 | 0 | 0 | 0 | 0 | 4 | 0 | -241.78 | 132.62 | -60.45 | 33.16 | 0.00 | 0 | 100 |
| R viscous | 4 | 87.7 | 0 | 0 | 0 | 0 | 4 | 0 | 0 | 0 | 0 | 0 | 0 | 4 | 0.3 | -389.81 | 135.27 | -97.45 | 33.82 | 0.00 | 0 | 100 |
| TBS1a IMP11001 4% | 4 | 50.5 | 3 | 0 | 0 | 2 | 0 | 25 | 10 | 0 | 0 | 0 | 0.5 | 5 | 0 | -1371.8 | 300.02 | -342.95 | 75.01 | 5.00 | 0.5 | 100 |
| TBS1a IMP11002 4% | 4 | 65.5 | 3 | 0 | 0 | 2 | 0 | 15 | 5 | 0 | 0 | 0 | 0.5 | 5 | 0 | -991.01 | 220.68 | -247.75 | 55.17 | 5.00 | 0.5 | 100 |
| TBS1a IMP11003 8% | 8 | 46.5 | 3 | 0 | 0 | 2 | 0 | 25 | 10 | 0 | 0 | 0 | 0.5 | 5 | 0 | -2673.9 | 613.24 | -334.24 | 76.66 | 5.00 | 0.5 | 100 |
| RD11089 | 8 | 46.5 | 3 | 0 | 0 | 2 | 0 | 25 | 10 | 0 | 0.5 | 0 | 0 | 5 | 0 | -1999.2 | 489.46 | -249.90 | 61.18 | 5.00 | 0.5 | 100 |
| RD11089A | 8 | 46.5 | 3 | 0 | 0 | 2 | 0 | 25 | 10 | 0 | 0.5 | 0 | 0 | 5 | 0.3 | -1454.6 | 425.39 | -181.83 | 53.17 | 5.00 | 0.5 | 100.3 |
| RD11087 | 8 | 55.9 | 0 | 0 | 0 | 0 | 0 | 27 | 6 | 0.2 | 0.3 | 0 | 0 | 2.6 | 0 | -2810.2 | 636.05 | -351.28 | 79.51 | 0.00 | 0.5 | 100 |
| RD11101 | 8 | 46.1 | 5 | 0 | 0 | 0 | 0 | 25 | 10 | 0.4 | 0.4 | 0 | 0 | 5.1 | 0 | -2085.1 | 525.63 | -260.64 | 65.70 | 5.00 | 0.8 | 100 |
| RD11102 | 8 | 46.1 | 5 | 0 | 0 | 0 | 0 | 25 | 10 | 0.4 | 0.4 | 0 | 0 | 6.1 | 0 | -2069.9 | 499.5 | -258.74 | 62.44 | 5.00 | 0.8 | 101 |
| RD11104 | 8 | 42.2 | 5 | 0 | 0 | 0 | 4 | 25 | 10 | 0.4 | 0.4 | 0 | 0 | 5 | 0 | -3397.2 | 755.62 | -424.65 | 94.45 | 5.00 | 0.8 | 100 |
| RD11038 | 4 | 57 | 3 | 0 | 0 | 2 | 0 | 20 | 10 | 0 | 0 | 0 | 0 | 4 | 0 | -1265.3 | 271.06 | -316.33 | 67.77 | 5.00 | 0 | 100 |
| RD11039 | 4 | 29 | 3 | 0 | 0 | 2 | 29 | 20 | 10 | 0 | 0 | 0 | 0 | 3 | 0 | -3084.4 | 508.88 | -771.10 | 127.22 | 5.00 | 0 | 100 |
| RD11040 | 4 | 57 | 0 | 0 | 0 | 0 | 0 | 25 | 10 | 0 | 0 | 0 | 0 | 4 | 0 | -312.27 | 389.27 | -78.07 | 97.32 | 0.00 | 0 | 100 |
| RD11042 | 4 | 29 | 0 | 0 | 0 | 0 | 29 | 25 | 10 | 0 | 0 | 0 | 0 | 3 | 0 | -1687.5 | 366.34 | -421.88 | 91.59 | 0.00 | 0 | 100 |
| RD11051 | 4 | 66.7 | 0 | 3 | 0 | 0 | 0 | 24 | 0 | 0 | 0.3 | 0 | 0 | 2 | 0 | -1614.1 | 313.35 | -403.53 | 78.34 | 3.00 | 0.3 | 100 |
| RD11053 | 4 | 61.7 | 3 | 0 | 0 | 0 | 0 | 22 | 6 | 0 | 0 | 0.3 | 0 | 3 | 0 | -1187.7 | 261.82 | -296.93 | 65.46 | 3.00 | 0.3 | 100 |
| RD11054 | 4 | 61.4 | 0 | 3 | 0 | 0 | 0 | 23 | 5 | 0 | 0.6 | 0 | 0 | 3 | 0 | -1214.3 | 244.7 | -303.58 | 61.18 | 3.00 | 0.6 | 100 |

| | | | | | | | | | | | | | | | | | | | | | |
|---|---|---|---|---|---|---|---|---|---|---|---|---|---|---|---|---|---|---|---|---|---|
| RD11055 | 4 | 62 | 0 | 0 | 3 | 0 | 0 | 23 | 5 | 0 | 0 | 0 | 0 | 3 | 0 | -1428.1 | 307.28 | -357.03 | 76.82 | 3.00 | 0 | 100 |
| RD11061 | 4 | 71 | 2 | 0 | 0 | 0 | 2 | 16 | 2 | 0 | 0 | 0 | 0 | 3 | 0 | -2327.6 | 397.43 | -581.90 | 99.36 | 2.00 | 0 | 100 |
| RD11063 | 4 | 70.8 | 1.5 | 1.5 | 0 | 0 | 0 | 18 | 0 | 0 | 0 | 0.2 | 0 | 4 | 0 | -924.39 | 187.46 | -231.10 | 46.87 | 3.00 | 0.2 | 100 |
| RD11078 | 4 | 68 | 0 | 1 | 0 | 0 | 0 | 16 | 8 | 0 | 0.1 | 0.1 | 0 | 3 | 0 | -1309.9 | 269.37 | -327.48 | 67.34 | 1.00 | 0.2 | 100.2 |
| RD11085 | 4 | 70.7 | 0 | 0 | 0 | 0 | 0 | 16 | 6 | 0.2 | 0.3 | 0 | 0 | 2.8 | 0 | -1550 | 272.4 | -387.50 | 68.10 | 0.00 | 0.5 | 100 |

The TBS-1A Gel In Vitro Release Rate Validation concerning Release Rate Study Summary for TBS-1A Gel 4.0% and TBS-1A Gel 4.5% are presented in Exhibits A and B submitted herewith.

These summaries summarize the release rate experiment data for exemplary TBS-1A Gels. There are four Nasobol Gels (0.15%, 0.6%, 4.0% and 4.5%) for the method validation. The purpose of the Day1 and Day2 test are to determine the specificity and intraday/interday precision of the slope(release rate), Day3 and Day4 are to evaluate the slope sensitivity to the sample strength variation.

**Reference List**

1. Tietz Textbook of Clinical Chemistry and Molecular Diagnostics, 4th edition, 2006. Editors; Burtis CA, Ashwood ER, and Bruns DE.

2. Wang C, Swerdloff RS. Androgen replacement therapy. Ann Med 1997; 29: 365-370.

3. Matsumoto AM. Andropause: clinical implications of the decline in serum Testosterone levels with aging in men. J Gerontol A Med Sci 2002; 57: M76-M99.

4. Haren MT, Kim MJ, Tariq SH, Wittert GA, Morley JE. Andropause: a quality-of-life issue in older males. Med Clin North Am 2006; 90: 1005-1023.

5. Nieschlag E. Testosterone treatment comes of age: new options for hypogonadal men. Clin Endocrinol (Oxf) 2006: 65: 275-281.

6. Tenover JL. The androgen-deficient aging male: current treatment options. Rev Urol 2003; 5 (Suppl): S22-S28.

7. Jockenhovel F. Testosterone therapy - what, when and to whom? Aging Male 2004; 7: 319-324.

8. Kunz GH, Klein KO, Clemons RD, Gottschalk ME, Jones KL. Virilization of young children after topical androgen use by their parents. Pediatrics 2004; 114: 282-284.

9. Brachet C, Vermeulen J, Heinrichs C. Children’s virilisation and the use of a Testosterone gel by their fathers. Eur J Pediatr 2005; 164: 646-647.

10. Bagchus WM, Hust R, Maris F, Schnabel PG, Houwing NS. Important effect of food on the bioavailability of oral Testosterone undecanoate. Pharmacotherapy 2003; 23: 319-325.

11. Haren M, Chapman IM, Haren MT, MacKintosh S, Coates P, Morley JE. Oral Testosterone supplementation increases muscle and decreases fat mass in healthy elderly males with low normal gonadal status. J Gerontol A Biol Sci Med Sci 2003; 58: 618-625.

12. Haren M, Chapman I, Coates P, Morley JE, Wittert G. Effect of 12 month oral Testosterone on Testosterone deficiency symptoms in symptomatic elderly males with low-normal gonadal status. Age Ageing 2005; 34: 123-130.

13. Mattern C, Hoffmann C, Morley JE, Badiu C. The Aging Male 2008; 11: 171-178.


## Claims

**1.** A testosterone gel formulation for nasal administration, said testosterone gel formulation comprising:

a) about 4.5% testosterone by weight of said gel formulation; and
b) a pharmaceutically acceptable vehicle.

**2.** The testosterone gel formulation of claim 1, wherein the gel formulation comprises a solvent, a wetting agent, and a viscosity increasing agent.

3. The testosterone gel formulation of claim 2, wherein the solvent is castor oil.

4. The testosterone gel formulation of claim 2, wherein said wetting agent is an oleoyl polyoxylglyceride.

5. The testosterone gel formulation of claim 2, wherein said viscosity increasing agent is colloidal silicon dioxide.

6. The testosterone gel formulation of claim 1, wherein said gel formulation comprises castor oil, oleoyl polyoxylglycerides and colloidal silicon dioxide.

7. A testosterone gel formulation of any preceding claim for use in:

a) treating hypogonadism in a male subject by intranasal administration;
b) treating testosterone deficiency in a male subject by intranasal administration; or
c) providing testosterone replacement therapy in a male subject by intranasal administration.

8. A method of preparing a testosterone gel formulation for nasal administration, said testosterone gel formulation comprising about 4.5% testosterone by weight of said gel formulation; and a pharmaceutically acceptable vehicle, the method comprising:

a) combining micronized testosterone into contact with a solvent to form a first mixture;
b) combining oleoyl polyoxylglycerides with the first mixture to form a second mixture; and
c) combining colloidal silicon dioxide with the second mixture to provide a testosterone gel formulation for nasal administration.

9. A testosterone gel formulation of any of claims 1 - 6, or the testosterone gel formulation for use of claim 7, wherein the testosterone gel formulation has a testosterone rate of diffusion through a Franz cell membrane of between about 28 to about 100 slope/mgT%.

10. A testosterone gel formulation of any of claims 1 - 6, or the testosterone gel formulation for use of claim 7, wherein the testosterone gel formulation has a testosterone rate of diffusion through a Franz cell membrane of between about 30 to about 95 slope/mgT%.

11. A testosterone gel formulation of any of claims 1 - 6, or the testosterone gel formulation for use of claim 7, wherein the testosterone gel formulation has a testosterone rate of diffusion through a Franz cell membrane of between about 28 to about 35 slope/mgT%.

## Patentansprüche

1. Testosteron-Gelformulierung zur nasalen Verabreichung, wobei die genannte Testosteron-Gelformulierung Folgendes umfasst:

a) etwa 4,5 % Testosteron bezogen auf das Gewicht der genannten Gelformulierung und
b) ein pharmazeutisch akzeptables Vehikel.

2. Testosteron-Gelformulierung nach Anspruch 1, wobei die Gelformulierung ein Lösungsmittel, ein Benetzungsmittel und ein viskositätserhöhendes Mittel umfasst.

3. Testosteron-Gelformulierung nach Anspruch 2, wobei das Lösungsmittel Rizinusöl ist.

4. Testosteron-Gelformulierung nach Anspruch 2, wobei das genannte Benetzungsmittel ein Oleoylpolyoxylglycerid ist.

5. Testosteron-Gelformulierung nach Anspruch 2, wobei das genannte viskositätserhöhende Mittel kolloidales Siliciumdioxid ist.

6. Testosteron-Gelformulierung nach Anspruch 1, wobei die genannte Gelformulierung Rizinusöl, Oleoylpolyoxylglyceride und kolloidales Siliciumdioxid umfasst.

7. Testosteron-Gelformulierung nach einem vorherigen Anspruch zur Verwendung bei:

a) der Behandlung von Hypogonadismus bei einem männlichen Subjekt durch intranasale Verabreichung;
b) der Behandlung von Testosteronmangel bei einem männlichen Subjekt durch intranasale Verabreichung; oder
c) der Bereitstellung einer Testosteronersatztherapie bei einem männlichen Subjekt durch intranasale Verabreichung.

8. Verfahren zur Herstellung einer Testosteron-Gelformulierung zur nasalen Verabreichung, wobei die genannte Testosteron-Gelformulierung etwa 4,5 % Testosteron bezogen auf das Gewicht der genannten Gelformulierung und ein pharmazeutisch akzeptables Vehikel umfasst, wobei das Verfahren Folgendes beinhaltet:

a) Kombinieren von mikronisiertem Testosteron in Kontakt mit einem Lösungsmittel, um ein erstes Gemisch zu bilden;
b) Kombinieren von Oleoylpolyoxylglyceriden mit dem ersten Gemisch, um ein zweites Gemisch zu bilden; und
c) Kombinieren von kolloidalem Siliciumdioxid mit dem zweiten Gemisch, um eine Testosteron-Gelformulierung zur nasalen Verabreichung bereitzustellen.

9. Testosteron-Gelformulierung nach einem der Ansprüche 1-6 oder Testosteron-Gelformulierung zur Verwendung nach Anspruch 7, wobei die Testosteron-Gelformulierung eine Testosterondiffusionsrate durch eine Franz-Zellmembran zwischen etwa 28 und etwa 100 Steigung/mgT% aufweist.

10. Testosteron-Gelformulierung nach einem der Ansprüche 1-6 oder Testosteron-Gelformulierung zur Verwendung nach Anspruch 7, wobei die Testosteron-Gelformulierung eine Testosterondiffusionsrate durch eine Franz-Zellmembran zwischen etwa 30 und etwa 95 Steigung/mgT% aufweist.

11. Testosteron-Gelformulierung nach einem der Ansprüche 1-6 oder Testosteron-Gelformulierung zur Verwendung nach Anspruch 7, wobei die Testosteron-Gelformulierung eine Testosterondiffusionsrate durch eine Franz-Zellmembran zwischen etwa 28 und etwa 35 Steigung/mgT% aufweist.

## Revendications

1. Formulation de gel à base de testostérone pour administration nasale, ladite formulation de gel à base de testostérone comprenant :

a) environ 4,5 % de testostérone par poids de ladite formulation de gel ; et
b) un véhicule pharmaceutiquement acceptable.

2. Formulation de gel à base de testostérone selon la revendication 1, où ladite formulation de gel comprend un solvant, un agent mouillant et un agent augmentant la viscosité.

3. Formulation de gel à base de testostérone selon la revendication 2, dans laquelle le solvant est de l'huile de ricin.

4. Formulation de gel à base de testostérone selon la revendication 2, dans laquelle ledit agent mouillant est du polyoxylglycéride d'oléoyle.

5. Formulation de gel à base de testostérone selon la revendication 2, dans laquelle ledit agent augmentant la viscosité est du dioxyde de silicium colloïdal.

6. Formulation de gel à base de testostérone selon la revendication 1, où ladite formulation de gel comprend de l'huile de ricin, du polyoxylglycéride d'oléoyle et du dioxyde de silicium colloïdal.

7. Formulation de gel à base de testostérone selon l'une quelconque des revendications précédentes, pour une utilisation :

a) dans le traitement de l'hypogonadisme chez un sujet mâle par administration intranasale ;
b) dans le traitement d'un déficit en testostérone chez un sujet mâle par administration intranasale ; ou bien
c) destinée à fournir un traitement substitutif de testostérone chez un sujet mâle par administration intranasale.

**8.** Procédé de préparation d'une formulation de gel à base de testostérone pour administration intranasale, ladite formulation de gel à base de testostérone comprenant environ 4,5 % de testostérone par poids de ladite formulation de gel ; et un véhicule pharmaceutiquement acceptable, le procédé comprenant :

a) combiner de la testostérone micronisée en contact avec un solvant pour former un premier mélange ;
b) combiner du polyoxylglycéride d'oléoyle avec le premier mélange pour former un deuxième mélange ; et
c) combiner du dioxyde de silicium colloïdal avec le deuxième mélange pour fournir une formulation de gel à base de testostérone pour administration nasale.

**9.** Formulation de gel à base de testostérone selon l'une quelconque des revendications 1-6, ou la formulation de gel à base de testostérone pour une utilisation selon la revendication 7, où la formulation de gel à base de testostérone a une vitesse de diffusion de testostérone à travers une membrane de cellule de Franz d'entre environ 28 à environ 100 de pente/mgT %.

**10.** Formulation de gel à base de testostérone selon l'une quelconque des revendications 1-6, ou la formulation de gel à base de testostérone pour une utilisation selon la revendication 7, où la formulation de gel à base de testostérone a une vitesse de diffusion de testostérone à travers une membrane de cellule de Franz d'entre environ 30 à environ 95 de pente/mgT %.

**11.** Formulation de gel à base de testostérone selon l'une quelconque des revendications 1-6, ou la formulation de gel à base de testostérone pour une utilisation selon la revendication 7, où la formulation de gel à base de testostérone a une vitesse de diffusion de testostérone à travers une membrane de cellule de Franz d'entre environ 28 à environ 35 de pente/mgT %.

134
130
102
132
100
144
128
140
150
126
122
120
124

FIG. 1

144
146
152
178
150
140
174
128
176
172
142
180
170
164
162
160

FIG. 2

200
202
230
234
232
244
228
240
250
226
222
280
220
224

FIG. 3

244
246
252
278
250
240
274
228
276
272
242
280
270
264
262
260
290
FIG. 4

Ø 16,4 +0,05 -0,10
62,6±0.3
69,15 ±0,55
FUT 15 ML POLI ( -)
BARREL
PISTON 15 ML ( -)
PISTON
FOND 15 ML ( -)
BASE

FIG. 5

Capot SP 15 ( )
OVERCAP SP 15
65,07
±0,8
Poussoir Digital ( )
DIGITAL ACTUATOR
Bague SP15 ( )
SNAP ON SP15
Joint de col ( )
Neck Gasket
Mécanisme VP39/140 H TA Ø14.7 ( )
MECHANISM VP 39/140 H TA Ø14.7
Ø24,2± 0,15

FIG. 6

Project

FIG. 7A

Project

FIG. 7B

Project
ian:
ent:

FIG. 8A

Project
ian:
ent:

FIG. 8B

FIG. 9A

FIG. 9B

**STEP 1**

Blow your nose to clear your nostrils.

**STEP 2**

Remove the cap.

**STEP 3**

Place your finger on the pump of the actuator and in front of a mirror, advance the tip of the actuator into your left nostril until the finger on the pump reaches the base of the nose.

**STEP 4**

Aim the tip of the actuator to the inner corner of your left eye. The opening on the tip of the actuator must face the nasal mucosa.

**STEP 5**

Depress the pump until it stops.

**STEP 6**

Slowly remove the actuator from your nose.

**STEP 7**

Repeat the process for the right nostril.

**STEP 8**

Place your finger on the pump of the actuator and, in front of a mirror, advance the tip of the actuator into your right nostril until the finger on the pump reaches the base of the nose.

**STEP 9**

Aim the tip of the actuator to the inner corner of your right eye. The opening on the tip of the actuator must face the nasal mucosa.

FIG. 10A

**STEP 10**

Depress the pump until it stops. Slowly remove the actuator from your nose.

**STEP 11**

Wipe away any gel that remains on the tip of the actuator using a clean dry swab.

**STEP 12**

Press the nostrils near the bridge of the nose lightly together and massage for one second. **Do not blow your nose or sniff for one hour after administration of the medication.**

FIG. 10B

TRIMEL
BIOPHAMA
TBS-1
TRIMEL
BIOPHAMA
TBS-1
IF YOU HAVE ANY QUESTIONS OR CONCERNS, PLEASE CONTACT YOUR SITE COORDINATOR.
PHONE #:
PATIENT INSTRUCTIONS
TBS-1 TESTOSTERONE INTRANASAL GEL PROCEDURE FOR MULTIPLE DOSE DISPENSER

FIG. 11

Franz Cell
Donor Compound
Donor Chamber
Flat Ground Joint
Membrane
Sampling Port
Heater/ circulator
Receptor Chamber
Water Jacket
Stirbar

FIG. 12

TRT
TBS-1 vs. Normal Pharmacokinetics
4.5 BID
Normal
Serum testosterone ng/dL
800.00
700.00
600.00
500.00
400.00
300.00
200.00
100.00
0.00
1.0
3.0
5.0
7.0
9.0
11.0
13.0
15.0
17.0
19.0
21.0
23.0
Time of Day. 24 Hour Clock

FIG. 13

Comparison between TBS 1 A 8% (Part I)
Diffusion (µg/cm2)
12000
10000
8000
6000
4000
2000
0
5
7
9
11
13
15
17
19
21
√times (minutes)
RD11089 y = 489.46x - 1999.2
R2 = 0.9995
RD11089A y = 425.39x - 1454.6
R2 = 0.9998
RD11087 y = 636.05x - 2810.2
R2 = 0.9976
RD11101 y = 526.63x - 2085.1
R2 = 0.9997

| Diffusion (µg/cm$^2$) | RD11089 | RD11089A | RD11087 | RD11101 |
|---|---|---|---|---|
| 1 hour | 1853 | 1813 | 2294 | 2020 |
| 2 hours | 3308 | 3229 | 4014 | 3636 |
| 3 hours | 4525 | 4279 | 5588 | 4971 |
| 4 hours | 5580 | 5141 | 6998 | 6127 |
| 5 hours | 6488 | 5892 | 8252 | 7407 |
| 6 hours | 7317 | 6609 | 9356 | 7913 |

FIG. 14

Comparison between TBS 1 A 8% (Part II)
Diffusion (μg/cm2)
12000
10000
8000
6000
4000
2000
0
5
7
9
11
13
15
17
19
21
√times (minutes)
RD11102
y = 499.5x - 2069.9
R2 = 0.9988
RD11104
y = 755.62x - 3397.2
R2 = 0.9979
IMP1103
y = 613.24x - 2673.9
R2 = 0.993

| Diffusion ($\mu g/cm^2$) | RD11102 | RD11104 | IMP1103 |
|---|---|---|---|
| 1 hour | 1894 | 2647 | 2342 |
| 2 hours | 3333 | 4702 | 3898 |
| 3 hours | 4545 | 6589 | 5237 |
| 4 hours | 5662 | 8299 | 6768 |
| 5 hours | 6584 | 9796 | 8046 |
| 6 hours | 7471 | 10980 | 9116 |

Note: RD11101 and RD11102 were run up to 24 hours.

FIG. 15

Comparison between 6 hours and 24 hours
(RD11101 and RD11102)
Diffusion (µg/cm2)
20000
18000
16000
14000
12000
10000
8000
6000
4000
2000
0
5
10
15
20
25
30
35
40
√times (minutes)
RD11101 6 hours
RD11102 6 hours
RD11101 24 hours
RD11102 24 hours
y = 526.63x - 2085.1
R2 = 0.9997
y = 499.5x - 2069.9
R2 = 0.9988
y = 535.69x - 2798.8
R2 = 0.9974
y = 396.57x - 612.14
R2 = 0.9874

| Diffusion (µg/cm²) | RD11101 6 hours | RD11102 6 hours | RD11101 24 hours | RD11102 24 hours |
|---|---|---|---|---|
| 1 hour | 2020 | 1894 | 1812 | 1957.7 |
| 2 hours | 3636 | 3333 | 3154 | 3427.49 |
| 3 hours | 4971 | 4545 | 4294 | 4626.42 |
| 4 hours | 6127 | 5662 | 5324 | 5705.54 |
| 5 hours | 7407 | 6584 | 6253 | 6682.36 |
| 6 hours | 7913 | 7471 | 7085 | 7581.86 |
| 24 hours | | | 17759 | 14031.69 |

FIG. 16

Comparison between TBS 1 A 4% (Part I)
Diffusion (µg/cm2)
7000
6000
5000
4000
3000
2000
1000
0
5
7
9
11
13
15
17
19
21
√times (minutes)
RD11038 y = 271.06x - 1265.3 R2 = 0.9961
RD11039 y = 508.88x - 3084.4 R2 = 0.9991
RD11040 y = 389.27x - 312.27 R2 = 0.9492
RD11042 y = 366.34x - 1687.5 R2 = 0.9914
RD11051 y = 313.35x - 1614.1 R2 = 0.9882

| Diffusion (µg/cm$^2$) | RD11038 | RD11039 | RD11040 | RD11042 | RD11051 |
|---|---|---|---|---|---|
| 1 hour | 1053 | | 2539.93 | 1331.36 | 940.02 |
| 2 hours | 1668 | 2539.93 | 3676.36 | 2210.28 | 1700.72 |
| 3 hours | 2281 | 3663.66 | 5434.28 | 3084.42 | 2407.13 |
| 4 hours | 3002 | 5434.09 | 5977.58 | 3897.68 | 3421.73 |
| 5 hours | 3433 | 5977.58 | 6574.52 | 4662.27 | 3758.81 |
| 6 hours | 3952 | 6574.52 | 6583.47 | 5425.09 | 4376.96 |

FIG. 17

Comparison between TBS 1 A 4% (Part II)
Diffusion (µg/cm2)
7000
6000
5000
4000
3000
2000
1000
0
5
7
9
11
13
15
17
19
21
√times (minutes)
RD11053 y = 261.82x - 1187.7
R2 = 0.9963
RD11055 y = 307.28x - 1428.1
R2 = 0.9986
RD11063 y = 187.46x - 924.39
R2 = 0.9922
RD11078 y = 269.37x - 1309.9
R2 = 0.9947
RD11085 y = 272.4x - 1550
R2 = 0.9952

| Diffusion (µg/cm$^2$) | RD11053 | RD11055 | RD11063 | RD11078 | RD11085 |
|---|---|---|---|---|---|
| 1 hour | 926.97 | 1018.43 | 607.92 | 912.21 | 1130.37 |
| 2 hours | 1587.49 | 1874.39 | 1075.25 | 1582.63 | 1421.98 |
| 3 hours | 2251.5 | 2656.54 | 1686.95 | 2224.12 | 2021.15 |
| 4 hours | 2836.99 | 3323.18 | 1930.65 | 2835.75 | 2606.84 |
| 5 hours | 3350.39 | 3902.94 | 2334.99 | 3355.76 | 3160.31 |
| 6 hours | 3829.88 | 4436.66 | 2712.56 | 3954.09 | 3698.21 |

FIG. 18

Comparison between TBS 1 A 4% (Part III)
Diffusion (µg/cm2)
7000
6000
5000
4000
3000
2000
1000
0
5
7
9
11
13
15
17
19
21
√times (minutes)
RD11054 y = 244.7x - 1214.3
R2 = 0.992
IMP11001 y = 300.02x - 1371.8
R2 = 0.9964
IMP11002 y = 220.68x - 991.01
R2 = 0.9941
RD11061 y = 397.43x - 2327.6
R2 = 0.9923

| Diffusion (µg/cm$^2$) | RD11054 | RD11061 | IMP11001 | IMP11002 |
|---|---|---|---|---|
| 1 hour | 1006.97 | 975.48 | 1054.424 | 807.4 |
| 2 hours | 1415.17 | 1344.55 | 1838.037 | 1360.268 |
| 3 hours | 2059.74 | 2853.74 | 2574.543 | 1922.042 |
| 4 hours | 2512.42 | 3760.88 | 3242.818 | 2378.231 |
| 5 hours | 2990.82 | 4593.06 | 3841.022 | 2816.161 |
| 6 hours | 3543.18 | 5330.24 | 4389.895 | 3285.301 |

FIG. 19

Comparison between TBS 1 A 4% (slower)
Diffusion (µg/cm²)
7000
6000
5000
4000
3000
2000
1000
0
5
7
9
11
13
15
17
19
21
√times (minutes)
RD11063 y = 187.46x - 924.39
R² = 0.9922
RD11085 y = 272.4x - 1550
R² = 0.9952
IMP11002 y = 220.68x - 991.01
R² = 0.9941

Note: RD11063 and RD11085 were run up to 24 hours.

FIG. 20

Comparison between 6 hours and 24
(RD11063 and RD11085)
Diffusion (μg/cm2)
10000
9000
8000
7000
6000
5000
4000
3000
2000
1000
0
5
10
15
20
25
30
35
40
√times (minutes)
RD11063 6 hours
y = 187.46x - 924.39
R2 = 0.9922
RD11085 6 hours
y = 272.4x - 1550
R2 = 0.9952
RD11063 24 hours
y = 245.07x - 1744.6
R2 = 0.9834
RD11085 24 hours
y = 303.11x - 2017.9
R2 = 0.9934

| Diffusion ($\mu g/cm^2$) | RD11063 6 hours | RD11085 6 hours | RD11063 24 hours | RD11085 24 hours |
|---|---|---|---|---|
| 1 hour | 607.92 | 1130.37 | 600.49 | 913.01 |
| 2 hours | 1075.25 | 1421.98 | 1053.99 | 1407.08 |
| 3 hours | 1686.95 | 2021.15 | 1449.92 | 1972.8 |
| 4 hours | 1930.65 | 2606.84 | 1813.75 | 2514.87 |
| 5 hours | 2334.99 | 3160.31 | 2185.79 | 3017.65 |
| 6 hours | 2712.56 | 3698.21 | 2528.07 | 3488.02 |
| 24 hours | | | 7828.4 | 9680.91 |

Note: we wanted to see if there was a difference if we add 400 mg of gel.

FIG. 21

Comparison between 400mg and 1 gram
(RD11063 )
Diffusion (µg/cm2)
4000
3000
2000
1000
0
5
7
9
11
13
15
17
19
21
√times (minutes)
400 mg y = 195.2x - 1044.9
R2 = 0.9955
1 grame y = 187.46x - 924.39
R2 = 0.9922

400mg

| √Times (minutes) | Diffusion (µg/cm$^2$) |
|---|---|
| 7.75 | 542.24 |
| 10.95 | 1042.73 |
| 12.88 | 1395.43 |
| 17.75 | 2432.99 |
| 18.97 | 2694.41 |

1gr

| √Times (minutes) | Diffusion (µg/cm$^2$) |
|---|---|
| 7.74 | 607.92 |
| 10.95 | 1075.25 |
| 14.32 | 1686.95 |
| 15.49 | 1930.65 |
| 17.32 | 2334.99 |
| 18.97 | 2712.56 |

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2010311707 A
- US 5578588 A
- US 5756071 A
- US 20050100564 A
- US 20070149454 A
- US 20090227550 A


### Non-patent literature cited in the description


- Tietz: Textbook of Clinical Chemistry and Molecular Diagnostics. 2006
- **WANG C ; SWERDLOFF R.S.** Androgen replacement therapy. *Ann Med,* 1997, vol. 29, 365-370
- **MATSUMOTO A.M.** Andropause: clinical implications of the decline in serum Testosterone levels with aging in men. *J Gerontol A Med Sci,* 2002, vol. 57, M76-M99
- **HAREN MT et al.** Andropause: a quality-of-life issue in older males. *Med Clin North Am,* 2006, vol. 90, 1005-1023
- **DIVER MJ. et al.** Diurnal rhythms of total, free and bioavailable testosterone and of SHBG in middle-aged men compared with those in young men. *Clinical Endocrinology,* 2003, vol. 58, 710-717
- **HARRISON'S.** *Principles of Internal Medicine,* 1997
- **ROBBINS.** *Pathologic Basis of Disease,* 1998
- *CHEMICAL ABSTRACTS,* 58-22-0
- **MATTERN, C. et al.** *The Aging Male,* December 2008, vol. 11 (4), 171-178
- Tietz Textbook of Clinical Chemistry and Molecular Diagnostics. 2006
- **WANG C ; SWERDLOFF RS.** Androgen replacement therapy. *Ann Med,* 1997, vol. 29, 365-370
- **MATSUMOTO AM.** Andropause: clinical implications of the decline in serum Testosterone levels with aging in men. *J Gerontol A Med Sci,* 2002, vol. 57, M76-M99
- **HAREN MT ; KIM MJ ; TARIQ SH ; WITTERT GA ; MORLEY JE.** Andropause: a quality-of-life issue in older males. *Med Clin North Am,* 2006, vol. 90, 1005-1023
- **NIESCHLAG E.** Testosterone treatment comes of age: new options for hypogonadal men. *Clin Endocrinol (Oxf),* 2006, vol. 65, 275-281
- **TENOVER JL.** The androgen-deficient aging male: current treatment options. *Rev Urol,* 2003, vol. 5, S22-S28
- **JOCKENHOVEL F.** Testosterone therapy - what, when and to whom?. *Aging Male,* 2004, vol. 7, 319-324
- **KUNZ GH ; KLEIN KO ; CLEMONS RD ; GOTTSCHALK ME ; JONES KL.** Virilization of young children after topical androgen use by their parents. *Pediatrics,* 2004, vol. 114, 282-284
- **BRACHET C ; VERMEULEN J ; HEINRICHS C.** Children's virilisation and the use of a Testosterone gel by their fathers. *Eur J Pediatr,* 2005, vol. 164, 646-647
- **BAGCHUS WM ; HUST R ; MARIS F ; SCHNABEL PG ; HOUWING NS.** Important effect of food on the bioavailability of oral Testosterone undecanoate. *Pharmacotherapy,* 2003, vol. 23, 319-325
- **HAREN M ; CHAPMAN IM ; HAREN MT ; MACKINTOSH S ; COATES P ; MORLEY JE.** Oral Testosterone supplementation increases muscle and decreases fat mass in healthy elderly males with low normal gonadal status. *J Gerontol A Biol Sci Med Sci,* 2003, vol. 58, 618-625
- **HAREN M ; CHAPMAN I ; COATES P ; MORLEY JE ; WITTERT G.** Effect of 12 month oral Testosterone on Testosterone deficiency symptoms in symptomatic elderly males with low-normal gonadal status. *Age Ageing,* 2005, vol. 34, 123-130
- **MATTERN C ; HOFFMANN C ; MORLEY JE ; BADIU C.** *The Aging Male,* 2008, vol. 11, 171-178